# EUROPEAN PATENT SPECIFICATION

(11) **EP 4 294 375 B1**
(45) Date of publication and mention of the grant of the patent: **01.04.2026**
(21) Application number: 22709099.0
(22) Date of filing: 17.02.2022
(51) Int. Cl.: A61K 9/51, A61K 35/12, A61K 39/00

(54) **METHODS OF LOADING EXTRACELLULAR VESICLES**
VERFAHREN ZUM LADEN VON EXTRAZELLULÄREN VESIKELN
PROCÉDÉS DE CHARGEMENT DE VÉSICULES EXTRACELLULAIRES

(30) Priority: 17.02.2021 US 202163150497 P
(43) Date of publication of application: 27.12.2023
(73) Proprietor: Lonza Sales AG, 4052 Basel (CH)
(72) Inventor: ELLIS, Kimberly, Cambridge, Massachusetts 02140 (US); NOYES, Aaron, Cambridge, Massachusetts 02140 (US); WOOD, Andrew, Cambridge, Massachusetts 02140 (US)
(74) Representative: Greiner, Elisabeth
(86) International application number: PCT/US2022/016825
(87) International publication number: WO 2022/178147

(56) References cited:
- WO-A1-2020/101740
- WO-A1-2020/191369
- WO-A1-2020/191377
- WO-A2-2020/191361
- US-A1- 2019 151 456

## Description

### FIELD OF DISCLOSURE

The invention is set out in the appended set of claims. The present disclosure relates to methods of loading an extracellular vesicle (EV) with a payload (e.g., antisense oligonucleotides) comprising mixing the payload with the EV (i) in a loading buffer comprising a salt at a concentration between about 110 mM to lower than about 200 mM; (ii) at a loading temperature higher than 4 °C; and (iii) for a loading duration longer than about one hour, wherein the loading buffer comprising the salt increases the loading of the payload to the EV by at least about 2 fold compared to a corresponding loading buffer without the salt or with a lower concentration of the salt. The present disclosure also relates to EVs (*e.g.,* exosomes) comprising a payload, wherein the payload is loaded using the methods described herein.

### BACKGROUND OF DISCLOSURE

Many bioactive compounds have potent biological activity that is of therapeutic interest. However, these compounds often exhibit toxicity in non-target organs. One way to limit exposure of non-target tissues is to chemically conjugate small molecules to affinity-based reagents such as antibodies, which can direct the therapeutic compound to specific cell types (Dosio, F. et al., Toxins (Basel) 3(7):848-883 (2011)), but this approach is limited by the number of molecules of the compound of interest that can be attached to an antibody (typically 2-6 molecules per antibody), and by the availability/existence of antibodies that specifically bind to targeted, relevant diseased/effector cells without binding to non-target cells. These two issues limit the use of antibody-drug conjugates (ADC) by decreasing potency and increasing systemic toxicity, respectively. Accordingly, there is a need for delivery systems with a higher payload than ADCs that can selectively target specific tissues or organs while at the same time limiting overall systemic exposure to the therapeutic compound.

EVs are important mediators of intercellular communication. As drug delivery vehicles, EVs offer many advantages over traditional drug delivery methods (*e.g.,* peptide immunization, DNA vaccines) as a new treatment modality in many therapeutic areas. For example, WO 2020/191361 A2 discloses EVs for vaccine delivery, US 2019/0151456 discloses exosomes comprising a target protein, WO 2020/191369 discloses multistep chromatographic methods for preparing EVs, and WO 2020/191377 discloses EVs comprising a biologically active molecule covalently linked to the EVs via a maleimide moiety. However, despite its advantages, many EVs have had limited clinical efficacy. For example, dendritic-cell derived exosomes (DEX) were investigated in a Phase II clinical trial as maintenance immunotherapy after first line chemotherapy in patients with inoperable non-small cell lung cancer (NSCLC). However, the trial was terminated because the primary endpoint (at least 50% of patients with progression-free survival (PFS) at 4 months after chemotherapy cessation) was not reached. Besse, B., et al., Oncoimmunology 5(4):e1071008 (2015).

Accordingly, new and more effective engineered EVs (*e.g.,* comprising greater payload concentration) are necessary to better enable therapeutic use and other applications of EV-based technologies.

### BRIEF SUMMARY OF THE DISCLOSURE

Provided herein is a method of loading a payload to an extracellular vesicle comprising mixing the payload with the extracellular vesicle (i) in a loading buffer comprising a salt at a concentration between about 110 mM to lower than about 200 mM; (ii) at a loading temperature higher than 4 °C; and (iii) for a loading duration longer than about one hour, wherein the loading buffer comprising the salt increases the loading of the payload to the EV by at least about 2 fold compared to a corresponding loading buffer without the salt or with a lower concentration of the salt.

In some aspects, the salt concentration is between about 110 mM and about 150 mM, between about 110 mM and about 130 mM, or between about 110 mM and about 120 mM. In certain aspects, the salt concentration is about 110 mM, about 120 mM, about 130 mM, about 140 mM, about 150 mM, about 160 mM, about 170 m, about 180 mM, or about 190 mM. In some aspects, the salt concentration is about 110 mM, about 120 mM, about 130 mM, about 140 mM, or about 150 mM.

Disclosed is a method of loading a payload to an extracellular vesicle comprising mixing the payload with the extracellular vesicle in a loading buffer comprising a salt at a concentration between about 200 mM and about 450 mM such as wherein the salt concentration is about 200 mM, about 250 mM, about 300 mM, about 350 mM, about 400 mM, or about 450 mM.

For any of the methods provided above, in some aspects, the salt comprises NaCl (sodium chloride), KCl (potassium chloride), PO₄ (phosphate salt), CaCl₂ (calcium chloride), MgCl₂ (magnesium chloride), Mg₂SO₄ (magnesium sulfate), ZnCl₂ (zinc chloride), MnCl₂ (manganese chloride), MnSO₄ (manganese sulfate), NaSCN (sodium thiocyanate), KSCN (potassium thiocyanate), LiCl (lithium chloride), K₂HPO₄ (dipotassium phosphate), Na₂SO₄ (sodium sulfate), NaPO₄ (sodium phosphate), K₂SO₄ (potassium phosphate), sodium acetate, sodium bromide, sodium iodide, potassium bromide, lithium bromide, sodium fluoride, potassium fluoride, lithium fluoride, lithium iodide, potassium acetate, lithium acetate, potassium iodide, calcium sulfate, sodium sulfate, chromium trichloride, chromium sulfate, sodium citrate, iron (III) chloride, yttrium (III) chloride, potassium sulfate, ferrous chloride, calcium citrate, magnesium phosphate, ferric chloride, arginine-HCl, or any combination thereof. In some aspects, the buffer comprising the salt increases the loading of the payload to the EV by at least about 2 fold, at least about 3 fold, at least about 4 fold, at least about 5 fold, at least about 6 fold, at least about 7 fold, at least about 8 fold, at least about 9 fold, at least about 10 fold, at least about 12 fold, at least about 14 fold, at least about 16 fold, at least about 18 fold, at least about 20 fold, at least about 25-fold, at least about 30-fold, at least about 35-fold, at least about 40-fold, at least about 45-fold, or at least about 50-fold or more compared to a corresponding loading buffer without the salt or with a lower concentration of the salt.

Provided herein is a method of loading a payload to an extracellular vesicle comprising mixing the payload with the extracellular vesicle at a loading temperature higher than 4 °C.

In some aspects, the loading temperature is between about 5 °C and about 40 °C, between about 10 °C and about 40 °C, between about 15 °C and about 40 °C, between about 20 °C and about 40 °C, between about 25 °C and about 40 °C, between about 30 °C and about 40 °C, between about 35 °C and about 40 °C, between about 5 °C and about 38 °C, between about 10 °C and about 38 °C, between about 15 °C and about 38 °C, between about 20 °C and about 38 °C, between about 25 °C and about 38 °C, between about 30 °C and about 38 °C, between about 35 °C and about 38 °C, between about 5 °C and about 37 °C, between about 10 °C and about 37 °C, between about 15 °C and about 37 °C, between about 20 °C and about 37 °C, between about 25 °C and about 37 °C, between about 30 °C and about 37 °C, or between about 35 °C and about 37 °C. In certain aspects, the loading temperature is about 5 °C, about 10°C, about 15 °C, about 20°C, about 25 °C, about 30°C, about 35 °C, about 37 °C, about 40 °C, about 50 °C, about 55 °C, about 60 °C, about 65 °C, or about 70 °C.

In some aspects, the loading temperature increases the loading of the payload to the EV by at least about 2 fold, at least about 3 fold, at least about 4 fold, at least about 5 fold, at least about 6 fold, at least about 7 fold, at least about 8 fold, at least about 9 fold, at least about 10 fold, at least about 12 fold, at least about 14 fold, at least about 16 fold, at least about 18 fold, at least about 20 fold, at least about 15-fold, at least about 20-fold, at least about 25-fold, at least about 30-fold, at least about 35-fold, at least about 40-fold, at least about 45-fold, or at least about 50-fold or more compared to the loading at a reference temperature, which is lower than the loading temperature.

Provided herein is a method of loading a payload to an extracellular vesicle comprising mixing the payload with the extracellular vesicle for a loading duration longer than about one hour.

In some aspects, the loading duration is between about one hour and about 48 hours, between about one hour and about 42 hours, between about one hour and about 36 hours, between about one hour and about 30 hours, between about one hour and about 24 hours, between about six hours and about 48 hours, between about six hours and about 42 hours, between about six hours and about 36 hours, between about six hours and about 30 hours, between about six hours and about 24 hours, between about 12 hours and about 48 hours, between about 12 hours and about 42 hours, between about 12 hours and about 36 hours, between about 12 hours and about 30 hours, or between about 12 hours and about 24 hours. In certain aspects, the loading duration is about 6 hours, about 12 hours, about 24 hours, about 30 hours, about 36 hours, about 42 hours, about 48 hours, about three days, about four days, about five days, about six days, or about seven days.

In some aspects, the loading duration increases the loading of the payload to the EV by at least about 2 fold, at least about 3 fold, at least about 4 fold, at least about 5 fold, at least about 6 fold, at least about 7 fold, at least about 8 fold, at least about 9 fold, or at least about 10 fold, at least about 12 fold, at least about 14 fold, at least about 16 fold, at least about 18 fold, at least about 20 fold, at least about 25-fold, at least about 30-fold, at least about 35-fold, at least about 40-fold, at least about 45-fold, or at least about 50-fold or more compared to the loading for a reference duration, which is shorter than the loading duration.

Also provided herein is a method of loading a payload to an extracellular vesicle comprising mixing the payload with the extracellular vesicle in a loading buffer comprising a salt at a concentration between about 110 mM to lower than 200 mM, at a loading temperature between about 5 °C and about 50 °C, and for a loading duration between one hour to about 48 hours.

In some aspects, the salt concentration is between about 110 mM and about 150 mM, between about 110 mM and about 130 mM, between about 110 mM and about 120 mM, about 110 mM, about 120 mM, about 130 mM, about 140 mM, about 150 mM, about 160 mM, about 170 m, about 180 mM, or about 190 mM. In some aspects, the salt concentration is about 110 mM, about 120 mM, about 130 mM, about 140 mM, or about 150 mM.

In some aspects, salt comprises NaCl (sodium chloride), KCl (potassium chloride), PO₄ (phosphate salt), CaCl₂ (calcium chloride), MgCl₂ (magnesium chloride), Mg₂SO₄ (magnesium sulfate), ZnCl₂ (zinc chloride), MnCl₂ (manganese chloride), MnSO₄ (manganese sulfate), NaSCN (sodium thiocyanate), KSCN (potassium thiocyanate), LiCl (lithium chloride), K₂HPO₄ (dipotassium phosphate), Na₂SO₄ (sodium sulfate), NaPO₄ (sodium phosphate), K₂SO₄ (potassium phosphate), sodium acetate, sodium bromide, sodium iodide, potassium bromide, lithium bromide, sodium fluoride, potassium fluoride, lithium fluoride, lithium iodide, potassium acetate, lithium acetate, potassium iodide, calcium sulfate, sodium sulfate, chromium trichloride, chromium sulfate, sodium citrate, iron (III) chloride, yttrium (III) chloride, potassium sulfate, ferrous chloride, calcium citrate, magnesium phosphate, ferric chloride, arginine-HCl, or any combination thereof.

In some aspects, the loading temperature is between about 5 °C and about 40 °C, between about 10 °C and about 40 °C, between about 15 °C and about 40 °C, between about 20 °C and about 40 °C, between about 25 °C and about 40 °C, between about 30 °C and about 40 °C, between about 35 °C and about 40 °C, between about 5 °C and about 38 °C, between about 10 °C and about 38 °C, between about 15 °C and about 38 °C, between about 20 °C and about 38 °C, between about 25 °C and about 38 °C, between about 30 °C and about 38 °C, between about 35 °C and about 38 °C, between about 5 °C and about 37 °C, between about 10 °C and about 37 °C, between about 15 °C and about 37 °C, between about 20 °C and about 37 °C, between about 25 °C and about 37 °C, between about 30 °C and about 37 °C, or between about 35 °C and about 37 °C. In certain aspects, the loading temperature is about 5 °C, about 10 °C, about 15 °C, about 20 °C, about 25 °C, about 30 °C, about 35 °C, about 37 °C, about 40 °C, or about 50 °C.

In some aspects, the loading duration is between about one hour and about 48 hours, between about one hour and about 42 hours, between about one hour and about 36 hours, between about one hour and about 30 hours, between about one hour and about 24 hours, between about six hours and about 48 hours, between about six hours and about 42 hours, between about six hours and about 36 hours, between about six hours and about 30 hours, between about six hours and about 24 hours, between about 12 hours and about 48 hours, between about 12 hours and about 42 hours, between about 12 hours and about 36 hours, between about 12 hours and about 30 hours, or between about 12 hours and about 24 hours. In certain aspects, the loading duration is about 6 hours, about 12 hours, about 24 hours, about 30 hours, about 36 hours, about 42 hours, or about 48 hours.

In some aspects, the salt concentration is between about 110 mM and about 150 mM, the loading temperature is between about 30 °C and about 40 °C, and the loading duration is between about 20 hours and about 30 hours. In some aspects, the salt concentration is between about 110 mM and about 150 mM, the loading temperature is between about 30 °C and about 40 °C, and the loading duration is between about 12 hours and about 30 hour.

Disclosed is a method of loading a payload to an extracellular vesicle comprising mixing the payload with the extracellular vesicle in a loading buffer comprising a salt at a concentration of about 100 mM, at a loading temperature of about 37 °C, and for a loading duration of about 24 hours.

In some aspects, the loading at the salt concentration at the loading temperature for the loading duration increases loading of the payload to the extracellular vesicle by at least about 2 fold, at least about 3 fold, at least about 4 fold, at least about 5 fold, at least about 6 fold, at least about 7 fold, at least about 8 fold, at least about 9 fold, at least about 10 fold, at least about 12 fold, at least about 14 fold, at least about 16 fold, at least about 18 fold, at least about 20 fold, at least about 25-fold, at least about 30-fold, at least about 35-fold, at least about 40-fold, at least about 45-fold, or at least about 50-fold or more compared to the loading without the salt, at a temperature of 4 °C, and/or for a duration of one hour.

Also provided herein is a method of loading a payload to an extracellular vesicle comprising mixing the payload with the extracellular vesicle in a loading buffer comprising a salt at a concentration of between about 150 mM and less than about 200 mM, at a loading temperature of between about 37 °C to about 70 °C, and for a loading duration between about 24 hours and about seven days. In some aspects, the salt concentration is about 150 mM. In some aspects, the salt comprises NaCl (sodium chloride), KCl (potassium chloride), PO₄ (phosphate salt), CaCl₂ (calcium chloride), MgCl₂ (magnesium chloride), Mg₂SO₄ (magnesium sulfate), ZnCl₂ (zinc chloride), MnCl₂ (manganese chloride), MnSO₄ (manganese sulfate), NaSCN (sodium thiocyanate), KSCN (potassium thiocyanate), LiCl (lithium chloride), K₂HPO₄ (dipotassium phosphate), Na₂SO₄ (sodium sulfate), NaPO₄ (sodium phosphate), K₂SO₄ (potassium phosphate), sodium acetate, sodium bromide, sodium iodide, potassium bromide, lithium bromide, sodium fluoride, potassium fluoride, lithium fluoride, lithium iodide, potassium acetate, lithium acetate, potassium iodide, calcium sulfate, sodium sulfate, chromium trichloride, chromium sulfate, sodium citrate, iron (III) chloride, yttrium (III) chloride, potassium sulfate, ferrous chloride, calcium citrate, magnesium phosphate, ferric chloride, arginine-HCl, or any combination thereof.

For the methods described in the above paragraph, in some aspects, the loading temperature is about 37 °C, about 40 °C, about 45 °C, about 50 °C, about 55 °C, about 60 °C, about 65 °C, or about 70 °C. In some aspects, the loading duration is about 24 hours, about two days, about three days, about four days, about five days, about six days, or about seven days.

For any of the methods provided above, in some aspects, the method further comprises, after the mixing, measuring the amount of payload loaded to the EVs and determining the load density of the EVs. In some aspects, one or more loading parameters are adjusted to further increase the load density of the EVs, and wherein the one or more loading parameters are selected from: a salt concentration of the loading buffer, loading temperature, loading duration, payload feed concentration, EV feed concentration, or a combination thereof. In some aspects, the amount of payload loaded to the EVs is measured using particle counters, nanoparticle tracking analysis (NTA), ultraviolet adsorption, visible adsorption, fluorescence, near infrared, infrared, static light scattering, dynamic light scattering, obscuration, Raman spectroscopy, turbidity, or a combination thereof.

Disclosed is a method of decreasing the dissociation of a payload from an extracellular vesicle (EV) after EV loading comprising loading the payload to the EV according to any of the loading methods provided herein (*e.g.,* such as those provided above). In some aspects, the dissociation of the payload from the EV is reduced by at least about 10%, at least about 20%, at least about 30%, at least about 40%, at least about 50%, at least about 60%, at least about 70%, at least about 80%, at least about 90%, or about 100% compared to the corresponding dissociation observed when the payload is not loaded to the EV according to any of the loading methods provided herein.

Disclosed is a method of increasing the stability of an extracellular vesicle (EV) loaded with a payload comprising loading the payload to the EV according to any of the loading methods provided herein. In some aspects, the stability of the EV loaded with the payload is increased by at least about at least about 1 fold, at least about 2 fold, at least about 3 fold, at least about 4 fold, at least about 5 fold, at least about 6 fold, at least about 7 fold, at least about 8 fold, at least about 9 fold, at least about 10 fold, at least about 12 fold, at least about 14 fold, at least about 16 fold, at least about 18 fold, at least about 20 fold, at least about 25-fold, at least about 30-fold, at least about 35-fold, at least about 40-fold, at least about 45-fold, or at least about 50-fold or more compared to the corresponding stability observed when the payload is not loaded to the EV according to any of the loading methods of the present disclosure.

Present disclosure further provides a method of improving one or more properties of an extracellular vesicle after loading with a payload comprising loading the payload to the EV according to any of the inventive loading methods provided herein, wherein the one or more properties comprise: decreasing the dissociation of the payload from the EV, increasing the stability of the EV loaded with the payload, inter-particle interaction, particle rigidity, particle size, or a combination thereof, preferably wherein the improvement in the one or more properties allow for increased filterability of the EV.

For any of the methods provided above, in some aspects, the loading buffer further comprises one or more components. In certain aspects, the one or more components comprise tris, sucrose, glucose, trehalose, mannose, sorbitol, mannitol, glycerol, histidine, arginine, methionine, tryptophan, tyrosine, sodium phosphate, potassium phosphate, polymers, surfactants (*e.g.,* polysorbates 80 and 20), chelating agents (*e.g.,* EDTA, citrate), inorganic acids, or any combination thereof.

In some aspects, the loading buffer further comprises sucrose. In certain aspects, the sucrose is at a concentration lower than about 10 % w/v, lower than about 9 % w/v, lower than about 8 % w/v, lower than about 7 % w/v, lower than about 6% w/v, lower than about 5 % w/v, lower than about 4 % w/v, lower than about 3% w/v, or lower than about 2.5 % w/v. In some aspects, the sucrose is at a concentration between about 1 % w/v and about 10 % w/v, between about 2 % w/v and about 10 % w/v, between about 3 % w/v and about 10 % w/v, between about 4 % w/v and about 10 % w/v, between about 5 % w/v and about 10 % w/v, between about 1 % w/v and about 8 % w/v, between about 2 % w/v and about 8 % w/v, between about 3 % w/v and about 8 % w/v, between about 4 % w/v and about 8 % w/v, between about 5 % w/v and about 8 % w/v, between about 1 % w/v and about 5 % w/v, between about 2 % w/v and about 5 % w/v, between about 3 % w/v and about 5 % w/v, between about 4 % w/v and about 5 % w/v, or between about 5 % w/v and about 6 % w/v.

In some aspects, the loading buffer is at an osmolarity between about 100 and about 600 mOsm/kg. In some aspects, the loading buffer is at an osmolarity between about 275 and about 450, between about 280 and about 450, between about 300 and about 450, between about 275 and about 400, between about 280 and about 400, between about 300 and about 400, between about 275 and about 380, between about 280 and about 380, between about 300 and about 380, between about 275 and about 350, between about 280 and about 350, between about 300 and about 350, between about 275 and about 310, between about 280 and about 310, or between about 300 and about 310 mOsm/kg. In certain aspects, the loading buffer is at an osmolarity of about 360, about 370, about 380, about 390, about 395, or about 400 mOsm/kg. In some aspects, the loading buffer is at an osmolarity between about 100 and about 600 mOsm/kg.

In some aspects, the loading buffer is at a pH between about 6 and about 8. In certain aspects, the pH is between about 6 and about 7 or between about 7 and about 8. In some aspects, the pH is about 6, about 7, or about 8. In some aspects, the loading buffer is at a pH of about 9.

In some aspects, a payload which can be loaded in an EV using any of the loading methods provided herein comprises a peptide, a small molecule, an oligonucleotide, an antisense oligonucleotide (ASO), a PMO, a mRNA, a miRNA, a lcRNA, an antagomir, a tRNA, a siRNA, peptide nucleic acid, a cell penetrating peptide, an adjuvant, a protein, a carbohydrate, a sugar, an amino acid, or any combination thereof.

In some aspects, the payload comprises a nucleic acid. In some aspects, the nucleic acid comprises a contiguous sequence in length that is less than about 40, less than about 35, less than about 30, less than about 25, less than about 20, less than about 19, less than about 18, less than about 17, less than about 16, or less than about 15 nucleotides.

In some aspects, the nucleic acid comprises a contiguous sequence in length between about 10 and about 30, between about 14 and about 30, between about 15 and about 30, between about 16 and about 30, between about 17 and about 30, between about 18 and about 30, between about 19 and about 30, between about 20 and about 30, between about 10 and about 25, between about 14 and about 25, between about 15 and about 25, between about 16 and about 25, between about 17 and about 25, between about 18 and about 25, between about 19 and about 25, between about 20 and about 25, between about 10 and about 22, between about 14 and about 22, between about 15 and about 22, between about 16 and about 22, between about 17 and about 22, between about 18 and about 22, between about 19 and about 22, or between about 20 and about 22.

In some aspects, the payload comprises an antisense oligonucleotide. In certain aspects, the antisense oligonucleotide specifically binds to one or more genes and reduces an expression of the protein encoded by the one or more genes. In some aspects, the one or more genes comprise *STAT6, Kras, Nras, PMP22, C*/*EBPβ, STAT3, NLRP3,* or any combination thereof.

In some aspects, the payload in the loading buffer is at a concentration between about 100 µM and about 1000 µM). In some aspects, the payload in the loading buffer is at a concentration between about 100 µM and about 200 µM, between about 200 µM and about 300 µM, between about 300 µM and about 400 µM, between about 400 µM and about 500 µM, between about 500 µM and about 600 µM, between about 600 µM and about 700 µM, between about 700 µM and about 800 µM, between about 800 µM and about 900 µM, or between about 900 µM and 1000 µM. In certain aspects, the payload in the loading buffer is at a concentration of about 100 µM, about 200 µM, about 300 µM, about 400 µM, about 500 µM, about 600 µM, about 700 µM, about 800 µM, about 900 µM, or about 1000 µM. In some aspects, the payload in the loading buffer is at a concentration between about 1000 µM and about 2500 µM. In some aspects, the payload comprises the nucleotide sequence set forth in any one of SEQ ID NOs: 41 to 112, and the concentration of the payload is about 600 µM.

In some aspects, the payload is conjugated to an anchoring moiety. In certain aspects, the anchoring moiety a sterol, GM1, a lipid, a phospholipid, a vitamin, a small molecule, a peptide, or a combination thereof.

In some aspects, the anchoring moiety comprises at least 6 carbon atoms, at least 7 carbon atoms, at least 8 carbon atoms, at least 9 carbon atoms, at least 10 carbon atoms, at least 11 carbon atoms, at least 12 carbon atoms, at least 13 carbon atoms, at least 14 carbon atoms, at least 15 carbon atoms, at least 16 carbon atoms, at least 17 carbon atoms, at least 18 carbon atoms, at least 19 carbon atoms, at least 20 carbon atoms, at least 25 carbon atoms, at least 30 carbon atoms, at least 35 carbon atoms, at least 40 carbon atoms, at least 45 carbon atoms, at least 50 carbon atoms, at least 55 carbon atoms, at least 60 carbon atoms, at least 65 carbon atoms, at least 70 carbon atoms, at least 75 carbon atoms, at least 80 carbon atoms, at least 85 carbon atoms, or at least 90 carbon atoms.

In some aspects, the anchoring moiety comprises a sterol, a steroid, a hopanoid, a hydroxysteroid, a secosteroid, an analog thereof, or any combination thereof. In certain aspects, the anchoring moiety comprises ergosterol, 7-dehydrocholesterol, cholesterol, 24S-hydroxycholesterol, lanosterol, cycloartenol, fucosterol, saringosterol, campesterol, β-sitosterol, sitostanol, coprostanol, avenasterol, stigmasterol, or any combination thereof. In some aspects, the anchoring moiety is a cholesterol or derivative having a structure selected from the group consisting of 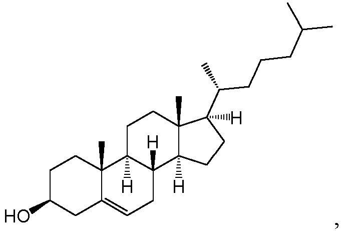 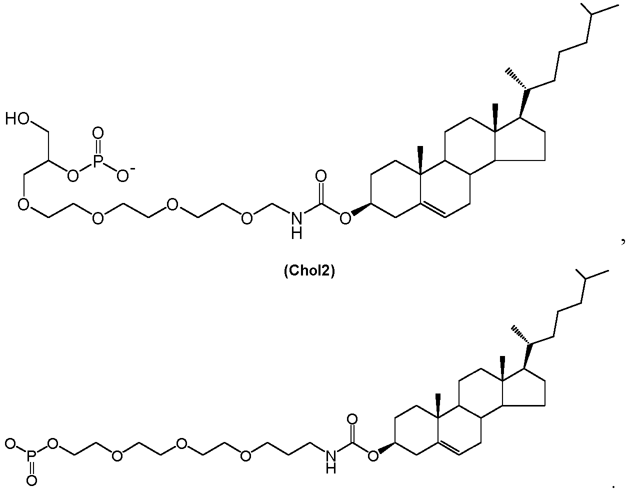 and
In some aspects, the anchoring moiety comprises a steroid, which is dihydrotestosterone, uvaol, hecigenin, diosgenin, progesterone, cortisol, or any combination thereof. In certain aspects, the anchoring moiety comprises a lipid.

In some aspects, the anchoring moiety comprises a C₂-C₆₀ chain. In certain aspects, the anchoring moiety comprises C₄-C₄₀, C₂-C₃₈, C₂-C₃₆, C₂-C₃₄, C₂-C₃₂, C₂-C₃₀, C₄-C₃₀, C₂-C₂₈, C₄-C₂₈, C₂- C₂₆, C₄-C₂₆, C₂-C₂₄, C₄-C₂₄, C₆-C₂₄, C₈-C₂₄, C₁₀-C₂₄, C₂-C₂₂, C₄-C₂₂, C₆-C₂₂, C₈-C₂₂, C₁₀-C₂₂, C₂-C₂₀, C₄-C₂₀, C₆-C₂₀, C₈-C₂₀, C₁₀-C₂₀, C₂-C₁₈, C₄-C₁₈, C₆-C₁₈, C₈-C₁₈, C₁₀-C₁₈, C₁₂-C₁₈, C₁₄-C₁₈, C₁₆-C₁₈, C₂-C₁₆, C₄-C₁₆, C₆-C₁₆, C₈-C₁₆, C₁₀-C₁₆, C₁₂-C₁₆, C₁₄-C₁₆, C₂-C₁₅, C₄-C₁₅, C₆-C₁₅, C₈-C₁₅, C₉-C₁₅, C₁₀-C₁₅, C₁₁-C₁₅, C₁₂-C₁₅, C₁₃-C₁₅, C₂-C₁₄, C₄-C₁₄, C₆-C₁₄, C₈-C₁₄, C₉-C₁₄, C₁₀-C₁₄, C₁₁-C₁₄, C₁₂-C₁₄, C₂-C₁₃, C₄-C₁₃, C₆-C₁₃, C₇-C₁₃, C₈-C₁₃, C₉-C₁₃, C₁₀-C₁₃, C₁₀-C₁₃, C₁₁-C₁₃, C₂-C₁₂, C₄-C₁₂, C₆-C₁₂, C₇-C₁₂, C₈-C₁₂, C₉-C₁₂, C₁₀-C₁₂, C₂-C₁₁, C₄-C₁₁, C₆-C₁₁, C₇-C₁₁, C₈-C₁₁, C₉-C₁₁, C₂-C₁₀, C₄-C₁₀, C₂-C₉, C₄-C₉, C₂-C₈, C₂-C₇, C₄-C₇, C₂-C₆, or C₄-C₆ chain.

In some aspects, the anchoring moiety comprises a straight chain fatty acid, a branched fatty acid, an unsaturated fatty acid, a monounsaturated fatty acid, a polyunsaturated fatty acid, a hydroxyl fatty acid, a polycarboxylic acid, or any combination thereof.

In some aspects, the anchoring moiety comprises a straight chain fatty acid, which is butyric acid, caproic acid, caprylic acid, capric acid, lauric acid, myristic acid, palmitic acid, stearic acid, arachic acid, behenic acid, lignoceric acid, hexacosanoic acid, octacosanoic acid, triacontanoic acid and n-dotriacontanoic acid, and those having an odd number of carbon atoms, such as propionic acid, n-valeric acid, enanthic acid, pelargonic acid, hendecanoic acid, tridecanoic acid, pentadecanoic acid, heptadecanoic acid, nonadecanoic acid, heneicosanoic acid, tricosanoic acid, pentacosanoic acid, heptacosanoic acid, or any combination thereof. In some aspects, the anchoring moiety comprises a branched fatty acid, which is isobutyric acid, isocaproic acid, isocaprylic acid, isocapric acid, isolauric acid, 11-methyldodecanoic acid, isomyristic acid, 13-methyl-tetradecanoic acid, isopalmitic acid, 15-methyl-hexadecanoic acid, isostearic acid, 17-methyloctadecanoic acid, isoarachic acid, 19-methyl-eicosanoic acid, α-ethyl-hexanoic acid, α-hexyldecanoic acid, α-heptylundecanoic acid, 2-decyltetradecanoic acid, 2-undecyltetradecanoic acid, 2-decylpentadecanoic acid, 2-undecylpentadecanoic acid, Fine oxocol 1800 acid (product of Nissan Chemical Industries, Ltd.), anteiso fatty acids terminating with an isobutyl group, such as 6-methyl-octanoic acid, 8-methyl-decanoic acid, 10-methyl-dodecanoic acid, 12-methyl-tetradecanoic acid, 14-methyl-hexadecanoic acid, 16-methyl-octadecanoic acid, 18-methyl-eicosanoic acid, 20-methyl-docosanoic acid, 22-methyl-tetracosanoic acid, 24-methyl-hexacosanoic acid, and 26-methyloctacosanoic acid, or any combination thereof. In some aspects, the anchoring moiety comprises an unsaturated fatty acid, which is 4-decenoic acid, caproleic acid, 4-dodecenoic acid, 5-dodecenoic acid, lauroleic acid, 4-tetradecenoic acid, 5-tetradecenoic acid, 9-tetradecenoic acid, palmitoleic acid, 6-octadecenoic acid, oleic acid, 9-octadecenoic acid, 11-octadecenoic acid, 9-eicosenoic acid, cis-11-eicosenoic acid, cetoleic acid, 13-docosenoic acid, 15-tetracosenoic acid, 17-hexacosenoic acid, 6,9,12,15-hexadecatetraenoic acid, linoleic acid, linolenic acid, α-eleostearic acid, β-eleostearic acid, punicic acid, 6,9,12,15-octadecatetraenoic acid, parinaric acid, 5,8,11,14-eicosatetraenoic acid, 5,8,11,14,17-eicosapentaenoic acid, 7,10,13,16,19-docosapentaenoic acid, 4,7,10,13,16,19-docosahexaenoic acid, or any combination thereof. In some aspects, the anchoring moiety comprises a hydroxy fatty acid, which is α-hydroxylauric acid, α-hydroxymyristic acid, α-hydroxypalmitic acid, α-hydroxystearic acid, ω-hydroxylauric acid, α-hydroxyarachic acid, 9-hydroxy-12-octadecenoic acid, ricinoleic acid, α-hydroxybehenic acid, 9-hydroxy-trans-10,12-octadecadienic acid, kamolenic acid, ipurolic acid, 9,10-dihydroxystearic acid, 12-hydroxystearic acid, or any combination thereof. In some aspects, the anchoring moiety comprises a polycarboxylic acid, which is oxalic acid, malonic acid, succinic acid, glutaric acid, adipic acid, pimelic acid, suberic acid, azelaic acid, sebacic acid, D,L-malic acid, or any combination thereof.

In some aspects, the anchoring moiety comprises a phospholipid. In certain aspects, the phospholipid is phosphatidyl choline, phosphatidyl ethanolamine, phosphatidyl glycerol, phosphatidyl serine, phosphatidic acid, 2 lysophosphatidyl choline, sphingomyelin, or any combination thereof.

In some aspects, the anchoring moiety comprises a vitamin. In some aspects, the anchoring moiety comprises vitamin D, vitamin K, niacin, pyridoxine, vitamin E, or any combination thereof.

In some aspects, the anchoring moiety further comprises a linker between the payload and the anchoring moiety.

In some aspects, the linker comprises a non-cleavable linker. In some aspects, the non-cleavable linker comprises polyethylene glycol (PEG), glycerol, alkyl, phosphorothioate, succinimide, maleimide, or any combination thereof. In some aspects, the non-cleavable linker comprises polyethylene glycol (PEG) characterized by a formula R3-(O-CH₂-CH₂)ₙ- or R3-(0-CH₂-CH₂)ₙ-O-, wherein R3 being hydrogen, methyl or ethyl and n is an integer between 2 and 200. In some aspects, the non-cleavable linker comprises diethylene glycol, triethylene glycol, tetraethylene glycol (TEG), hexaethylene glycol (HEG), pentaethylene glycol, or any combination thereof. In some aspects, wherein the linker comprises a polyglycerol (PG) having the formula ((R3-O-(CH₂-CHOH-CH₂O)ₙ-), wherein R3 is hydrogen, methyl or ethyl, and n is an integer between 3 and 200. In some aspects, the linker comprises a diglycerol, triglycerol, tetraglycerol (TG), pentaglycerol, a hexaglycerol (HG), or any combination thereof. In some aspects, the linker comprises alkyl.

In some aspects, the linker comprises a cleavable linker. In some aspects, cleavable linker is a redox cleavable linker, a reactive oxygen species cleavable linker, a pH dependent cleavable linker, an enzymatic cleavable linker, a protease cleavable linker, an esterase cleavable linker, an oxidoreductate cleavable linker, a phospholipase cleavable linker, a phosphatase cleavable linker, a photoactivated cleavable linker, a self-immolative linker, or any combination thereof. In some aspects, the cleavable linker is a self-immolative linker. In some aspects, the cleavable linker is a cinnamyl group, a naphthyl group, a biphenyl group, a heterocyclic ring, a homoaromatic group, coumarin, furan, thiophene, thiazole, oxazole, isoxazole, pyrrole, pyrazole, pyridine, imidazone, triazole, or any combination thereof.

In some aspects, wherein the linker has the formula:

-Aₐ-Y_{y}-

wherein each -A- is independently an amino acid unit, a is independently an integer from 1 to 12; -Y- is a spacer unit, and y is 0, 1, or 2. In some aspects, -Aₐ- is a dipeptide, a tripeptide, a tetrapeptide, a pentapeptide, or a hexapeptide. In some aspects, a is 2 and -Aₐ- is selected from the group consisting of valine-alanine, valine-citrulline, glutamic acid-valine-citrulline, phenylalanine-lysine, N-methylvaline-citrulline, glycine-histidine-leucine-glycine, cyclohexylalanine-lysine, and beta-alanine-lysine. In some aspects, -Aₐ- is valine-alanine or valine-citrulline or glutamic acid-valine-citrulline. In some aspects, -Aₐ- is arginine, lysine, or both. In some aspects, y is 1. In some aspects, -Y- is a self-immolative spacer. In some aspects, - Y_{y}- has the formula (V): 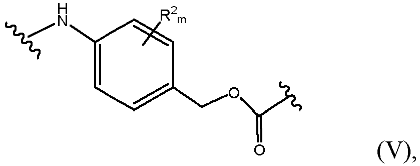 wherein each R² is independently C₁₋₈ alkyl, -O-(C₁₋₈ alkyl), halogen, nitro, or cyano; and m is an integer from 0 to 4. In some aspects, m is 0, 1, or 2. In some aspects, m is 0. In some aspects, the cleavable linker is valine-alanine-p-aminobenzylcarbamate or valine-citrulline-p-aminobenzylcarbamate.

In some aspects, the anchoring moiety comprises:

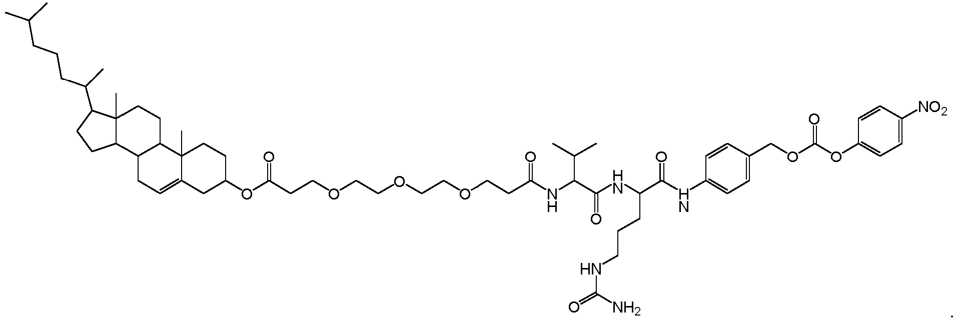

In some aspects, the method comprises (i) a payload comprises the nucleotide sequence set forth in any one of SEQ ID NOs: 41 to 112, and (ii) a linker selected from the linker combinations of TABLE 1 or TABLE 2.

In some aspects, the payload is conjugated to a cholesterol. In some aspects, the payload comprises 5'GbsCbsAbsdAsdGsdAsdTs(5MdC)s(5MdC)s(5MdC)sdGsdGsdAsdTsdTs(5MdC)sdGsGbsTbs Cb3', wherein Nb is an LNA, dN is a DNA, (5MdC) is 5-Methyl-dC, s is phosphorothioate, TEG is triethylene glycol, and HEG is hexaethylene glycol. In some aspects, the payload is linked to the cholesterol by TEG-HEG.

In some aspects, the extracellular vesicle is at a concentration of at least about 1x10¹¹, at least about 2x10¹¹, at least about 3x10¹¹, at least about 4x10¹¹, at least about 5x10¹¹, at least about 6x10¹¹, at least about 7x10¹¹, at least about 8x10¹¹, at least about 9x10¹¹, at least about 1x10¹², at least about 2x10¹², at least about 3x10¹², at least about 4x10¹², at least about 5x10¹², at least about 6x10¹², at least about 7x10¹², at least about 8x10¹², at least about 9x10¹², at least about 1x10¹³, at least about 2x10¹³, at least about 3x10¹³, at least about 4x10¹³, at least about 5x10¹³, at least about 6x10¹³, at least about 7x10¹³, at least about 8x10¹³, at least about 9x10¹³, or at least about 1x10¹⁴ particles/mL. In some aspects, the extracellular vesicle is at a concentration between about 1x10¹² and about 5x10¹³ particles/mL, about 1x10¹² and about 4x10¹³ particles/mL, about 1x10¹² and about 3x10¹³ particles/mL, about 1x10¹² and about 2x10¹³ particles/mL, about 2x10¹² and about 5x10¹³ particles/mL, about 2x10¹² and about 4x10¹³ particles/mL, about 2x10¹² and about 3x10¹³ particles/mL, about 2x10¹² and about 2x10¹³ particles/mL, about 3x10¹² and about 5x10¹³ particles/mL, about 3x10¹² and about 4x10¹³ particles/mL, about 3x10¹² and about 3x10¹³ particles/mL, about 3x10¹² and about 2x10¹³ particles/mL, about 4x10¹² and about 5x10¹³ particles/mL, about 4x10¹² and about 4x10¹³ particles/mL, about 4x10¹² and about 3x10¹³ particles/mL, about 4x10¹² and about 2x10¹³ particles/mL, about 5x10¹² and about 5x10¹³ particles/mL, about 5x10¹² and about 4x10¹³ particles/mL, about 5x10¹² and about 3x10¹³ particles/mL, or about 5x10¹² and about 2x10¹³ particles/mL. In some aspects, the extracellular vesicle is at a concentration between about 5x10¹² and about 2x10¹³ particles/mL. In some aspects, the extracellular vesicle is at a concentration of about 1x10¹³ particles/mL.

In some aspects, the extracellular vesicle further comprises a protein in the membrane of the extracellular vesicle. In some aspects, the protein comprises prostaglandin F2 receptor negative regulator (the PTGFRN protein); basigin (the BSG protein); immunoglobulin superfamily member 2 (the IGSF2 protein); immunoglobulin superfamily member 3 (the IGSF3 protein); immunoglobulin superfamily member 8 (the IGSF8 protein); integrin beta-1 (the ITGB1 protein); integrin alpha-4 (the ITGA4 protein); 4F2 cell-surface antigen heavy chain (the SLC3A2 protein); a class of ATP transporter proteins (the ATP1A1, ATP1A2, ATP1A3, ATP1A4, ATP1B3, ATP2B1, ATP2B2, ATP2B3, ATP2B4 proteins); a functional fragment thereof; and any combination thereof. In some aspects, the protein comprises a PTGFRN protein or a functional fragment thereof.

Provided herein is an extracellular vesicle prepared by any one of the inventive methods disclosed herein.

In some aspects, the extracellular vesicle comprising payloads on the external surface of the extracellular vesicle, wherein the number of payloads on the surface of the extracellular vesicle is at least about 1000, at least about 2000, at least about 3000, at least about 4000, at least about 5000, at least about 6000, at least about 7000, at least about 8000, at least about 9000, at least about 10,000, at least about 11,000, at least about 12000, at least about 13,000, at least about 14,000, at least about 15,000, at least about 16,000, at least about 17,000, at least about 18,000, at least about 20,000, at least about 22,000, at least about 24,000, at least about 26,000, at least about 28,000, at least about 30,000, at least about 35,000, or at least about 40,000. In some aspects, the number of payloads on the surface of the extracellular vesicle is between about 5,000 and about 20,000, about 6,000 and about 20,000, about 7,000 and about 20,000, about 8,000 and about 20,000, about 9,000 and about 20,000, about 10,000 and about 20,000, about 5,000 and about 18,000, about 6,000 and about 18,000, about 7,000 and about 18,000, about 8,000 and about 18,000, about 9,000 and about 18,000, about 10,000 and about 18,000, about 5,000 and about 15,000, about 6,000 and about 15,000, about 7,000 and about 15,000, about 8,000 and about 15,000, about 9,000 and about 15,000, or about 10,000 and about 15,000. In some aspects, the number of payloads on the surface of the extracellular vesicle is between about 10,000 and about 15,000. In some aspects, the payload comprises the nucleotide sequence set forth in any one of SEQ ID NOs: 41 to 112.

In some aspects, the payload of an extracellular vesicle disclosed herein is conjugated to a sterol, GM1, a lipid, a phospholipid, a vitamin, a small molecule, a peptide, or a combination thereof. In some aspects, the payload is conjugated to a cholesterol. In some aspects, the payload comprises the structure: 5'GbsCbsAbsdAsdGsdAsdTs(5MdC)s(5MdC)s(5MdC)sdGsdGsdAsdTsdTs(5MdC)sdGsGbsTbs Cb3', wherein Nb is an LNA, dN is a DNA, (5MdC) is 5-Methyl-dC, and s is phosphorothioate.

Disclosed is a population of extracellular vesicles, comprising any of the extracellular vesicles described herein. In some aspects, at least about 30%, at least about 40%, at least about 50%, at least about 60%, at least about 70%, at least about 80%, at least about 90%, at least about 95%, at least about 96%, at least about 97%, at least about 98%, at least about 99% or about 100% of the extracellular vesicles are the extracellular vesicle of any one of the extracellular vesicles described herein.

Disclosed is a composition comprising any of the extracellular vesicles or population of extracellular vesicles described herein. Provided herein is a pharmaceutical composition comprising the inventive extracellular vesicles described herein.

Also provided herein is a method of treating a disease or condition in a subject in need thereof comprising administering the extracellular vesicle or the population of extracellular vesicles described herein. The references to the methods of treatment by therapy or in vivo diagnosis methods within this description are to be interpreted as references to compounds, pharmaceutical compositions and medicaments of the present invention for use in those methods.

### BRIEF DESCRIPTION OF THE DRAWINGS/FIGURES

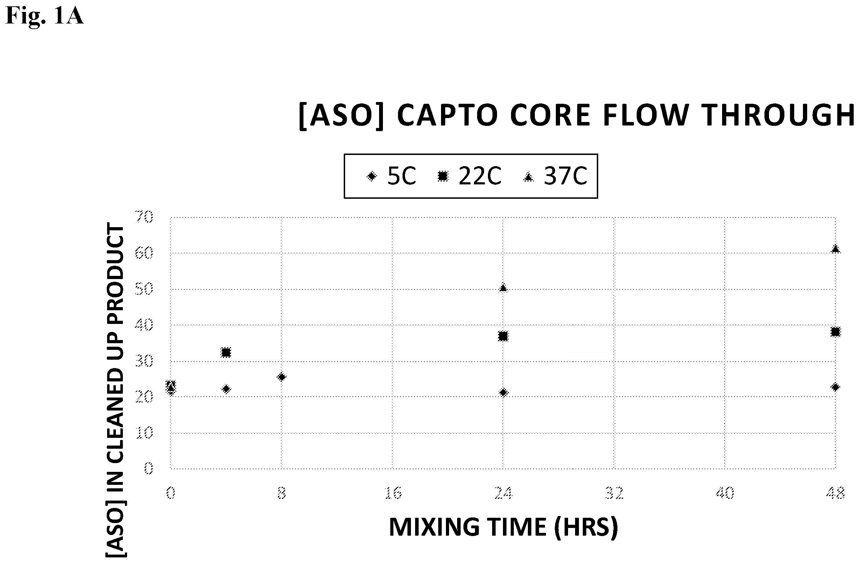

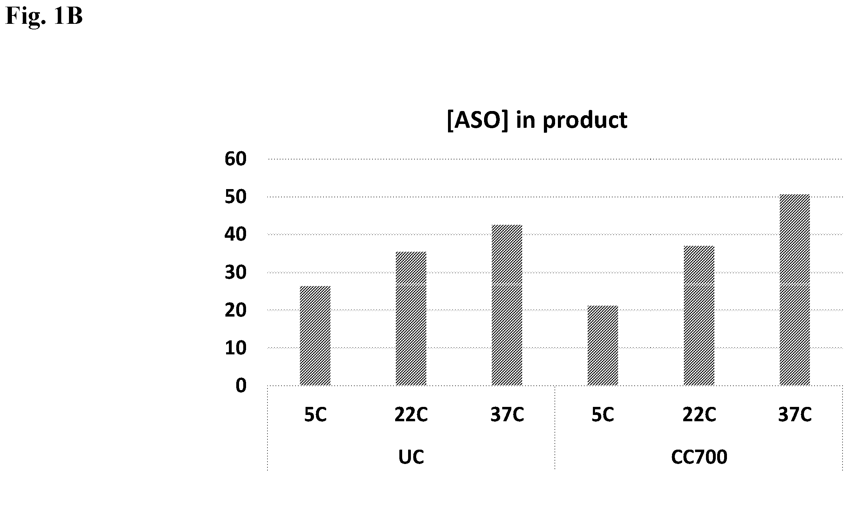

FIG. 1A shows the effects of increasing time and temperature on ASO loading of extracellular vesicles. FIG. 1B shows the concentration of ASOs loaded in EVs after cleanup with ultracentrifugation (UC) and chromatography using Capto Core 700 resin (CC700).

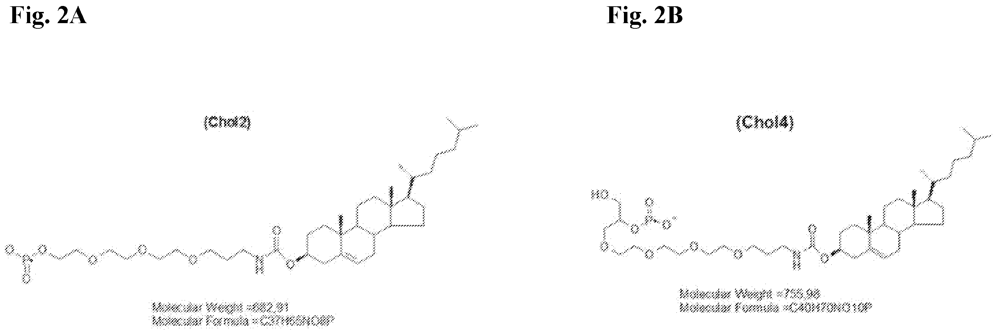

FIGs. 2A and 2B provide illustration of two exemplary cholesterol moieties that can be used to conjugate a payload (*e.g.,* ASO) disclosed herein. FIG. 2A provides the structure for Chol2. FIG. 2B provides the structure for Chol4.

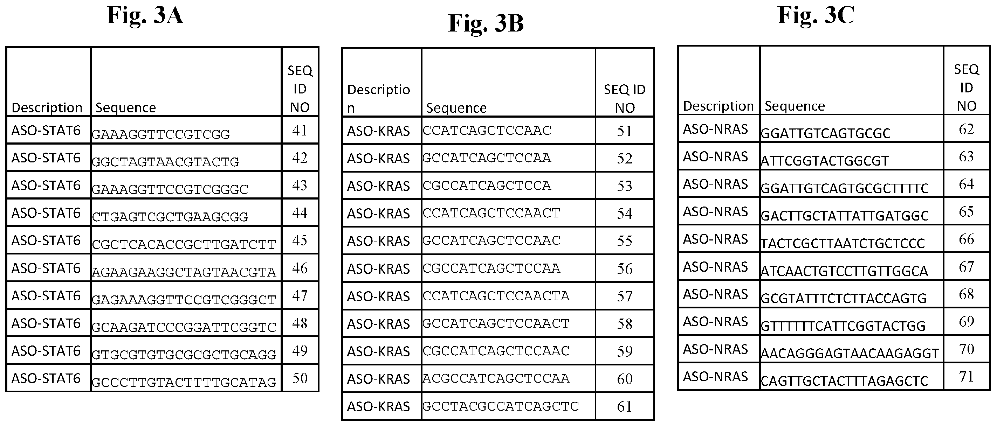

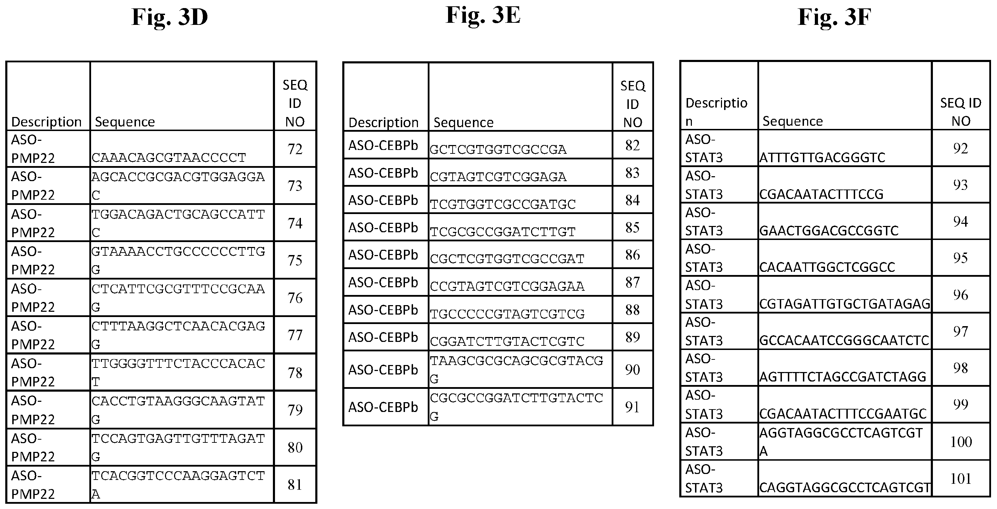

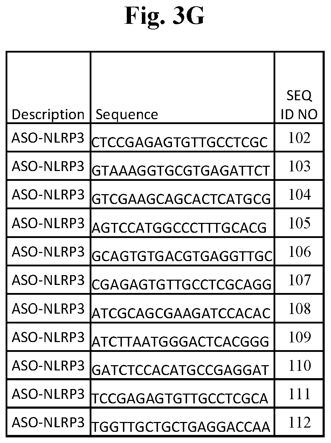

FIGs. 3A, 3B, 3C, 3D, 3E, 3F, and 3G provide tables listing the sequences for exemplary ASOs that can be loaded onto EVs using the loading methods provided herein. In FIG. 3A, the ASOs target the *STAT6* transcript. In FIG. 3B, the ASOs target the *KRAS* transcript. In FIG. 3C, the ASOs target the *NRAS* transcript. In FIG. 3D, the ASOs target the *PMP22* transcript. In FIG. 3E, the ASOs target the *C*/*EBPβ* transcript. In FIG. 3F, the ASOs target the *STAT3* transcript. In FIG. 3G, the ASOs target the *NLRP3* transcript.

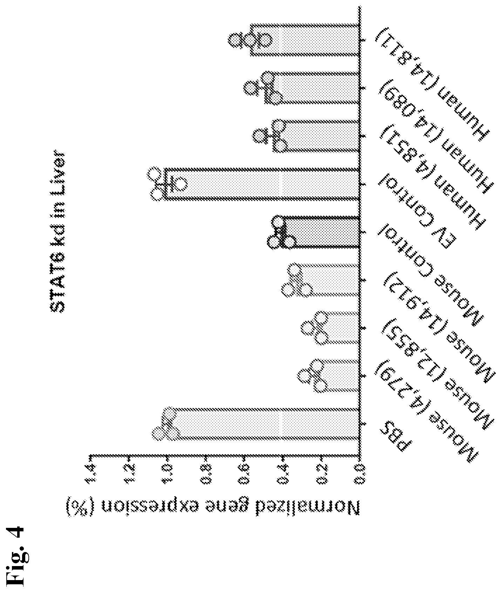

FIG. 4 shows the knockdown of STAT6 expression in the liver after treatment with EVs loaded with different amounts of mouse and human STAT6-targeting ASOs. The specific ASO density of the different EVs are shown in parentheses. STAT6 expression is shown normalized to the control (PBS-treated alone or treated with the vehicle control).

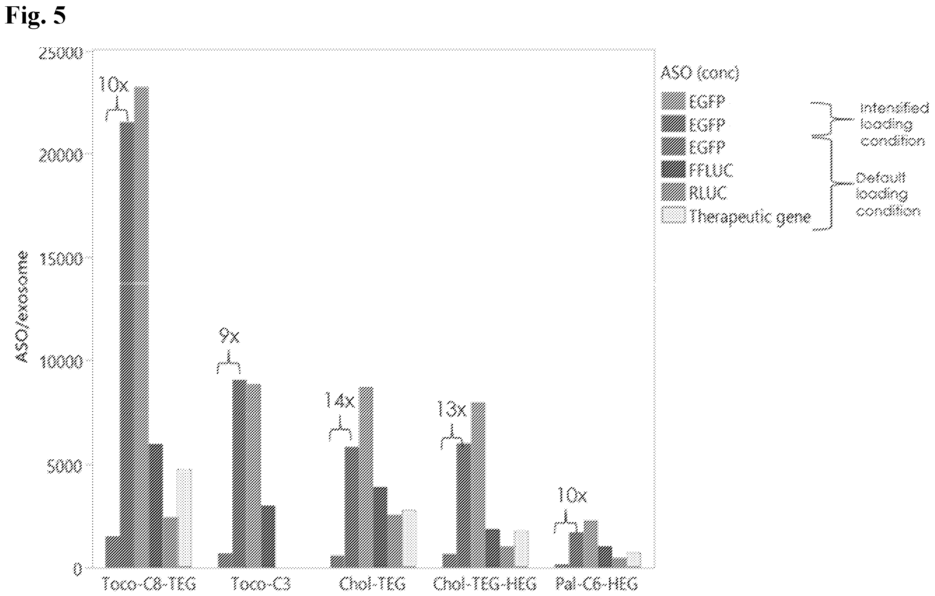

FIG. 5 provides a comparison of the loading density (ASOs per EV) for four different ASO sequences synthesized with different lipid-linkers. The ASOs targeted one of the following: green fluorescent protein ("EGFP"), firefly luciferase ("FFLUC"), Renilla luciferase ("RLUC"), and a therapeutic gene ("therapeutic gene"). Toco= tocopherol, Chol = cholesterol, Pal=palmitate, C6=hexamethylene, C8=octamethylene, TEG =tetraethylene glycol, HEG = hexaethylene glycol.

### DETAILED DESCRIPTION

The present disclosure is directed to methods of loading a payload (*e.g.,* antisense oligonucleotides) in an EV (*e.g.,* on the exterior surface of the EVs), comprising modulating (*e.g.,* increasing or decreasing) one or more parameters of the loading process (referred to herein as "loading parameters"). Generally, the one or more parameters comprise: salt concentration, loading temperature, loading duration, payload feed concentration, EV feed concentration, or combinations thereof. As demonstrated herein, modulating the one or more parameters can increase the amount of the payload that is loaded in the EVs (*e.g.,* on the exterior surface of the EVs). In one aspect, the invention relates to a method of loading a payload to an extracellular vesicle (EV) comprising mixing the payload with the EV
(i) in a loading buffer comprising a salt at a concentration between about 110 mM to lower than about 200 mM;
(ii) at a loading temperature higher than 4 °C; and
(iii) for a loading duration longer than about one hour,
wherein the loading buffer comprising the salt increases the loading of the payload to the EV by at least about 2 fold compared to a corresponding loading buffer without the salt or with a lower concentration of the salt. Non-limiting examples of the various aspects are shown in the present disclosure.

Before the present disclosure is described in greater detail, it is to be understood that this invention is not limited to the particular compositions or process steps described, as such can, of course, vary. Any recited method can be carried out in the order of events recited or in any other order which is logically possible.

The headings provided herein are not limitations of the various aspects of the disclosure, which can be defined by reference to the specification as a whole. It is also to be understood that the terminology used herein is for the purpose of describing particular aspects only, and is not intended to be limiting, since the scope of the present disclosure will be limited only by the appended claims.

Accordingly, the terms defined immediately below are more fully defined by reference to the specification in its entirety.

### I. Definitions

In order that the present description can be more readily understood, certain terms are first defined. Additional definitions are set forth throughout the detailed description.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein. It is further noted that the claims can be drafted to exclude any optional element. As such, this statement is intended to serve as antecedent basis for use of such exclusive terminology as "solely," "only" and the like in connection with the recitation of claim elements, or use of a negative limitation.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the disclosure. Thus, ranges recited herein are understood to be shorthand for all of the values within the range, inclusive of the recited endpoints. For example, a range of 1 to 10 is understood to include any number, combination of numbers, or sub-range from the group consisting of 1, 2, 3, 4, 5, 6, 7, 8, 9, and 10.

Where a value is explicitly recited, it is to be understood that values which are about the same quantity or amount as the recited value are also within the scope of the disclosure. Where a combination is disclosed, each subcombination of the elements of that combination is also specifically disclosed and is within the scope of the disclosure. Conversely, where different elements or groups of elements are individually disclosed, combinations thereof are also disclosed. Where any element of a disclosure is disclosed as having a plurality of alternatives, examples of that disclosure in which each alternative is excluded singly or in any combination with the other alternatives are also hereby disclosed; more than one element of a disclosure can have such exclusions, and all combinations of elements having such exclusions are hereby disclosed.

Nucleotides are referred to by their commonly accepted single-letter codes. Unless otherwise indicated, nucleotide sequences are written left to right in 5' to 3' orientation. Nucleotides are referred to herein by their commonly known one-letter symbols recommended by the IUPAC-IUB Biochemical Nomenclature Commission. Accordingly, A represents adenine, C represents cytosine, G represents guanine, T represents thymine, U represents uracil.

Amino acid sequences are written left to right in amino to carboxy orientation. Amino acids are referred to herein by either their commonly known three letter symbols or by the one-letter symbols recommended by the IUPAC-IUB Biochemical Nomenclature Commission.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, *e.g.,* 10 percent, up or down (higher or lower).

The terms "administration," "administering," and grammatical variants thereof refer to introducing a composition, such as an EV of the present disclosure, into a subject via a pharmaceutically acceptable route. The introduction of a composition, such as an EV of the present disclosure, into a subject is by any suitable route, including intratumorally, orally, pulmonarily, intranasally, parenterally (intravenously, intra-arterially, intramuscularly, intraperitoneally, or subcutaneously), rectally, intralymphatically, intrathecally, periocularly or topically. Administration includes self-administration and the administration by another. A suitable route of administration allows the composition or the agent to perform its intended function. For example, if a suitable route is intravenous, the composition is administered by introducing the composition or agent into a vein of the subject.

As used herein, the term "agonist" refers to a molecule that binds to a receptor and activates the receptor to produce a biological response. Receptors can be activated by either an endogenous or an exogenous agonist. Non-limiting examples of endogenous agonist include hormones, neurotransmitters, and cyclic dinucleotides. Non-limiting examples of exogenous agonist include drugs, small molecules, and cyclic dinucleotides. The agonist can be a full, partial, or inverse agonist.

The term "amino acid substitution" refers to replacing an amino acid residue present in a parent or reference sequence (*e.g.,* a wild type sequence) with another amino acid residue. An amino acid can be substituted in a parent or reference sequence *(e.g.,* a wild type polypeptide sequence), for example, via chemical peptide synthesis or through recombinant methods known in the art. Accordingly, a reference to a "substitution at position X" refers to the substitution of an amino acid present at position X with an alternative amino acid residue. In some aspects, substitution patterns can be described according to the schema AnY, wherein A is the single letter code corresponding to the amino acid naturally or originally present at position n, and Y is the substituting amino acid residue. In some aspects, substitution patterns can be described according to the schema An(YZ), wherein A is the single letter code corresponding to the amino acid residue substituting the amino acid naturally or originally present at position n, and Y and Z are alternative substituting amino acid residues that can replace A.

The terms "anion" and "cation" refer to negatively and positively charged ions, respectively. A "divalent" cation refers to a cation with a valence of 2+. Examples of divalent cations include, but are not limited to, Ca²⁺, Mg²⁺, Co²⁺, Ni²⁺, Zn²⁺, Ba²⁺, Sr²⁺, Al²⁺, Ag²⁺, Cu²⁺, and Mn²⁺. A "monovalent" cation refers to a cation with a valence of 1+. Examples of monovalent cations include, but are not limited to, Li⁺, K⁺, Na⁺, NH₄⁺, Cu⁺. Examples of anions include, but are not limited to, SCN⁻, Cl⁻, SO₄⁻, and PO₄. In some aspects, the anion and/or the cation (*e.g.,* monovalent cation or divalent cation) can be present in a salt, *e.g.,* a mixture of at least one anion and at least one cation of complementary valences. Any salt comprising an anion or a cation disclosed herein can be used in the methods disclosed herein.

As used herein, the term "antagonist" refers to a molecule that blocks or dampens an agonist mediated response rather than provoking a biological response itself upon bind to a receptor. Many antagonists achieve their potency by competing with endogenous ligands or substrates at structurally defined binding sites on the receptors. Non-limiting examples of antagonists include alpha blockers, beta-blocker, and calcium channel blockers. The antagonist can be a competitive, non-competitive, or uncompetitive antagonist.

As used herein, the term "antibody" encompasses an immunoglobulin whether natural or partly or wholly synthetically produced, and fragments thereof. The term also covers any protein having a binding domain that is homologous to an immunoglobulin binding domain. "Antibody" further includes a polypeptide comprising a framework region from an immunoglobulin gene or fragments thereof that specifically binds and recognizes an antigen. Use of the term antibody is meant to include whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, *e.g.,* scFv, (scFv)₂, Fab, Fab', and F(ab')₂, F(ab1)₂, Fv, dAb, and Fd fragments, diabodies, and antibody-related polypeptides. Antibody includes bispecific antibodies and multispecific antibodies so long as they exhibit the desired biological activity or function. In some aspects of the present disclosure, the biologically active molecule is an antibody or a molecule comprising an antigen binding fragment thereof.

The terms "antibody-drug conjugate" and "ADC" are used interchangeably and refer to an antibody linked, *e.g.,* covalently, to a therapeutic agent (sometimes referred to herein as agent, drug, or active pharmaceutical ingredient) or agents. In some aspects of the present disclosure, the biologically active molecule is an antibody-drug conjugate.

As used herein, the term "approximately," as applied to one or more values of interest, refers to a value that is similar to a stated reference value. In certain aspects, the term "approximately" refers to a range of values that fall within 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The term "aryl" refers to a carbocyclic aromatic group. Examples of aryl groups include, but are not limited to, phenyl, naphthyl and anthracenyl. A carbocyclic aromatic group can be unsubstituted or substituted with one or more groups including, but not limited to, -C₁₋₈ alkyl, -O-(C₁₋₈ alkyl), -aryl, -C(O)R', -OC(O)R', -C(O)OR', -C(O)NH₂, -C(O)NHR', -C(O)N(R')₂-, -NHC(O)R', -S(O)₂R', -S(O)R', -OH, -halogen, -N₃, -NH₂, -NH(R'), -N(R')₂ and -CN, wherein each R' is independently H, -C₁₋₈ alkyl, or aryl.

The term "arylene" refers to an aryl group which has two covalent bonds and can be in the ortho, meta, or para configurations as shown in the following structures: 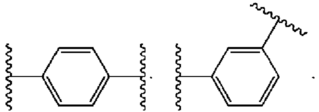 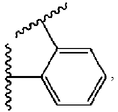 in which the phenyl group can be unsubstituted or substituted with up to four groups including, but not limited to, -C₁₋₈ alkyl, -O-(C₁₋₈ alkyl), -aryl, -C(O)R', -OC(O)R', -C(O)OR', -C(O)NH₂, - C(O)NHR', -C(O)N(R')₂-, -NHC(O)R', -S(O)₂R', -S(O)R', -OH, -halogen, -N₃, -NH₂, -NH(R'), - N(R')₂ and -CN, wherein each R' is independently H, -C₁₋₈ alkyl, or aryl.

As used herein, the term "arylalkyl" or "aralkyl" is meant to include those radicals in which an aryl group or heteroaryl group is attached to an alkyl group to create the radicals - alkyl-aryl and -alkyl-heteroaryl, wherein alkyl, aryl and heteroaryl are defined herein. Exemplary "arylalkyl" or "aralkyl" groups include benzyl, phenethyl, pyridylmethyl and the like.

As, used herein, the term "aryloxy" refers to the group -O-aryl, where aryl is as defined herein. In certain aspects, the aryl portion of the aryloxy group is phenyl or naphthyl.

The term "heteroaryl" or "heteroaromatic" refers to a polyunsaturated, 5-, 6- or 7-membered aromatic moiety containing at least one heteroatom (*e.g.,* 1 to 5 heteroatoms, such as 1-3 heteroatoms) selected from N, O, S, Si and B (for example, N, O and S), wherein the nitrogen and sulfur atoms are optionally oxidized, and the nitrogen atom(s) are optionally quaternized. The "heteroaryl" group can be a single ring or be fused to other aryl, heteroaryl, cycloalkyl or heterocycloalkyl rings (*e.g.,* from 1 to 3 other rings). When the "heteroaryl" group includes a fused aryl, cycloalkyl or heterocycloalkyl ring, then the "heteroaryl" group is attached to the remainder of the molecule via the heteroaryl ring. A heteroaryl group can be attached to the remainder of the molecule through a carbon- or heteroatom.

In some aspects, the heteroaryl group has from 4 to 10 carbon atoms and from 1 to 5 heteroatoms selected from O, S and N. Non-limiting examples of heteroaryl groups include pyridyl, pyrimidinyl, quinolinyl, benzothienyl, indolyl, indolinyl, pyridazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, triazolyl, tetrazolyl, isothiazolyl, naphthyridinyl, isochromanyl, chromanyl, tetrahydroisoquinolinyl, isoindolinyl, isobenzotetrahydrofuranyl, isobenzotetrahydrothienyl, isobenzothienyl, benzoxazolyl, pyridopyridyl, benzotetrahydrofuranyl, benzotetrahydrothienyl, purinyl, benzodioxolyl, triazinyl, pteridinyl, benzothiazolyl, imidazopyridyl, imidazothiazolyl, dihydrobenzisoxazinyl, benzisoxazinyl, benzoxazinyl, dihydrobenzisothiazinyl, benzopyranyl, benzothiopyranyl, chromonyl, chromanonyl, pyridyl-N-oxide, tetrahydroquinolinyl, dihydroquinolinyl, dihydroquinolinonyl, dihydroisoquinolinonyl, dihydrocoumarinyl, dihydroisocoumarinyl, isoindolinonyl, benzodioxanyl, benzoxazolinonyl, pyrrolyl N-oxide, pyrimidinyl N-oxide, pyridazinyl N-oxide, pyrazinyl N-oxide, quinolinyl N-oxide, indolyl N-oxide, indolinyl N-oxide, isoquinolyl N-oxide, quinazolinyl N-oxide, quinoxalinyl N-oxide, phthalazinyl N-oxide, imidazolyl N-oxide, isoxazolyl N-oxide, oxazolyl N-oxide, thiazolyl N-oxide, indolizinyl N-oxide, indazolyl N-oxide, benzothiazolyl N-oxide, benzimidazolyl N-oxide, pyrrolyl N-oxide, oxadiazolyl N-oxide, thiadiazolyl N-oxide, triazolyl N-oxide, tetrazolyl N-oxide, benzothiopyranyl S-oxide, benzothiopyranyl S,S-dioxide. Exemplary heteroaryl groups include imidazolyl, pyrazolyl, thiadiazolyl, triazolyl, isoxazolyl, isothiazolyl, imidazolyl, thiazolyl, oxadiazolyl, and pyridyl. Other exemplary heteroaryl groups include 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, pyridin-4-yl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable aryl group substituents described below.

As used herein, the term "alkyl," by itself or as part of another substituent, means, unless otherwise stated, a straight or branched chain hydrocarbon radical having the number of carbon atoms designated (*e.g.,* C₁-C₁₀ means one to ten carbon atoms). Typically, an alkyl group will have from 1 to 24 carbon atoms, for example having from 1 to 10 carbon atoms, from 1 to 8 carbon atoms or from 1 to 6 carbon atoms. A "lower alkyl" group is an alkyl group having from 1 to 4 carbon atoms. The term "alkyl" includes di- and multivalent radicals. For example, the term "alkyl" includes "alkylene" wherever appropriate, *e.g.,* when the formula indicates that the alkyl group is divalent or when substituents are joined to form a ring. Non-limiting examples of alkyl radicals include, but are not limited to, methyl, ethyl, *n*-propyl, *iso*-propyl, *n*-butyl, *tert-*butyl*, iso*butyl, *sec*-butyl, as well as homologs and isomers of, for example, *n*-pentyl, *n*-hexyl, *n*-heptyl and *n*-octyl.

The term "alkylene" by itself or as part of another substituent means a divalent (diradical) alkyl group, wherein alkyl is defined herein. "Alkylene" is exemplified, but not limited, by -CH₂CH₂CH₂CH₂-. In some aspects, an "alkylene" group comprises 1 to 24 carbon atoms, for example, having 10 or fewer carbon atoms (*e.g.,* 1 to 8 or 1 to 6 carbon atoms). A "lower alkylene" group is an alkylene group having from 1 to 4 carbon atoms.

The term "alkenyl" by itself or as part of another substituent refers to a straight or branched chain hydrocarbon radical having from 2 to 24 carbon atoms and at least one double bond. A typical alkenyl group has from 2 to 10 carbon atoms and at least one double bond. In certain aspects, alkenyl groups have from 2 to 8 carbon atoms or from 2 to 6 carbon atoms and from 1 to 3 double bonds. Exemplary alkenyl groups include vinyl, 2-propenyl, 1-but-3-enyl, crotyl, 2-(butadienyl), 2,4-pentadienyl, 3-(1,4-pentadienyl), 2-isopentenyl, 1-pent-3-enyl, 1-hex-5-enyl and the like.

The term "alkynyl" by itself or as part of another substituent refers to a straight or branched chain, unsaturated or polyunsaturated hydrocarbon radical having from 2 to 24 carbon atoms and at least one triple bond. A typical "alkynyl" group has from 2 to 10 carbon atoms and at least one triple bond. In one aspect of the disclosure, alkynyl groups have from 2 to 6 carbon atoms and at least one triple bond. Exemplary alkynyl groups include prop-1-ynyl, prop-2-ynyl (*i.e.,* propargyl), ethynyl and 3-butynyl.

The terms "alkoxy," "alkylamino" and "alkylthio" (or thioalkoxy) are used in their conventional sense and refer to alkyl groups that are attached to the remainder of the molecule via an oxygen atom, an amino group, or a sulfur atom, respectively.

The term "heteroalkyl," by itself or in combination with another term, refers to a stable, straight or branched chain hydrocarbon radical consisting of the stated number of carbon atoms (*e.g.,* C₂-C₁₀, or C₂-C₈) and at least one heteroatom chosen , *e.g.,* from N, O, S, Si, B and P (in certain aspects, N, O and S), wherein the nitrogen, sulfur and phosphorus atoms are optionally oxidized, and the nitrogen atom(s) are optionally quaternized. The heteroatom(s) is/are placed at any interior position of the heteroalkyl group. Examples of heteroalkyl groups include, but are not limited to, -CH₂-CH₂-O-CH₃, -CH₂-CH₂-NH-CH₃, -CH₂-CH₂-N(CH₃)-CH₃, -CH₂-S-CH₂-CH₃, - CH₂-CH₂-S(O)-CH₃, -CH₂-CH₂-S(O)₂-CH₃, -CH=CH-O-CH₃, -CH₂-Si(CH₃)₃, -CH₂-CH=N-OCH₃, and -CH=CH-N(CH₃)-CH₃. Up to two heteroatoms can be consecutive, such as, for example, -CH₂-NH-OCH₃ and -CH₂-O-Si(CH₃)₃.

The term "heteroalkylene," by itself or as part of another substituent, refers to a divalent radical derived from heteroalkyl, as exemplified, but not limited by, -CH₂-CH₂-S-CH₂-CH₂- and -CH₂-S-CH₂-CH₂-NH-CH₂-. Typically, a heteroalkyl group will have from 3 to 24 atoms (carbon and heteroatoms, excluding hydrogen) (3- to 24-membered heteroalkyl). In another example, the heteroalkyl group has a total of 3 to 10 atoms (3- to 10-membered heteroalkyl) or from 3 to 8 atoms (3- to 8-membered heteroalkyl). The term "heteroalkyl" includes "heteroalkylene" wherever appropriate, *e.g*., when the formula indicates that the heteroalkyl group is divalent or when substituents are joined to form a ring.

The term "cycloalkyl," by itself or in combination with other terms, represents a saturated or unsaturated, non-aromatic carbocyclic radical having from 3 to 24 carbon atoms, for example, having from 3 to 12 carbon atoms (*e.g*., C₃-C₈ cycloalkyl or C₃-C₆ cycloalkyl). Examples of cycloalkyl include, but are not limited to, cyclopropyl, cyclobutyl, cyclopentyl, cyclohexyl, cycloheptyl, 1-cyclohexenyl, 3-cyclohexenyl, cycloheptyl and the like. The term "cycloalkyl" also includes bridged, polycyclic (*e.g.,* bicyclic) structures, such as norbornyl, adamantyl and bicyclo[2.2.1]heptyl. The "cycloalkyl" group can be fused to at least one (*e.g.,* 1 to 3) other ring selected from aryl (*e.g.,* phenyl), heteroaryl (*e.g.,* pyridyl) and non-aromatic (*e.g.,* carbocyclic or heterocyclic) rings. When the "cycloalkyl" group includes a fused aryl, heteroaryl or heterocyclic ring, then the "cycloalkyl" group is attached to the remainder of the molecule via the carbocyclic ring.

The term "heterocycloalkyl," "heterocyclic," "heterocycle," or "heterocyclyl," by itself or in combination with other terms, represents a carbocyclic, non-aromatic ring (*e.g.,* 3- to 8-membered ring and for example, 4-, 5-, 6- or 7-membered ring) containing at least one and up to 5 heteroatoms selected from, *e.g.,* N, O, S, Si, B and P (for example, N, O and S), wherein the nitrogen, sulfur and phosphorus atoms are optionally oxidized, and the nitrogen atom(s) are optionally quaternized (*e.g*., from 1 to 4 heteroatoms selected from nitrogen, oxygen and sulfur), or a fused ring system of 4- to 8-membered rings, containing at least one and up to 10 heteroatoms (*e.g.,* from 1 to 5 heteroatoms selected from N, O and S) in stable combinations known to those of skill in the art. Exemplary heterocycloalkyl groups include a fused phenyl ring. When the "heterocyclic" group includes a fused aryl, heteroaryl or cycloalkyl ring, then the "heterocyclic" group is attached to the remainder of the molecule via a heterocycle. A heteroatom can occupy the position at which the heterocycle is attached to the remainder of the molecule.

As used herein, the term "associated with" refers to the interaction between a first moiety (*e.g.,* payload) and a second moiety (*e.g.,* EV). For instance, in some aspects, the first moiety can be encapsulated within the second moiety (*e.g.,* a payload can be in the lumen of the EV). In some aspects, the first moiety can be linked or fused to the second moiety (*e.g.,* a payload can be linked to the exterior surface and/or the luminal surface of an EV).

The term "biologically active molecule," as used herein, refers to any molecule that can be attached to an EV via an anchoring moiety, wherein the molecule can have a therapeutic or prophylactic effect in a subject in need thereof, or be used for diagnostic purposes. In some aspects, such molecules of interest are also referred to herein as "moieties of interest." Accordingly, by way of example, the term biologically active molecule include proteins (*e.g.*, antibodies, proteins, polypeptides, and derivatives, fragments, and variants thereof), lipids and derivatives thereof, carbohydrates (*e.g*., glycan portions in glycoproteins), or small molecules. In some aspects, the biologically active molecule comprises an antigen, adjuvant, immune modulator, targeting moiety, or combinations thereof. Non-limiting examples of such biologically active molecules are provided in, *e.g.,* WO 2020/0191361 A2. In certain aspects, the biologically active molecule is a payload (*e.g.*, antisense oligonucleotide).

The term "C₁₋₈ alkyl" as used herein refers to a straight chain or branched, saturated hydrocarbon having from 1 to 8 carbon atoms. Representative "C₁₋₈ alkyl" groups include, but are not limited to, methyl, ethyl, n-propyl, n-butyl, n-pentyl, n-hexyl, n-heptyl, n-octyl, isopropyl, sec-butyl, isobutyl, tert-butyl, isopentyl, and 2-methylbutyl.

The term "C₁₋₁₀ alkylene" refers to a saturated, straight chain hydrocarbon group of the formula -(CH₂)₁₋₁₀-. Examples of C₁₋₁₀ alkylene include methylene, ethylene, propylene, butylene, pentylene, hexylene, heptylene, octylene, nonylene, and decalene.

The term "C₃₋₈ carbocycle" refers to a 3-, 4-, 5-, 6-, 7- or 8-membered saturated or unsaturated non-aromatic carbocyclic ring. Representative C₃₋₈ carbocycles include, but are not limited to, cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and -cyclooctadienyl. A C₃₋₈ carbocycle group can be unsubstituted or substituted with one or more groups including, but not limited to, -C₁₋₈ alkyl, -O-(C₁₋₈ alkyl), aryl, -C(O)R', - OC(O)R', -C(O)OR', -C(O)NH₂, -C(O)NHR', -C(O)N(R')₂-, NHC(O)R', -S(O)₂R', -S(O)R', -OH, - halogen, -N₃, -NH₂, -NH(R'), -N(R')₂ and -CN, where each R' is independently H, -C₁₋₈ alkyl, or aryl.

The term "C₃₋₈ carbocyclo" refers to a C₃₋₈ carbocycle group defined above wherein one or more of the carbocycle's hydrogen atoms is replaced with a bond.

The term "C₃₋₈ heterocycle" refers to an aromatic or non-aromatic C₃₋₈ carbocycle in which one to four of the ring carbon atoms are independently replaced with a heteroatom selected from the group consisting of O, S and N. Representative examples of a C₃₋₈ heterocycle include, but are not limited to, benzofuranyl, benzothiophene, indolyl, benzopyrazolyl, coumarinyl, isoquinolinyl, pyrrolyl, thiophenyl, furanyl, thiazolyl, imidazolyl, pyrazolyl, triazolyl, quinolinyl, pyrimidinyl, pyridinyl, pyridonyl, pyrazinyl, pyridazinyl, isothiazolyl, isoxazolyl and tetrazolyl. A C₃₋₈ heterocycle can be unsubstituted or substituted with up to seven groups including, but not limited to, -C₁₋₈ alkyl, -O-(C₁₋₈ alkyl), -aryl, -C(O)R', -OC(O)R', -C(O)OR', -C(O)NH₂, - C(O)NHR', -C(O)N(R')₂, -NHC(O)R', -S(O)₂R', -S(O)R', -OH, -halogen, -N₃, -NH₂, -NH(R'), - N(R')₂, and -CN, wherein each R' is independently H, -C₁₋₈ alkyl, or aryl.

The term "C₃₋₈ heterocyclo" refers to a C₃₋₈ heterocycle group defined above wherein one of the heterocycle group's hydrogen atoms is replaced with a bond. A C₃₋₈ heterocyclo can be unsubstituted or substituted with up to six groups including, but not limited to, -C₁₋₈ alkyl, -O-(C₁₋₈ alkyl), -aryl, -C(O)R', -OC(O)R', -C(O)OR', -C(O)NH₂, -C(O)NHR', -C(O)N(R')₂, - NHC(O)R', -S(O)₂R', -S(O)R', -OH, -halogen, -N₃, -NH₂, -NH(R'), -N(R')₂ and -CN, wherein each R' is independently H, -C₁₋₈ alkyl, or aryl.

A "conservative amino acid substitution" is one in which the amino acid residue is replaced with an amino acid residue having a similar side chain. Families of amino acid residues having similar side chains have been defined in the art, including basic side chains (*e.g*., lysine, arginine, histidine), acidic side chains (*e.g*., aspartic acid, glutamic acid), uncharged polar side chains (*e.g.*, glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Thus, if an amino acid in a polypeptide is replaced with another amino acid from the same side chain family, the substitution is considered to be conservative. In certain aspects, a string of amino acids can be conservatively replaced with a structurally similar string that differs in order and/or composition of side chain family members.

As used herein, the term "conserved" refers to nucleotides or amino acid residues of a polynucleotide sequence or polypeptide sequence, respectively, that are those that occur unaltered in the same position of two or more sequences being compared. Nucleotides or amino acids that are relatively conserved are those that are conserved amongst more related sequences than nucleotides or amino acids appearing elsewhere in the sequences.

In some aspects, two or more sequences are said to be "completely conserved" or "identical" if they are 100% identical to one another. In some aspects, two or more sequences are said to be "highly conserved" if they are at least 70% identical, at least 80% identical, at least 90% identical, or at least 95% identical to one another. In some aspects, two or more sequences are said to be "highly conserved" if they are about 70% identical, about 80% identical, about 90% identical, about 95%, about 98%, or about 99% identical to one another. In some aspects, two or more sequences are said to be "conserved" if they are at least 30% identical, at least 40% identical, at least 50% identical, at least 60% identical, at least 70% identical, at least 80% identical, at least 90% identical, or at least 95% identical to one another. In some aspects, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence can apply to the entire length of a polynucleotide or polypeptide or can apply to a portion, region or feature thereof.

The terms "excipient" and "carrier" are used interchangeably and refer to an inert substance added to a pharmaceutical composition to further facilitate administration of a compound.

As used herein, the terms "extracellular vesicle," "EV," and grammatical variants thereof, are used interchangeably and refer to a cell-derived vesicle comprising a membrane that encloses an internal space. Extracellular vesicles comprise all membrane-bound vesicles (*e.g.,* exosomes, nanovesicles) that have a smaller diameter than the cell from which they are derived. In some aspects, extracellular vesicles range in diameter from 20 nm to 1000 nm, and can comprise various macromolecular payload either within the internal space (*i.e.,* lumen), displayed on the external surface of the extracellular vesicle, and/or spanning the membrane. In some aspects, the payload can comprise nucleic acids, proteins, carbohydrates, lipids, small molecules, and/or combinations thereof. In certain aspects, an extracellular vehicle comprises an exosomal protein. By way of example and without limitation, extracellular vesicles include apoptotic bodies, fragments of cells, vesicles derived from cells by direct or indirect manipulation (*e.g*., by serial extrusion or treatment with alkaline solutions), vesiculated organelles, and vesicles produced by living cells (*e.g.,* by direct plasma membrane budding or fusion of the late endosome with the plasma membrane). Extracellular vesicles can be derived from a living or dead organism, explanted tissues or organs, prokaryotic or eukaryotic cells, and/or cultured cells. In some aspects, the extracellular vesicles are produced by cells that express one or more transgene products. Unless indicated otherwise, EV comprises an exosome, nanovesicles, microsomes, microvesicles, extracellular bodies, apoptotic bodies, or combinations thereof. In certain aspects, an EV useful for the present disclosure is an exosome.

As used herein, the term "exosome" refers to an extracellular vesicle with a diameter between 20-300 nm (*e.g*., between 40-200 nm). Exosomes comprise a membrane that encloses an internal space (*i.e.,* lumen), and, in some aspects, can be generated from a cell (*e.g.,* producer cell) by direct plasma membrane budding or by fusion of the late endosome with the plasma membrane. In certain aspects, an exosome comprises an exosomal protein. As described *infra,* exosome can be derived from a producer cell, and isolated from the producer cell based on its size, density, biochemical parameters, or a combination thereof. In some aspects, the exosomes of the present disclosure are produced by cells that express one or more transgene products.

As used herein, the term "functional fragment" refers to a protein fragment that retains protein function. For instance, in some aspects, a functional fragment of an exosomal protein, *e.g.,* PTGFRN, retains the ability to anchor a biologically active molecule on the luminal surface or on the external surface of the EV.

Whether a fragment is a functional fragment can be assessed by any art known methods to determine the protein content of EVs including Western Blots, FACS analysis and fusions of the fragments with autofluorescent proteins like, *e.g.,* GFP.

As used herein, "anchoring" a biologically active molecule on the luminal or external surface of an EV of the present disclosure refers to attaching covalently the biologically active molecule to the luminal or external surface of the EV, respectively.

As used herein, the term "homology" refers to the overall relatedness between polymeric molecules, *e.g.* between nucleic acid molecules (*e.g.* DNA molecules and/or RNA molecules) and/or between polypeptide molecules. Generally, the term "homology" implies an evolutionary relationship between two molecules. Thus, two molecules that are homologous will have a common evolutionary ancestor. In the context of the present disclosure, the term homology encompasses both to identity and similarity.

In some aspects, polymeric molecules are considered to be "homologous" to one another if at least 25%, 30%, 35%, 40%, 45%, 50%, 55%, 60%, 65%, 70%, 75%, 80%, 85%, 90%, 95%, or 99% of the monomers in the molecule are identical (exactly the same monomer) or are similar (conservative substitutions). The term "homologous" necessarily refers to a comparison between at least two sequences (polynucleotide or polypeptide sequences).

In the context of the present disclosure, substitutions (even when they are referred to as amino acid substitution) are conducted at the nucleic acid level, *i.e.,* substituting an amino acid residue with an alternative amino acid residue is conducted by substituting the codon encoding the first amino acid with a codon encoding the second amino acid.

As used herein, the term "identity" refers to the overall monomer conservation between polymeric molecules, *e.g*., between polypeptide molecules or polynucleotide molecules (*e.g.* DNA molecules and/or RNA molecules). The term "identical" without any additional qualifiers, *e.g*., protein A is identical to protein B, implies the sequences are 100% identical (100% sequence identity). Describing two sequences as, *e.g.,* "70% identical," is equivalent to describing them as having, *e.g.,* "70% sequence identity."

Calculation of the percent identity of two polypeptide sequences, for example, can be performed by aligning the two sequences for optimal comparison purposes (*e.g.*, gaps can be introduced in one or both of a first and a second polypeptide sequences for optimal alignment and non-identical sequences can be disregarded for comparison purposes). In certain aspects, the length of a sequence aligned for comparison purposes is at least 30%, at least 40%, at least 50%, at least 60%, at least 70%, at least 80%, at least 90%, at least 95%, or 100% of the length of the reference sequence. The amino acids at corresponding amino acid positions are then compared.

When a position in the first sequence is occupied by the same amino acid as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm.

Suitable software programs are available from various sources, and for alignment of both protein and nucleotide sequences. One suitable program to determine percent sequence identity is bl2seq, part of the BLAST suite of program available from the U.S. government's National Center for Biotechnology Information BLAST web site (blast.ncbi.nlm.nih.gov). Bl2seq performs a comparison between two sequences using either the BLASTN or BLASTP algorithm. BLASTN is used to compare nucleic acid sequences, while BLASTP is used to compare amino acid sequences. Other suitable programs are, *e.g.,* Needle, Stretcher, Water, or Matcher, part of the EMBOSS suite of bioinformatics programs and also available from the European Bioinformatics Institute (EBI) at worldwideweb.ebi.ac.uk/Tools/psa.

Sequence alignments can be conducted using methods known in the art such as MAFFT, Clustal (ClustalW, Clustal X or Clustal Omega), MUSCLE, etc.

Different regions within a single polynucleotide or polypeptide target sequence that aligns with a polynucleotide or polypeptide reference sequence can each have their own percent sequence identity. It is noted that the percent sequence identity value is rounded to the nearest tenth. For example, 80.11, 80.12, 80.13, and 80.14 are rounded down to 80.1, while 80.15, 80.16, 80.17, 80.18, and 80.19 are rounded up to 80.2. It also is noted that the length value will always be an integer.

In certain aspects, the percentage identity (%ID) or of a first amino acid sequence (or nucleic acid sequence) to a second amino acid sequence (or nucleic acid sequence) is calculated as %ID = 100 x (Y/Z), where Y is the number of amino acid residues (or nucleobases) scored as identical matches in the alignment of the first and second sequences (as aligned by visual inspection or a particular sequence alignment program) and Z is the total number of residues in the second sequence. If the length of a first sequence is longer than the second sequence, the percent identity of the first sequence to the second sequence will be higher than the percent identity of the second sequence to the first sequence.

One skilled in the art will appreciate that the generation of a sequence alignment for the calculation of a percent sequence identity is not limited to binary sequence-sequence comparisons exclusively driven by primary sequence data. It will also be appreciated that sequence alignments can be generated by integrating sequence data with data from heterogeneous sources such as structural data *(e.g.,* crystallographic protein structures), functional data *(e.g.,* location of mutations), or phylogenetic data. A suitable program that integrates heterogeneous data to generate a multiple sequence alignment is T-Coffee, available at www.tcoffee.org, and alternatively available, e.g., from the EBI. It will also be appreciated that the final alignment used to calculate percent sequence identity can be curated either automatically or manually.

An "immune response", as used herein, refers to a biological response within a vertebrate against foreign agents or abnormal, *e.g*., cancerous cells, which response protects the organism against these agents and diseases caused by them. An immune response is mediated by the action of one or more cells of the immune system (for example, a T lymphocyte, B lymphocyte, natural killer (NK) cell, macrophage, eosinophil, mast cell, dendritic cell or neutrophil) and soluble macromolecules produced by any of these cells or the liver (including antibodies, cytokines, and complement) that results in selective targeting, binding to, damage to, destruction of, and/or elimination from the vertebrate's body of invading pathogens, cells or tissues infected with pathogens, cancerous or other abnormal cells, or, in cases of autoimmunity or pathological inflammation, normal human cells or tissues. An immune reaction includes, *e.g*., activation or inhibition of a T cell, *e.g.,* an effector T cell, a Th cell, a CD4+ cell, a CD8+ T cell, or a Treg cell, or activation or inhibition of any other cell of the immune system, *e.g*., NK cell. Accordingly an immune response can comprise a humoral immune response (*e.g*., mediated by B-cells), cellular immune response (*e.g*., mediated by T cells), or both humoral and cellular immune responses. In some aspects of the present disclosure, the biologically active molecule is a molecule capable of eliciting an immune response.

In some aspects, an immune response is an "inhibitory" immune response. An inhibitory immune response is an immune response that blocks or diminishes the effects of a stimulus (*e.g.,* antigen). In certain aspects, the inhibitory immune response comprises the production of inhibitory antibodies against the stimulus. In some aspects, an immune response is a "stimulatory" immune response. A stimulatory immune response is an immune response that results in the generation of effectors cells (*e.g.,* cytotoxic T lymphocytes) that can destroy and clear a target antigen (*e.g.,* tumor antigen or viruses).

As used herein, the terms "isolated," "purified," "extracted," and grammatical variants thereof are used interchangeably and refer to the state of a preparation of desired EVs (*e.g.,* a plurality of EVs of known or unknown amount and/or concentration), that has undergone one or more processes of purification, *e.g*., a selection or an enrichment of the desired EV preparation. In some aspects, isolating or purifying as used herein is the process of removing, partially removing (*e.g.,* a fraction) of the EVs from a sample containing producer cells. In some aspects, an isolated EV composition has no detectable undesired activity or, alternatively, the level or amount of the undesired activity is at or below an acceptable level or amount. In some aspects, an isolated EV composition has an amount and/or concentration of desired EVs at or above an acceptable amount and/or concentration. In some aspects, the isolated EVs composition is enriched as compared to the starting material (*e.g.,* producer cell preparations) from which the composition is obtained. This enrichment can be by at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, at least about 99%, at least about 99.9%, at least about 99.99%, at least about 99.999%, at least about 99.9999%, or greater than 99.9999% as compared to the starting material. In some aspects, isolated EV preparations are substantially free of residual biological products. In some aspects, the isolated EV preparations are 100% free, at least about 99% free, at least about 98% free, at least about 97% free, at least about 96% free, at least about 95% free, at least about 94% free, at least about 93% free, at least about 92% free, at least about 91% free, or at least about 90% free of any contaminating biological matter. Residual biological products can include abiotic materials (including chemicals) or unwanted nucleic acids, proteins, lipids, or metabolites. Substantially free of residual biological products can also mean that the EV composition contains no detectable producer cells and that only EVs are detectable.

The terms "linked," "fused," and grammatical variants thereof are used interchangeably and refer to a first moiety, *e.g.,* a first amino acid sequence or nucleotide sequence, covalently or non-covalently joined to a second moiety, *e.g.,* a second amino acid sequence or nucleotide sequence, respectively. The first moiety can be directly joined or juxtaposed to the second moiety or alternatively an intervening moiety can covalently join the first moiety to the second moiety. The term "linked" means not only a fusion of a first moiety to a second moiety at the C-terminus or the N-terminus, but also includes insertion of the whole first moiety (or the second moiety) into any two points, *e.g*., amino acids, in the second moiety (or the first moiety, respectively). In one aspect, the first moiety is linked to a second moiety by a peptide bond or a linker. The first moiety can be linked to a second moiety by a phosphodiester bond or a linker. The linker can be a peptide or a polypeptide (for polypeptide chains) or a nucleotide or a nucleotide chain (for nucleotide chains) or any chemical moiety (for polypeptide or polynucleotide chains or any chemical molecules). The term "linked" is also indicated by a hyphen (-).

As used herein, the term "loading" or "loaded" refers to the introduction of a moiety of interest (*e.g.,* payload, *e.g.,* ASO) into, onto, or otherwise associate with an EV, such that the moiety of interest is associated with the EV. As described herein, in some aspects, an EV loaded with a payload refers to an EV where the payload is linked or conjugated to the exterior surface of the EV.

The term "loading efficiency" refers to a ratio of payload (e.g., antisense oligonucleotide) loaded onto an EV compared to total payload (e.g., antisense oligonucleotide) in solution (e.g., payload loaded/payload total). As is apparent from the present disclosure, in some aspects, an increase in loading efficiency means that more of the payload (*e.g.,* ASOs) in the solution (comprising the mixture of EVs and payloads) has been loaded onto the EVs compared to a reference value (*e.g.,* loading efficiency observed with a loading method that differs from the present disclosure). Unless indicated, the term "loading efficiency" and "loading density" can be used interchangeably herein.

As used herein the term "lumen-engineered EV" refers to an EV with the luminal surface of the membrane or the lumen of the EV modified in its composition so that the luminal surface or the lumen of the engineered EV is different from that of the EV prior to the modification or of the naturally occurring EV.

The engineering can be directly in the lumen (i.e., the void within the EV) or in the membrane of the EV, in particular the luminal surface of the EV, so that the lumen and/or the luminal surface of the EV is changed. For example, the membrane is modified in its composition of a protein, a lipid, a small molecule, a carbohydrate, *etc.* so that the luminal surface of the EV is modified. Similarly, the contents in the lumen can be modified. The composition can be changed by a chemical, a physical, or a biological method or by being produced from a cell previously modified by a chemical, a physical, or a biological method. Specifically, the composition can be changed by a genetic engineering or by being produced from a cell previously modified by genetic engineering. In some aspects, a lumen-engineered EV comprises an exogenous protein (*i.e.,* a protein that the EV does not naturally express) or a fragment or variant thereof that can be exposed on the luminal surface or lumen of the EV or can be an anchoring point (attachment) for a moiety exposed on the inner layer of the EV.

As used herein, the term "macromolecule" refers to nucleic acids, proteins, lipids, carbohydrates, metabolites, or combinations thereof.

The term "modified," when used in the context of EVs described herein, refers to an alteration or engineering of an EV and/or its producer cell, such that the modified EV is different from a naturally-occurring EV. In some aspects, a modified EV described herein comprises a membrane that differs in composition of a protein, a lipid, a small molecular, a carbohydrate, *etc.* compared to the membrane of a naturally-occurring EV. For instance, as demonstrated herein, the EVs described herein have been modified to comprise an increased amount of a payload (*e.g.,* antisense oligonucleotide) that is not naturally found in EVs. In certain aspects, such modifications to the membrane change the exterior surface of the EV (*e.g.,* surface-engineered EVs). In certain aspects, such modifications to the membrane change the luminal surface of the EV (*e.g.,* lumen-engineered EV described herein).

As used herein, the terms "modified protein" or "protein modification" refers to a protein having at least 15% identity to the non-mutant amino acid sequence of the protein. A modification of a protein includes a fragment or a variant of the protein. A modification of a protein can further include chemical, or physical modification to a fragment or a variant of the protein.

As used herein, the terms "modulate," "modify," and grammatical variants thereof, generally refer when applied to a specific concentration, level, expression, function or behavior, to the ability to alter, by increasing or decreasing, *e.g.,* directly or indirectly promoting/stimulating/up-regulating or interfering with/inhibiting/down-regulating the specific concentration, level, expression, function or behavior, such as, *e.g.,* to act as an antagonist or agonist. In some instances, a modulator can increase and/or decrease a certain concentration, level, activity or function relative to a control, or relative to the average level of activity that would generally be expected or relative to a control level of activity.

As used herein, the term "nanovesicle" refers to an extracellular vesicle with a diameter between 20-250 nm (*e.g.,* between 30-150 nm) and is generated from a cell (*e.g.,* producer cell) by direct or indirect manipulation such that the nanovesicle would not be produced by the cell without the manipulation. Appropriate manipulations of the cell to produce the nanovesicles include but are not limited to serial extrusion, treatment with alkaline solutions, sonication, or combinations thereof. In some aspects, production of nanovesicles can result in the destruction of the producer cell. In some aspects, population of nanovesicles described herein are substantially free of vesicles that are derived from cells by way of direct budding from the plasma membrane or fusion of the late endosome with the plasma membrane. Nanovesicles, once derived from a producer cell, can be isolated from the producer cell based on its size, density, biochemical parameters, or a combination thereof.

As used herein, the term "payload" refers to a biologically active molecule (*e.g.,* a therapeutic agent) that acts on a target (*e.g.,* a target cell) that is contacted with the EV described herein. Non-limiting examples of payloads that can be introduced into an EV include therapeutic agents such as, nucleotides (*e.g.,* nucleotides comprising a detectable moiety or a toxin or that disrupt transcription), nucleic acids (*e.g.,* DNA or mRNA molecules that encode a polypeptide such as an enzyme, or RNA molecules that have regulatory function such as miRNA, dsDNA, lncRNA, siRNA, phosphorodiamidate morpholino oligomer (PMO), peptide-conjugated phosphorodiamidate morpholino oligomer (PPMO), and antisense oligonucleotides), amino acids (*e.g.*, amino acids comprising a detectable moiety or a toxin or that disrupt translation), polypeptides (*e.g.,* enzymes), lipids, carbohydrates, and small molecules (*e.g.,* small molecule drugs and toxins). In some aspects, the payload comprises an antisense oligonucleotide.

The terms "pharmaceutically-acceptable carrier," "pharmaceutically-acceptable excipient," and grammatical variations thereof, encompass any of the agents approved by a regulatory agency of the U.S. Federal government or listed in the U.S. Pharmacopeia for use in animals, including humans, as well as any carrier or diluent that does not cause the production of undesirable physiological effects to a degree that prohibits administration of the composition to a subject and does not abrogate the biological activity and properties of the administered compound. Included are excipients and carriers that are useful in preparing a pharmaceutical composition and are generally safe, non-toxic, and desirable.

As used herein, the term "pharmaceutical composition" refers to one or more of the compounds described herein (*e.g.,* EVs), mixed or intermingled with, or suspended in one or more other chemical components, such as pharmaceutically-acceptable carriers and excipients. One purpose of a pharmaceutical composition is to facilitate administration of preparations of EVs to a subject.

The term "polynucleotide" as used herein refers to polymers of nucleotides of any length, including ribonucleotides, deoxyribonucleotides, analogs thereof, or mixtures thereof. This term refers to the primary structure of the molecule. Thus, the term includes triple-, double- and single-stranded deoxyribonucleic acid ("DNA"), as well as triple-, double- and single-stranded ribonucleic acid ("RNA"). It also includes modified, for example by alkylation, and/or by capping, and unmodified forms of the polynucleotide. In certain aspects, the term "polynucleotide" includes polydeoxyribonucleotides (containing 2-deoxy-D-ribose), polyribonucleotides (containing D-ribose), including tRNA, rRNA, hRNA, siRNA and mRNA, whether spliced or unspliced, any other type of polynucleotide which is an N- or C-glycoside of a purine or pyrimidine base, and other polymers containing normucleotidic backbones, for example, polyamide (*e.g.,* peptide nucleic acids "PNAs") and polymorpholino polymers, and other synthetic sequence-specific nucleic acid polymers providing that the polymers contain nucleobases in a configuration which allows for base pairing and base stacking, such as is found in DNA and RNA. In particular aspects, the polynucleotide comprises an mRNA. In certain aspects, the mRNA is a synthetic mRNA. In some aspects, the synthetic mRNA comprises at least one unnatural nucleobase. In some aspects, all nucleobases of a certain class have been replaced with unnatural nucleobases (*e.g.,* all uridines in a polynucleotide disclosed herein can be replaced with an unnatural nucleobase, *e.g.*, 5-methoxyuridine). In some aspects of the present disclosure, the biologically active molecule is a polynucleotide (*e.g.,* antisense oligonucleotide).

The terms "polypeptide," "peptide," and "protein" are used interchangeably herein to refer to polymers of amino acids of any length. The polymer can comprise modified amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids such as homocysteine, ornithine, p-acetylphenylalanine, D-amino acids, and creatine), as well as other modifications known in the art.

The term "polypeptide," as used herein, refers to proteins, polypeptides, and peptides of any size, structure, or function. Polypeptides include gene products, naturally occurring polypeptides, synthetic polypeptides, homologs, orthologs, paralogs, fragments and other equivalents, variants, and analogs of the foregoing. A polypeptide can be a single polypeptide or can be a multi-molecular complex such as a dimer, trimer or tetramer. They can also comprise single chain or multichain polypeptides. Most commonly disulfide linkages are found in multichain polypeptides. The term polypeptide can also apply to amino acid polymers in which one or more amino acid residues are an artificial chemical analogue of a corresponding naturally occurring amino acid. In some aspects, a "peptide" can be less than or equal to 50 amino acids long, *e.g.,* about 5, 10, 15, 20, 25, 30, 35, 40, 45, or 50 amino acids long.

The terms "prevent," "preventing," and variants thereof as used herein, refer partially or completely delaying onset of an disease, disorder and/or condition; partially or completely delaying onset of one or more symptoms, features, or clinical manifestations of a particular disease, disorder, and/or condition; partially or completely delaying onset of one or more symptoms, features, or manifestations of a particular disease, disorder, and/or condition; partially or completely delaying progression from a particular disease, disorder and/or condition; and/or decreasing the risk of developing pathology associated with the disease, disorder, and/or condition. In some aspects, preventing an outcome is achieved through prophylactic treatment.

As used herein, the term "producer cell" refers to a cell used for generating an EV. A producer cell can be a cell cultured *in vitro,* or a cell *in vivo.* A producer cell includes, but not limited to, a cell known to be effective in generating EVs, *e.g.,* HEK293 cells, Chinese hamster ovary (CHO) cells, mesenchymal stem cells (MSCs), BJ human foreskin fibroblast cells, fHDF fibroblast cells, AGE.HN^{®} neuronal precursor cells, CAP^{®} amniocyte cells, adipose mesenchymal stem cells, RPTEC/TERT1 cells. In certain aspects, a producer cell is not an antigen-presenting cell. In some aspects, a producer cell is not a dendritic cell, a B cell, a mast cell, a macrophage, a neutrophil, Kupffer-Browicz cell, cell derived from any of these cells, or any combination thereof.

As used herein, "prophylactic" refers to a therapeutic or course of action used to prevent the onset of a disease or condition, or to prevent or delay a symptom associated with a disease or condition.

As used herein, a "prophylaxis" refers to a measure taken to maintain health and prevent or delay the onset of a bleeding episode, or to prevent or delay symptoms associated with a disease or condition.

A "recombinant" polypeptide or protein refers to a polypeptide or protein produced via recombinant DNA technology. Recombinantly produced polypeptides and proteins expressed in engineered host cells are considered isolated for the purpose of the disclosure, as are native or recombinant polypeptides which have been separated, fractionated, or partially or substantially purified by any suitable technique. The polypeptides disclosed herein can be recombinantly produced using methods known in the art. Alternatively, the proteins and peptides disclosed herein can be chemically synthesized. In some aspects of the present disclosure, the exosomal proteins present in EVs, are recombinantly produced by overexpressing the exosomal proteins in the producer cells, so that levels of exosomal proteins in the resulting EVs are significantly increased with respect to the levels of exosomal proteins present in EVs of producer cells not overexpressing such exosomal proteins.

As used herein, the term "exosomal proteins" refers to EV proteins that have been identified on the surface of EVs. *See, e.g.,* U.S. Pat. No. 10,195,290. Non-limiting examples of exosomal proteins include: prostaglandin F2 receptor negative regulator ("PTGFRN"); basigin ("BSG"); immunoglobulin superfamily member 2 ("IGSF2"); immunoglobulin superfamily member 3 ("IGSF3 "); immunoglobulin superfamily member 8 ("IGSF8"); integrin beta-1 ("ITGB1"); integrin alpha-4 ("ITGA4 "); 4F2 cell-surface antigen heavy chain ("SLC3A2"); and a class of ATP transporter proteins ("ATP1A1," "ATP1A2," "ATP1A3," "ATP1A4," "ATP1B3," "ATP2B1," "ATP2B2," "ATP2B3," "ATP2B"). In some aspects, an exosomal protein can be a whole protein or a fragment thereof (*e.g.,* functional fragment, *e.g.,* the smallest fragment that is capable of anchoring another moiety on the external surface or on the luminal surface of the EV. In some aspects, an exosomal protein can anchor a biologically active molecule to the external surface or the lumen of the EV. Non-limiting examples of other exosomal proteins that can be used with the present disclosure include: aminopeptidase N (CD13); Neprilysin, AKA membrane metalloendopeptidase (MME); ectonucleotide pyrophosphatase/phosphodiesterase family member 1 (ENPP1); Neuropilin-1 (NRP1); CD9, CD63, CD81, PDGFR, GPI anchor proteins, lactadherin, LAMP2, and LAMP2B.

Additional exosomal proteins that have been identified within the lumen of E. *See, e.g.,* U.S. Publ. No. 2020/0347112 A1. Non-limiting examples of exosomal proteins include: myristoylated alanine rich Protein Kinase C substrate ("MARCKS"); myristoylated alanine rich Protein Kinase C substrate like 1 ("MARCKSL 1 "); and brain acid soluble protein 1 ("BASP1"). In some aspects, an exosomal protein can be a whole protein or a fragment thereof (*e.g*., functional fragment, *e.g.,* the smallest fragment that is capable of anchoring a moiety on the luminal surface of the EV. In some aspects, an exosomal can anchor a moiety to the luminal surface of the EV. In some aspects of the present disclosure, a moiety can be covalently attached to an exosome.

As used herein, the term "similarity" refers to the overall relatedness between polymeric molecules, *e.g.* between polynucleotide molecules (*e.g.* DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art. It is understood that percentage of similarity is contingent on the comparison scale used, i.e., whether the amino acids are compared, *e.g.,* according to their evolutionary proximity, charge, volume, flexibility, polarity, hydrophobicity, aromaticity, isoelectric point, antigenicity, or combinations thereof.

The term "spacer" as used herein refers to a bifunctional chemical moiety which is capable of covalently linking together two spaced moieties (*e.g.,* a cleavable linker and a biologically active molecule) into a normally stable dipartate molecule.

Unless otherwise indicated, reference to a compound that has one or more stereocenters intends each stereoisomer, and all combinations of stereoisomers, thereof.

The terms "subject," "patient," "individual," and "host," and variants thereof are used interchangeably herein and refer to any mammalian subject, including without limitation, humans, domestic animals (*e.g.,* dogs, cats and the like), farm animals (*e.g.,* cows, sheep, pigs, horses and the like), and laboratory animals (*e.g.,* monkey, rats, mice, rabbits, guinea pigs and the like) for whom diagnosis, treatment, or therapy is desired, particularly humans. The methods described herein are applicable to both human therapy and veterinary applications.

As used herein, the term "substantially free" means that the sample comprising EVs, comprises less than 10% of macromolecules, e.g., contaminants, by mass/volume (m/v) percentage concentration. Some fractions can contain less than 0.001%, less than 0.01%, less than 0.05%, less than 0.1%, less than 0.2%, less than 0.3%, less than 0.4%, less than 0.5%, less than 0.6%, less than 0.7%, less than 0.8%, less than 0.9%, less than 1%, less than 2%, less than 3%, less than 4%, less than 5%, less than 6%, less than 7%, less than 8%, less than 9%, or less than 10% (m/v) of macromolecules.

As used herein the term "surface-engineered EV" refers to an EV with the membrane or the surface of the EV modified in its composition so that the surface of the engineered EV is different from that of the EV prior to the modification or of the naturally occurring EV.

As used herein the term "surface-engineered exosome" refers to an exosome with the membrane or the surface of the exosome (external surface or luminal surface) modified in its composition so that the surface of the engineered exosome is different from that of the exosome prior to the modification or of the naturally occurring exosome.

The engineering can be on the surface and/or in the membrane of the EV so that the surface of the EV is changed. For example, the membrane can be modified in its composition of, *e.g.,* a protein, a lipid, a small molecule, a carbohydrate, or a combination thereof. The composition can be changed by a chemical, a physical, or a biological method or by being produced from a cell previously or concurrently modified by a chemical, a physical, or a biological method. Specifically, the composition can be changed by a genetic engineering or by being produced from a cell previously modified by genetic engineering. In some aspects, a surface-engineered EV comprises an exogenous protein (*i.e.,* a protein that the EV does not naturally express) or a fragment or variant thereof that can be exposed to the surface of the EV or can be an anchoring point (attachment) for a moiety exposed on the surface of the EV. In some aspects, a surface-engineered EV comprises a higher expression (*e.g.,* higher number) of a natural EV, *e.g.,* exosomal protein (*e.g.,* PTGFRN) or a fragment or variant thereof that can be exposed to the surface of the EV or can be an anchoring point (attachment) for a moiety exposed on the surface of the EV.

As used herein the term "therapeutically effective amount" is the amount of reagent or pharmaceutical compound comprising an EV described herein that is sufficient to a produce a desired therapeutic effect, pharmacologic and/or physiologic effect on a subject in need thereof. A therapeutically effective amount can be a "prophylactically effective amount" as prophylaxis can be considered therapy.

The terms "treat," "treatment," or "treating," as used herein refers to, *e.g.,* the reduction in severity of a disease or condition; the reduction in the duration of a disease course; the amelioration or elimination of one or more symptoms associated with a disease or condition; the provision of beneficial effects to a subject with a disease or condition, without necessarily curing the disease or condition. The term also include prophylaxis or prevention of a disease or condition or its symptoms thereof. In one aspect, the term "treating" or "treatment" means inducing an immune response in a subject against an antigen.

As used herein, the term "variant" of a molecule (*e.g*., functional molecule, antigen, or exosomal proteins) refers to a molecule that shares certain structural and functional identities with another molecule upon comparison by a method known in the art. For example, a variant of a protein can include a substitution, insertion, deletion, frame shift or rearrangement in another protein.

Naturally occurring variants are called "allelic variants," and refer to one of several alternate forms of a gene occupying a given locus on a chromosome of an organism (Genes II, Lewin, B., ed., John Wiley & Sons, New York (1985)). These allelic variants can vary at either the polynucleotide and/or polypeptide level and are included in the present disclosure. Alternatively, non-naturally occurring variants can be produced by mutagenesis techniques or by direct synthesis.

Using known methods of protein engineering and recombinant DNA technology, variants can be generated to improve or alter the characteristics of the polypeptides. For instance, one or more amino acids can be deleted from the N-terminus or C-terminus of the secreted protein without substantial loss of biological function. Ron et al., J. Biol. Chem. 268: 2984-2988 (1993), reported variant KGF proteins having heparin binding activity even after deleting 3, 8, or 27 amino-terminal amino acid residues. Similarly, interferon gamma exhibited up to ten times higher activity after deleting 8-10 amino acid residues from the carboxy terminus of this protein. (Dobeli et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See Abstract.) In fact, only 23 unique amino acid sequences, out of more than 3,500 nucleotide sequences examined, produced a protein that significantly differed in activity from wild-type.

As stated above, variants or derivatives include, *e.g.,* modified polypeptides. In some aspects, variants or derivatives of, *e.g.,* polypeptides, polynucleotides, lipids, glycoproteins, are the result of chemical modification and/or endogenous modification. In some aspects, variants or derivatives are the result of *in vivo* modification. In some aspects, variants or derivatives are the result of *in vitro* modification. In yet some aspects, variant or derivatives are the result of intracellular modification in producer cells.

### II. Method of Loading a Payload

Described herein are methods of loading an EV with a payload, such that the loading efficiency of the EV is increased.

In some aspects, a method of producing an EV comprises modifying the isolated EV by directly introducing one or more moieties of interest (*e.g.,* payload) into or onto the EVs. For instance, as described herein, a payload (*e.g.,* ASO) can be introduced to the EV by mixing the payload with the EV, *e.g.,* under the loading conditions described herein. In some aspects, the payload is conjugated to a cholesterol, such that the mixing of the payload with the EV allows the cholesterol to interact with the lipid membrane of the EV, and thereby, attach the payload to the exterior surface of the EV. As used herein, the term "mixing" refers to the process of bringing a payload and an EV in closer proximity, such that the EV can be loaded with the payload, *e.g.,* the payload associates with (*e.g.,* conjugated to) the exterior surface of the EV, the payload associates with (*e.g.,* conjugated to) the luminal surface of the EV, the payload is within the lumen of the EV, or any combination thereof. Unless indicated otherwise, the mixing can performed using any suitable methods known in the art. For example, in some aspects, the mixing can occur in a batch mode (*e.g.,* payloads and EVs are added at the beginning of the mixing process). In some aspects, the mixing can occur in a continuous process (*e.g.,* payloads and EVs are continuously added to the mixture during the course of the mixing process, *e.g.,* in a continuous stirred-tank reactor (CSTR)). In some aspects, the mixing can occur in a semi-batch mode. In some aspects, the mixing can occur in a semi-continuous mode.

In certain aspects, the one or more moieties (*e.g.,* payload) are introduced to the EV by transfection. In some aspects, the one or more moieties (*e.g.,* payload) can be introduced into the EV using synthetic macromolecules such as cationic lipids and polymers (Papapetrou et al., Gene Therapy 12: S118-S130 (2005)). In certain aspects, chemicals such as calcium phosphate, cyclodextrin, or polybrene, can be used to introduce the one or more moieties (*e.g.,* payload) to the EV.

In some aspects, one or more moieties (*e.g.,* payload) can be conjugated to the surface of the EV (*i.e.,* conjugated or linked directly to the exterior surface of the EV or to the luminal surface of the EV). Conjugation can be achieved chemically or enzymatically, by methods known in the art.

In some aspects, the EV comprises one or more moieties (*e.g.,* payload) that are chemically conjugated. Chemical conjugation can be accomplished by covalent bonding of the one or more moieties (*e.g.,* payload and/or targeting moiety) to another molecule, with or without use of a linker or affinity ligand disclosed herein. The formation of such conjugates is within the skill of artisans and various techniques are known for accomplishing the conjugation, with the choice of the particular technique being guided by the materials to be conjugated. In certain aspects, polypeptides are conjugated to the EV. In some aspects, non-polypeptides, such as lipids, carbohydrates, nucleic acids, and small molecules, are conjugated to the EV.

As described herein, loading a payload to an EV using the methods provided herein can not only increase the loading efficiency but also improve one or more additional properties of the EVs. As demonstrated herein (*see, e.g.,* Example 6), in some aspects, the loading parameters provided herein (*e.g.,* salt concentration, loading temperature, loading duration, payload feed concentration, EV feed concentration) can improve the stability of the EVs after the loading of the payload. As used herein, the term "stability" refers to the ability of a compound (*e.g.,* EVs loaded with a payload, such as those described herein) to retain its chemical, physical, microbiological, and/or biopharmaceutical properties within specified limits throughout its shelf-life. For example, in some aspects, the payload-loaded EVs remain stable after multiple (*e.g.,* at least two, at least three, at least four, or at least five) freeze-and-thaw treatments. In some aspects, the payload-loaded EVs remain stable and are less likely to aggregate during subsequent purification steps. Not to be bound by any one theory, because of the increased stability, the payload remains associated with the EV once loaded to the EVs. Accordingly, in some aspects, the loading methods provided herein can help decrease the dissociation of a payload from an EV after the EV loading. And, as is apparent from the present disclosure, in some aspects, the increased loading efficiency can improve the potency of the EVs (*see, e.g.,* Example 4).

### II.A. Salt Concentration

In some aspects, the present disclosure provides a method of loading an EV with a payload, comprising mixing the payload with the EV in a buffer (also referred to herein as the "loading buffer") comprising a salt, wherein the concentration of the salt ("salt concentration") is increased compared to a reference buffer (*e.g.,* corresponding buffer that does not comprise the salt or has a salt concentration that is less than that of the loading buffer of the present disclosure). In some aspects, the salt concentration of the loading buffer is greater than about 0.1-fold, greater than about 0.2-fold, greater than about 0.3-fold, greater than about 0.4-fold, greater than about 0.5-fold, greater than about 0.6-fold, greater than about 0.7-fold, greater than about 0.8-fold, greater than about 0.9-fold, greater than about 1-fold, greater than about 2-fold, greater than about 3-fold, greater than about 4-fold, greater than about 5-fold, greater than about 6-fold, greater than about 7-fold, greater than about 8-fold, greater than about 9-fold, greater than about 10-fold, greater than about 15-fold, greater than about 20-fold, greater than about 25-fold, greater than about 30-fold, greater than about 35-fold, greater than about 40-fold, greater than about 45-fold, or greater than about 50-fold, compared to the salt concentration of the reference buffer.

In some aspects, the salt concentration of the loading buffer is at least about 120 mM, at least about 130 mM, at least about 140 mM, at least about 150 mM, at least about 160 mM, at least about 170 mM, at least about 180 mM, or at least about 190 mM. In some aspects, the salt concentration of the loading buffer is less than about 190 mM, less than about 180 mM, less than about 170 mM, less than about 160 mM, less than about 150 mM, less than about 140 mM, less than about 130 mM, or less than about 120 mM.

In some aspects, the salt concentration of the loading buffer is between about 110 mM and about 150 mM, between about 110 mM and about 140 mM, between about 110 mM and about 130 mM, or between about 110 mM and about 120 mM, .

In some aspects, the salt concentration of the loading buffer is about 110 mM, about 120 mM, about 130 mM, about 140 mM, about 150 mM, about 160 mM, about 170 m, about 180 mM, or about 190 mM. In certain aspects, the salt concentration of the loading buffer is about 110 mM, about 120 mM, about 130 mM, about 140 mM, or about 150 mM. In certain aspects, the salt concentration of the loading buffer is about 110 mM. In certain aspects, the salt concentration of the loading buffer is about 120 mM. In some aspects, the salt concentration of the loading buffer is about 130 mM. In some aspects, the salt concentration of the loading buffer is about 140 mM. In some aspects, the salt concentration of the loading buffer is about 150 mM.

As demonstrated herein, the salt concentration of the loading buffer can affect the loading efficiency of a payload (*e.g.,* antisense oligonucleotide) in an EV. For instance, Applicant has identified that an increase in salt concentration (*e.g.,* particularly within the range of salt concentrations disclosed herein) of the loading buffer can increase the loading efficiency of an EV. Accordingly, mixing a payload (*e.g.,* antisense oligonucleotide) with an EV in a loading buffer comprising one or more of the salt concentrations disclosed herein increases the loading of the payload in the EV (*e.g.,* onto the exterior surface, onto the luminal surface, and/or within the lumen) by at least about 2-fold such as at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to loading the payload in an EV in the reference buffer (*e.g.,* does not comprise the salt or comprises the salt but at a lower concentration). As is apparent from the present disclosure, the increase in the loading of the payload results in an increase in the payload concentration of the EV.

For instance, in some aspects, mixing the payload and the EVs in a loading buffer with increased salt concentration (*e.g.,* range of salt concentrations described herein) increases the amount of payload (*e.g.,* number of antisense oligonucleotides) that is associated with the exterior surface of the EV. In some aspects, the amount of payload that is associated with the exterior surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is loaded with the reference buffer). In some aspects, mixing the payload and the EVs in a loading buffer with increased salt concentration increases the amount of payload that is associated with the luminal surface of the EV. In some aspects, the amount of payload that is associated with the luminal surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the luminal surface of the EV when the payload is loaded with the reference buffer). In some aspects, mixing the payload and the EVs in a loading buffer with increased salt concentration increases the amount of payload that is associated with the lumen of the EV. In some aspects, the amount of payload that is associated with the lumen of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload within the lumen of the EV when the payload is loaded with the reference buffer). In some aspects, mixing the payload and the EVs in a loading buffer with increased salt concentration increases the amount of payload that is associated with the exterior surface of the EV, the luminal surface of the EV, the lumen of the EV, or any combination thereof.

The loading buffer described herein can comprise any suitable salts known in the art. In some aspects, the salt comprises a monovalent salt, divalent salt, trivalent salt, or combinations thereof. In certain aspects, the salt comprises a monovalent salt. In some aspects, the salt comprises a divalent salt. In some aspects, the salt comprises a trivalent salt. Non-limiting examples of such salts are provided further below. In some aspects, the salt comprises NaCl (sodium chloride), KCl (potassium chloride), PO₄ (phosphate salt), CaCl₂ (calcium chloride), MgCl₂ (magnesium chloride), Mg₂SO₄ (magnesium sulfate), ZnCl₂ (zinc chloride), MnCl₂ (manganese chloride), MnSO₄ (manganese sulfate), NaSCN (sodium thiocyanate), KSCN (potassium thiocyanate), LiCl (lithium chloride), K₂HPO₄ (dipotassium phosphate), Na₂SO₄ (sodium sulfate), NaPO4 (sodium phosphate), K₂SO₄ (potassium phosphate), sodium acetate, sodium bromide, sodium iodide, potassium bromide, lithium bromide, sodium fluoride, potassium fluoride, lithium fluoride, lithium iodide, potassium acetate, lithium acetate, potassium iodide, calcium sulfate, sodium sulfate, chromium trichloride, chromium sulfate, sodium citrate, iron (III) chloride, yttrium (III) chloride, potassium sulfate, ferrous chloride, calcium citrate, magnesium phosphate, ferric chloride, arginine-HCl, or any combination thereof. In some aspects, the salt comprises NaCl.

In some aspects, increased loading efficiency can be achieved by any of the following: changing salt or salt concentration, changing pH, or adding organic modifiers, organic solvents, small molecules, detergents, zwitterions, amino acids, polymers, polyols (e.g., sucrose, glucose, trehalose, mannose, sorbitol, mannitol, glycerol, etc.) anti-oxidants (e.g., methionine), EDTA, EGTA, Polysorbate 20, Polysorbate 80, ethylene glycol, propylene glycol, polyethylene glycol, polypropylene glycol, and/or urea, adding excipients that alter the polarity of the solution, adding excipients that modulate the structure of the EVs, or any combination thereof.

In some aspects, increased loading efficiency can be achieved by increasing the concentration of a monovalent salt (e.g., sodium chloride, potassium chloride, sodium bromide, lithium chloride, sodium iodide, potassium bromide, lithium bromide, sodium fluoride, potassium fluoride, lithium fluoride, lithium iodide, sodium acetate, potassium acetate, lithium acetate, and potassium iodide), a divalent or trivalent salt (e.g., calcium chloride, magnesium chloride, calcium sulfate, sodium sulfate, magnesium sulfate, chromium trichloride, chromium sulfate, sodium citrate, iron (III) chloride, yttrium (III) chloride, potassium phosphate, potassium sulfate, sodium phosphate, ferrous chloride, calcium citrate, magnesium phosphate, and ferric chloride), or any combination thereof.

### II.B. Loading Temperature

As demonstrated herein, Applicant has further identified that increasing the temperature at which the payload and the EVs are mixed (also referred to herein as "loading temperature") can also increase the loading efficiency of the EV. Accordingly, the present disclosure provides a method of loading an EV with a payload, comprising mixing the payload with the EV at a loading temperature that is higher than about 4 °C. In some aspects, the loading temperature is at least about 5 °C, at least about 10 °C, at least about 15 °C, at least about 20 °C, at least about 25 °C, at least about 30 °C, at least about 35 °C, at least about 37 °C, at least about 40 °C, at least about 45 °C, at least about 50 °C, at least about 55 °C, at least about 60 °C, at least about 65 °C, at least about 70 °C, at least about 75 °C, or at least about 80 °C. In certain aspects, the loading temperature is between about 5 °C and about 40 °C, between about 10 °C and about 40 °C, between about 15 °C and about 40 °C, between about 20 °C and about 40 °C, between about 25 °C and about 40 °C, between about 30 °C and about 40 °C, between about 35 °C and about 40 °C, between about 5 °C and about 38 °C, between about 10 °C and about 38 °C, between about 15 °C and about 38 °C, between about 20 °C and about 38 °C, between about 25 °C and about 38 °C, between about 30 °C and about 38 °C, between about 35 °C and about 38 °C, between about 5 °C and about 37 °C, between about 10 °C and about 37 °C, between about 15 °C and about 37 °C, between about 20 °C and about 37 °C, between about 25 °C and about 37 °C, between about 30 °C and about 37 °C, or between about 35 °C and about 37 °C. In some aspects, the loading temperature is about 5 °C, about 10 °C, about 15 °C, about 20 °C, about 25 °C, about 30 °C, about 35 °C, about 37 °C, about 40 °C, about 45 °C, about 50 °C, about 55 °C, about 60 °C, about 65 °C, about 70 °C, about 75 °C, or about 80 °C.

In some aspects, the loading temperature is between about 37 °C to about 70 °C. In some aspects, the loading temperature is about 37 °C. In some aspects, the loading temperature is about 40 °C. In some aspects, the loading temperature is about 45 °C. In some aspects, the loading temperature is about 50 °C. In some aspects, the loading temperature is about 55 °C. In some aspects, the loading temperature is about 60 °C. In some aspects, the loading temperature is about 65 °C. In some aspects, the loading temperature is about 70 °C. In some aspects, the loading temperature is about 75 °C. In some aspects, the loading temperature is about 80 °C.

In some aspects, mixing the payload and the EVs at a loading temperature described herein increases the loading of the payload in the EV (*e.g.,* onto the exterior surface, onto the luminal surface, and/or within the lumen) by at least about 0.5-fold, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to loading the payload in an EV at a reference temperature (*e.g.,* lower than the loading temperature). As is apparent from the present disclosure, the increase in the loading of the payload results in an increase in the payload concentration of the EV.

In some aspects, increasing the loading temperature at which the payload and the EV are mixed increases the amount of payload (*e.g.,* number of antisense oligonucleotides) that is associated with the exterior surface of the EV. In some aspects, the amount of payload that is associated with the exterior surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is loaded at the reference temperature). In some aspects, increasing the loading temperature at which the payload and the EV are mixed increases the amount of payload that is associated with the luminal surface of the EV. In some aspects, the amount of payload that is associated with the luminal surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the luminal surface of the EV when the payload is loaded at the reference temperature). In some aspects, increasing the loading temperature at which the payload and the EVs are mixed increases the amount of payload that is associated with the lumen of the EVs. In some aspects, the amount of payload that is associated with the lumen of the EVs is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload within the lumen of the EV when the payload is loaded at the reference temperature). In some aspects, increasing the loading temperature at which the payload and the EVs are mixed increases the amount of payload that is associated with the exterior surface of the EVs, the luminal surface of the EVs, the lumen of the EVs, or any combination thereof.

### II.C. Loading Duration

As demonstrated herein, in some aspects, increasing the time in which a payload and the EVs are allowed to mix (also referred to herein as "loading duration") can also increase the loading efficiency of the payload (*e.g.,* antisense oligonucleotide) in the EV. The present disclosure provides a method of loading an EV with a payload, comprising mixing the payload with the EV at a loading duration longer than about one hour. In certain aspects, the loading duration is between longer than about one hour and about 48 hours, between longer than about one hour and about 42 hours, between longer than about one hour and about 36 hours, between longer than about one hour and about 30 hours, between longer than about one hour and about 24 hours, between about six hours and about 48 hours, between about six hours and about 42 hours, between about six hours and about 36 hours, between about six hours and about 30 hours, between about six hours and about 24 hours, between about 12 hours and about 48 hours, between about 12 hours and about 42 hours, between about 12 hours and about 36 hours, between about 12 hours and about 30 hours, or between about 12 hours and about 24 hours. In certain aspects, the loading duration is about six hours, about 12 hours, about 24 hours, about 30 hours, about 36 hours, about 42 hours, or about 48 hours.

In some aspects, the loading duration is between longer than about one hour and about 10 days. In some aspects, the loading duration is between about 24 hours and about seven days. In some aspects, the loading duration is about six hours. In some aspects, the loading duration is about 12 hours. In some aspects, the loading duration is about 24 hours. In some aspects, the loading duration is about two days, In some aspects, the loading duration is about three days. In some aspects, the loading duration is about four days, In some aspects, the loading duration is about five days. In some aspects, the loading duration is about six days. In some aspects, the loading duration is about seven days. In some aspects, the loading duration is about eight days. In some aspects, the loading duration is about nine days. In some aspects, the loading duration is about 10 days.

In some aspects, mixing the payload and the EVs at a loading duration described herein (*e.g.,* at least about 24 hours) increases the loading of the payload in the EV (*e.g.,* onto the exterior surface, onto the luminal surface, and/or within the lumen) by at least about 0.5-fold, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to loading the payload in an EV at a reference duration (*e.g.,* shorter than the loading duration). As is apparent from the present disclosure, the increase in the loading of the payload results in an increase in the payload concentration of the EV.

In some aspects, increasing the loading duration increases the amount of payload (*e.g.,* number of antisense oligonucleotides) that is associated with the exterior surface of the EV. In some aspects, the amount of payload that is associated with the exterior surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV for the reference duration). In some aspects, increasing the loading duration increases the amount of payload that is associated with the luminal surface of the EV. In some aspects, the amount of payload that is associated with the luminal surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the luminal surface of the EV when the payload is mixed with the EV for the reference duration). In some aspects, increasing the loading duration increases the amount of payload that is associated with the lumen of the EVs. In some aspects, the amount of payload that is associated with the lumen of the EVs is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the luminal surface of the EV when the payload is mixed with the EV for the reference duration). In some aspects, increasing the loading duration increases the amount of payload that is associated with the exterior surface of the EVs, the luminal surface of the EVs, the lumen of the EVs, or any combination thereof.

### II.D. Payload Feed Concentration

In some aspects, a method of loading an EV with a payload (*e.g.,* antisense oligonucleotide) provided herein comprises increasing the payload feed concentration at which the EV and the payload are mixed, wherein the increase in the payload feed concentration enhances the loading efficiency of the payload. As used herein, the term "payload feed concentration" refers to the amount of payload that is mixed with EVs in the loading methods described herein.

In some aspects, the payload feed concentration is at least about 10 µM, at least about 20 µM, at least about 30 µM, at least about 40 µM, at least about 50 µM, at least about 60 µM, at least about 70 µM, at least about 80 µM, at least about 90 µM, at least about 100 µM, at least about 150 µM, at least about 200 µM, at least about 250 µM, at least about 300 µM, at least about 350 µM, at least about 400 µM, at least about 450 µM, at least about 500 µM, at least about 550 µM, at least about 600 µM, at least about 650 µM, at least about 700 µM, at least about 750 µM, at least about 800 µM, at least about 850 µM, at least about 900 µM, at least about 950 µM, at least about 1000 µM, at least about 1050 µM, at least about 1100 µM, at least about 1150 µM, at least about 1200 µM, at least about 1250 µM, at least about 1300 µM, at least about 1350 µM, at least about 1400 µM, at least about 1450 µM, at least about 1500 µM, at least about 1600 µM, at least about 1700 µM, at least about 1800 µM, at least about 1900 µM, at least about 2000 µM, at least about 2100 µM, at least about 2200 µM, at least about 2300 µM, at least about 2400 µM, at least about 2500 µM, at least about 2600 µM, at least about 2700 µM, at least about 2800 µM, at least about 2900 µM, or at least about 3000 µM. In some aspects, the payload feed concentration is about 600 µM. In some aspects, the payload feed concentration is about 800 µM.

In some aspects, the payload feed concentration is between about 50 µM and about 1000 µM, between about 100 µM and about 1000 µM, between about 200 µM and about 1000 µM, between about 300 µM and about 1000 µM, between about 400 µM and about 1000 µM, between about 500 µM and about 1000 µM, between about 50 µM and about 900 µM, between about 100 µM and about 900 µM, between about 200 µM and about 900 µM, between about 300 µM and about 900 µM, between about 400 µM and about 900 µM, between about 500 µM and about 900 µM, between about 50 µM and about 800 µM, between about 100 µM and about 800 µM, between about 200 µM and about 800 µM, between about 300 µM and about 800 µM, between about 400 µM and about 800 µM, between about 500 µM and about 800 µM, between about 50 µM and about 700 µM, between about 100 µM and about 700 µM, between about 200 µM and about 700 µM, between about 300 µM and about 700 µM, between about 400 µM and about 700 µM, or between about 500 µM and about 700. In some aspects, the payload feed concentration is between about 1000 µM and about 1100 µM, between about 1200 µM and about 1300 µM, between about 1300 µM and about 1400 µM, between about 1400 µM and about 1500 µM, between about 1500 µM and about 1600 µM, between about 1600 µM and about 1700 µM, between about 1700 µM and about 1800 µM, between about 1800 µM and about 1900 µM, between about 1900 µM and about 2000 µM, between about 2000 µM and about 2100 µM, between about 2100 µM and about 2200 µM, between about 2200 µM and about 2300 µM, between about 2300 µM and about 2400 µM, between about 2400 µM and about 2500 µM, between about 2500 µM and about 2600 µM, between about 2600 µM and about 2700 µM, between about 2700 µM and about 2800 µM, between about 2800 µM and about 2900 µM, or between about 2900 µM and about 3000 µM.

As described herein, in some aspects, increasing the payload feed concentration increases the loading of the payload in the EV (*e.g.,* onto the exterior surface, onto the luminal surface, and/or within the lumen) by at least about 0.5-fold, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared a reference payload feed concentration (*e.g.,* less than the payload feed concentration described herein). As is apparent from the present disclosure, the increase in the loading of the payload results in an increase in the payload concentration of the EV.

In some aspects, increasing the payload feed concentration increases the amount of payload (*e.g.,* number of antisense oligonucleotides) that is associated with the exterior surface of the EV. In some aspects, the amount of payload that is associated with the exterior surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference payload feed concentration). In some aspects, increasing the payload feed concentration increases the amount of payload that is associated with the luminal surface of the EV. In some aspects, the amount of payload that is associated with the luminal surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference payload feed concentration). In some aspects, increasing the payload feed concentration increases the amount of payload that is associated with the lumen of the EVs. In some aspects, the amount of payload that is associated with the lumen of the EVs is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference payload feed concentration). In some aspects, increasing the payload feed concentration increases the amount of payload that is associated with the exterior surface of the EVs, the luminal surface of the EVs, the lumen of the EVs, or any combination thereof.

### II.E. EV Feed Concentration

In some aspects, the present disclosure provides a method of loading an EV with a payload (*e.g.,* antisense oligonucleotide), comprising mixing the payload and the EV at an increased EV feed concentration, wherein the increase in the EV feed concentration enhances the loading efficiency of the payload. As used herein, the term "EV feed concentration" refers to the amount of EVs that is mixed with a payload in the loading methods of the present disclosure.

In some aspects, the EV feed concentration is at least about 1x10¹¹ particles/mL (p/mL), at least about 2x10¹¹ p/mL, at least about 3x10¹¹ p/mL, at least about 4x10¹¹ p/mL, at least about 5x10¹¹ p/mL, at least about 6x10¹¹ p/mL, at least about 7x10¹¹ p/mL, at least about 8x10¹¹ p/mL, at least about 9x10¹¹ p/mL, at least about 0.1x10¹³ p/mL, at least about 0.2x10¹³ p/mL, at least about 0.3x10¹³ p/mL, at least about 0.4x10¹³ p/mL, at least about 0.5x10¹³ p/mL, at least about 0.6x10¹³ p/mL, at least about 0.7x10¹³ p/mL, at least about 0.8x10¹³ p/mL, at least about 0.9x10¹³ p/mL, at least about 1.0x10¹³ p/mL, at least about 1.1x10¹³ p/mL, at least about 1.2x10¹³ p/mL, at least about 1.3x10¹³ p/mL, at least about 1.4x10¹³ p/mL, at least about 1.5x10¹³ p/mL, at least about 1.6x10¹³ p/mL, at least about 1.7x10¹³ p/mL, at least about 1.8x10¹³ p/mL, at least about 1.9x10¹³ p/mL, at least about 2.0x10¹³ p/mL, at least about 2.1x10¹³ p/mL, at least about 2.2x10¹³ p/mL, at least about 2.3x10¹³ p/mL, at least about 2.4x10¹³ p/mL, at least about 2.5x10¹³ p/mL, at least about 2.6x10¹³ p/mL, at least about 2.7x10¹³ p/mL, at least about 2.8x10¹³ p/mL, at least about 2.9x10¹³ p/mL, at least about 3.0x10¹³ p/mL, at least about 4x10¹³ p/mL, at least about 5x10¹³ p/mL, at least about 6x10¹³ p/mL, at least about 7x10¹³ p/mL, at least about 8x10¹³ p/mL, at least about 9x10¹³ p/mL, or at least about 1x10¹⁴ p/mL. In certain aspects, the EV feed concentration is about 1.0x10¹³ p/mL. In some aspects, the EV feed concentration is about 1.4x10¹³ p/mL. In some aspects, the EV feed concentration is about 6.0x10¹² p/mL.

In some aspects, the EV feed concentration is between about 0.2x10¹³ p/mL and about 2.0x10¹³ p/mL, between about 0.4x10¹³ p/mL and about 2.0x10¹³ p/mL, between about 0.6x10¹³ p/mL and about 2.0x10¹³ p/mL, between about 0.8x10¹³ p/mL and about 2.0x10¹³ p/mL, between about 1.0x10¹³ p/mL and about 2.0x10¹³ p/mL, between about 0.2x10¹³ p/mL and about 1.8x10¹³ p/mL, between about 0.4x10¹³ p/mL and about 1.8x10¹³ p/mL, between about 0.6x10¹³ p/mL and about 1.8x10¹³ p/mL, between about 0.8x10¹³ p/mL and about 1.8x10¹³ p/mL, between about 1.0x10¹³ p/mL and about 1.8x10¹³ p/mL, between about 0.2x10¹³ p/mL and about 1.6x10¹³ p/mL, between about 0.4x10¹³ p/mL and about 1.6x10¹³ p/mL, between about 0.6x10¹³ p/mL and about 1.6x10¹³ p/mL, between about 0.8x10¹³ p/mL and about 1.6x10¹³ p/mL, between about 1.0x10¹³ p/mL and about 1.6x10¹³ p/mL, between about 0.2x10¹³ p/mL and about 1.4x10¹³ p/mL, between about 0.4x10¹³ p/mL and about 1.4x10¹³ p/mL, between about 0.6x10¹³ p/mL and about 1.4x10¹³ p/mL, between about 0.8x10¹³ p/mL and about 1.4x10¹³ p/mL, between about 1.0x10¹³ p/mL and about 1.4x10¹³ p/mL, between about 0.2x10¹³ p/mL and about 1.2x10¹³ p/mL, between about 0.4x10¹³ p/mL and about 1.2x10¹³ p/mL, between about 0.6x10¹³ p/mL and about 1.2x10¹³ p/mL, between about 0.8x10¹³ p/mL and about 1.2x10¹³ p/mL, or between about 1.0x10¹³ p/mL and about 1.2x10¹³ p/mL. In some aspects, the EV feed concentration is between about 2x10¹³ p/mL and about 1x10¹⁴ p/mL, between about 3x10¹³ p/mL and about 1x10¹⁴ p/mL, between about 4x10¹³ p/mL and about 1x10¹⁴ p/mL, between about 5x10¹³ p/mL and about 1x10¹⁴ p/mL, between about 6x10¹³ p/mL and about 1x10¹⁴ p/mL, between about 7x10¹³ p/mL and about 1x10¹⁴ p/mL, between about 8x10¹³ p/mL and about 1x10¹⁴ p/mL, or between about 9x10¹³ p/mL and about 1x10¹⁴ p/mL.

In some aspects, increasing the EV feed concentration increases the loading of the payload in the EV (*e.g.,* onto the exterior surface, onto the luminal surface, and/or within the lumen) by at least about 0.5-fold, at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared a reference EV feed concentration (*e.g.,* less than the feed concentration described herein). As is apparent from the present disclosure, the increase in the loading of the payload results in an increase in the payload concentration of the EV.

In some aspects, increasing the EV feed concentration increases the amount of payload (*e.g.,* number of antisense oligonucleotides) that is associated with the exterior surface of the EV. In some aspects, the amount of payload that is associated with the exterior surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference EV feed concentration). In some aspects, increasing the EV feed concentration increases the amount of payload that is associated with the luminal surface of the EV. In some aspects, the amount of payload that is associated with the luminal surface of the EV is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference EV feed concentration). In some aspects, increasing the EV feed concentration increases the amount of payload that is associated with the lumen of the EVs. In some aspects, the amount of payload that is associated with the lumen of the EVs is increased by at least about 1-fold, at least about 2-fold, at least about 3-fold, at least about 4-fold, at least about 5-fold, at least about 6-fold, at least about 7-fold, at least about 8-fold, at least *a*bout 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g.,* amount of payload on the exterior surface of the EV when the payload is mixed with the EV at the reference EV feed concentration). In some aspects, increasing the EV feed concentration increases the amount of payload that is associated with the exterior surface of the EVs, the luminal surface of the EVs, the lumen of the EVs, or any combination thereof.

As is apparent from the present disclosure, in some aspects, a loading method of the present disclosure can comprise modulating multiple (*e.g.,* two or more) loading parameters, such as those described herein. In some aspects, the salt concentration, loading temperature, loading duration, payload feed concentration, EV feed concentration, or combinations thereof, are increased.

The method of loading EVs with a payload provided herein comprises increasing both the salt concentration of the loading buffer (i.e. a salt a concentration between about 110 mM to lower than about 200 mM; e.g., between about 150 mM to lower than about 200 mM) and the loading temperature (i.e. higher than 4 °C; *e.g.,* about 37 °C or higher). In some aspects, when the payload and EVs are mixed under such a condition, the loading efficiency is increased by at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to the loading efficiency observed when the payload and the EV are mixed: (i) in a loading buffer with reduced salt concentration; (ii) at a decreased loading temperature; or (iii) in a loading buffer with reduced salt concentration and at a decreased loading temperature.

In some aspects, loading methods provided herein can comprise modulating multiple loading parameters to increase the loading efficiency of a payload in an EV. For example, the method of loading EVs with a payload provided herein comprises increasing both the salt concentration of the loading buffer (i.e. a salt a concentration between about 110 mM to lower than about 200 mM; *e.g.,* about 150 mM to lower than about 200 mM) and the loading duration (i.e. longer than about one hour; *e.g.,* for at least about 24 hours). Further, the method comprises increasing the loading temperature (i.e. higher than 4 °C; *e.g.,* about 37 °C or higher). In some aspects, when the payload and EVs are mixed under such a condition, the loading efficiency is increased by at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to the loading efficiency observed when the payload and the EV are mixed: (i) in a loading buffer with reduced salt concentration; (ii) at a decreased loading temperature; (iii) for shorter loading duration; or (iv) in any combination of (i) to (iii).

In some aspects, loading methods provided herein can comprise increasing both the salt concentration of the loading buffer (i.e. a salt a concentration between about 110 mM to lower than about 200 mM; *e.g.,* about 150 mM to lower than about 200 mM) and the payload feed concentration (*e.g.,* about 800 µM or more). In some aspects, a loading method comprises increasing both the loading temperature (i.e. higher than 4 °C; *e.g.,* about 37 °C or higher) and the payload feed concentration (*e.g.,* about 800 µM or more). In some aspects, a method of loading an EV with a payload provided herein comprises increasing both the loading duration (i.e. longer than about one hour; *e.g.,* for at least about 24 hours) and the payload feed concentration (*e.g.,* about 800 µM or more). In some aspects, to increase the loading efficiency, loading method provided herein comprises increasing the salt concentration of the loading buffer (i.e. a salt a concentration between about 110 mM to lower than about 200 mM; *e.g.,* about 150 mM to lower than about 200 mM), the loading temperature (i.e. higher than 4 °C; *e.g.,* about 37 °C or higher), the loading duration (i.e. longer than about one hour; *e.g.,* for at least about 24 hours), and the payload feed concentration (*e.g.,* about 800 µM or more). In some aspects, when the payload and EVs are mixed under such a condition, the loading efficiency is increased by at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to the loading efficiency observed when the payload and the EV are mixed: (i) in a loading buffer with reduced salt concentration; (ii) at a decreased loading temperature; (iii) for shorter loading duration; (iv) with reduced payload feed concentration; or (v) in any combination of (i) to (iv).

In some aspects, a method of loading an EV with a payload provided herein comprises increasing both the salt concentration of the loading buffer (i.e. a salt a concentration between about 110 mM to lower than about 200 mM; *e.g.,* about 150 mM to lower than about 200 mM) and the EV feed concentration (*e.g.,* about 1.4x10¹³ p/mL or higher). In some aspects, a loading method comprises increasing both the loading temperature (i.e. higher than 4 °C; *e.g.,* about 37 °C or higher) and the EV feed concentration (*e.g.,* about 1.4x10¹³ p/mL or higher). In some aspects, a loading method comprises increasing both the loading duration (i.e. longer than about one hour; *e.g.,* for at least about 24 hours) and the EV feed concentration (*e.g.,* about 1.4x10¹³ p/mL or higher). In some aspects, the loading method comprises increasing both the payload feed concentration (*e.g.,* about 800 µM or more) and the EV feed concentration (*e.g.,* about 1.4x10¹³ p/mL or higher). In some aspects, an EV loading method described herein comprises increasing: the salt concentration of the loading buffer (i.e. a salt a concentration between about 110 mM to lower than about 200 mM; *e.g.,* about 150 mM to lower than about 200 mM), the loading temperature (i.e. higher than 4 °C; *e.g.,* about 37 °C or higher), the loading duration (i.e. longer than about one hour; *e.g.,* for at least about 24 hours), the payload feed concentration (*e.g.,* about 800 µM or more), and the EV feed concentration (*e.g.,* about 1.4x10¹³ p/mL or higher). In some aspects, when the payload and EVs are mixed under such a condition, the loading efficiency is increased by at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to the loading efficiency observed when the payload and the EV are mixed: (i) in a loading buffer with reduced salt concentration; (ii) at a decreased loading temperature; (iii) for shorter loading duration; (iv) with reduced payload feed concentration; (v) with reduced EV feed concentration; or (vi) in any combination of (i) to (v).

As demonstrated herein, it is not necessary to adjust (*e.g.,* increase) all of the loading parameters provided above (salt concentration, loading temperature, loading duration, payload feed concentration, and EV feed concentration) to increase the loading efficiency of an EV. In some aspects, increasing one or more of the loading parameters described herein can allow other loading parameters to remain constant or reduced without negatively affecting loading efficiency. It will be apparent to those skilled in the arts based on the disclosures provided herein, that increasing the loading duration can allow other loading parameters (*e.g.,* salt concentration, loading temperature, payload feed concentration, and/or EV feed concentration) to remain constant or be reduced without negatively affecting loading efficiency.

In some aspects, (i) the salt concentration of the loading buffer is between about 110 mM to less than about 200 mM, (ii) the loading temperature is between about 5 °C and about 40 °C, and (iii) the loading duration is between higher than about one hour and about 48 hours. In certain aspects, (i) the salt concentration is between about 110 mM and about 150 mM, (ii) the loading temperature is between about 15 °C and about 40 °C, and (iii) the loading duration is between about 12 hours and about 36 hours. In certain aspects, (i) the salt concentration is about 110 mM, (ii) the loading temperature is about 37 °C, and (iii) the loading duration is about 24 hours. In some aspects, (i) the salt concentration is between about 150 mM and lower than about 200 mM, (ii) the loading temperature is between about 37 °C to about 70 °C, and (iii) the loading duration is between about 24 hours and about seven days.

In some aspects, the above exemplary method further comprises increasing the payload feed concentration, the EV feed concentration, or both. Accordingly, in certain aspects, (i) the salt concentration of the loading buffer is less than about 200 mM, (ii) the loading temperature is between about 5 °C and about 40 °C, (iii) the loading duration is between longer than about one hour and about 48 hours, (iv) the payload feed concentration is between about 50 µM and about 1000 µM, and (v) the EV feed concentration is between about 0.2x10¹³ p/mL and about 2.0x10¹³ p/mL. In certain aspects, (i) the salt concentration is between about 110 mM and about 150 mM, (ii) the loading temperature is between about 15 °C and about 40 °C, (iii) the loading duration is between about 12 hours and about 36 hours, (iv) the payload feed concentration is between about 100 µM and about 1000 µM, and (v) the EV feed concentration is between about 0.5x10¹³ p/mL and about 2.0x10¹³ p/mL. In some aspects, (i) the salt concentration is about 110 mM, (ii) the loading temperature is about 37 °C, (iii) the loading duration is about 24 hours, (iv) the payload feed concentration is about 600 µM, and (v) the EV feed concentration is about 1.0x10¹³ p/mL. In some aspects, (i) the salt concentration is between about 150 mM and less than about 200 mM, (ii) the loading temperature is between about 37 °C to about 70 °C, (iii) the loading duration is between about 24 hours and about seven days, (iv) the payload feed concentration is between about 6x10¹² p/mL and about 1.4x10¹³ p/mL.

In some aspects, loading an EV with a payload (*e.g.,* antisense oligonucleotide) using the above recited salt concentration, loading temperature, loading duration, payload feed concentration, and/or EV feed concentration increases the loading of the payload in the EV (*e.g.,* onto the exterior surface, onto the luminal surface, and/or within the lumen) by at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to loading the payload with a reduced salt concentration (*e.g.,* buffer does not comprise a salt), decreased loading temperature (*e.g.,* about 4 °C or less), shorter loading duration (*e.g.,* about one hour or less), reduced payload feed concentration (*e.g.,* less than about 50 µM), and/or reduced EV feed concentration (*e.g.,* less than about 0.5x10¹³ p/mL).

In some aspects, using the above recited salt concentration, loading temperature, loading duration, payload feed concentration, and/or EV feed concentration increases the amount of payload (*e.g.,* number of antisense oligonucleotides) that is associated with the exterior surface of the EV, *e.g.,* by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g*., amount of payload on the exterior surface of the EV when the payload is loaded using reduced salt concentration (*e.g.,* buffer does not comprise a salt), decreased loading temperature (*e.g.,* about 4 °C or less), shorter loading duration (*e.g.,* about one hour or less), reduced payload feed concentration (*e.g.,* less than about 50 µM), and/or reduced EV feed concentration (*e.g.,* less than about 0.5x10¹³ p/mL)). In some aspects, using the above recited salt concentration, loading temperature, loading duration, payload feed concentration, and/or EV feed concentration increases the amount of payload (*e.g.,* number of antisense oligonucleotides) that is associated with the luminal surface of the EV, *e.g.,* by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g*., amount of payload on the luminal surface of the EV when the payload is loaded using reduced salt concentration (*e.g.,* buffer does not comprise a salt), decreased loading temperature (*e.g.,* about 4 °C or less), shorter loading duration (*e.g.,* about one hour or less), reduced payload feed concentration (*e.g.,* less than about 50 µM), and/or reduced EV feed concentration (*e.g.,* less than about 0.5x10¹³ p/mL)). In some aspects, using the above recited salt concentration, loading temperature, loading duration, payload feed concentration, and/or EV feed concentration increases the amount of payload (*e.g.,* number of antisense oligonucleotides) that is associated with the lumen of the EV, *e.g.,* by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference amount (*e.g*., amount of payload within the lumen of the EV when the payload is loaded using reduced salt concentration (*e.g.,* buffer does not comprise a salt), decreased loading temperature (*e.g.,* about 4 °C or less), shorter loading duration (*e.g.,* about one hour or less), reduced payload feed concentration (*e.g.,* less than about 50 µM), and/or reduced EV feed concentration (*e.g.,* less than about 0.5x10¹³ p/mL)).

### II.F. Other Loading Parameters

### II.F.1. Sucrose Concentration

In some aspects, the loading methods described herein can further comprise modulating one or more additional parameters during the loading process. For instance, in some aspects, a loading buffer useful for the present disclosure (*e.g.,* comprising a salt within the range of concentrations recited herein) further comprises one or more components. Non-limiting examples of such one or more components include a sucrose, histidine, arginine, methionine, sodium phosphate, potassium phosphate, or any combination thereof.

In some aspects, a loading buffer that can be used with the present disclosure additionally comprises a sucrose. In certain aspects, the concentration of the sucrose in the loading buffer is at least about 1 % w/v, at least about 2 % w/v, at least about 3% w/v, at least about 4% w/v, at least about 5% w/v, at least about 6% w/v, at least about 7% w/v, at least about 8% w/v, at least about 9% w/v, or at least about 10% w/v. In some aspects, the concentration of the sucrose is about 10% w/v, about 9% w/v, about 8% w/v, about 7% w/v, about 6% w/v, about 5% w/v, about 4% w/v, about 3% w/v, about 2% w/v or about 1% w/v.

In some aspects, the concentration of the sucrose in the loading buffer is between about 1 % w/v and about 10 % w/v, between about 2 % w/v and about 10 % w/v, between about 3 % w/v and about 10 % w/v, between about 4 % w/v and about 10 % w/v, between about 5 % w/v and about 10 % w/v, between about 1% w/v and about 9% w/v, between about 2% w/v and about 9% w/v, between about 3% w/v and about 9% w/v, between about 4% w/v and about 9% w/v, between about 5% w/v and about 9% w/v, between about 1% w/v and about 8% w/v, between about 2% w/v and about 8% w/v, between about 3% w/v and about 8% w/v, between about 4% w/v and about 8% w/v, between about 5% w/v and about 8% w/v, between about 1% w/v and about 7% w/v, between about 2% w/v and about 7% w/v, between about 3% w/v and about 7% w/v, between about 4% w/v and about 7% w/v, between about 5% w/v and about 7% w/v, between about 1% w/v and about 6% w/v, between about 2% w/v and about 6% w/v, between about 3% w/v and about 6% w/v, between about 4% w/v and about 6% w/v, or between about 5% w/v and about 6% w/v. In certain aspects, the sucrose concentration of the loading buffer is about 5% w/v.

### II.F.2. Osmolarity

In some aspects, a loading method described herein comprises modulating the osmolarity of the loading buffer used to mix a payload and the EV as described herein. In certain aspects, the osmolarity of the loading buffer is between about 100 mOsm/kg to about 600 mOsm/kg (*e.g.,* between about 250 mOsm/kg to about 500 mOsm/kg). In certain aspects, the osmolarity of the loading buffer is between about 275 mOsm/kg and about 450 mOsm/kg, between about 280 mOsm/kg and about 450 mOsm/kg, between about 300 mOsm/kg and about 450 mOsm/kg, between about 275 mOsm/kg and about 400 mOsm/kg, between about 280 mOsm/kg and about 400 mOsm/kg, between about 300 mOsm/kg and about 400 mOsm/kg, between about 275 mOsm/kg and about 380 mOsm/kg, between about 280 mOsm/kg and about 380 mOsm/kg, between about 300 mOsm/kg and about 380 mOsm/kg, between about 275 mOsm/kg and about 350 mOsm/kg, between about 280 mOsm/kg and about 350 mOsm/kg, between about 300 mOsm/kg and about 350 mOsm/kg, between about 275 mOsm/kg and about 310 mOsm/kg, between about 280 mOsm/kg and about 310 mOsm/kg, or between about 300 mOsm/kg and about 310 mOsm/kg.

In some aspects, the osmolarity of the loading buffer is about 360 mOsm/kg, about 370 mOsm/kg, about 380 mOsm/kg, about 390 mOsm/kg, about 395 mOsm/kg, or about 400 mOsm/kg. In some aspects, the osmolarity of the loading buffer is about 395 mOsm/kg.

### II.F.3. pH

In some aspects, a loading buffer which can be used (*e.g.,* to mix a payload with EVs) with the present disclosure has a pH between about 6 and about 8. In certain aspects, the pH of the loading buffer is between about 6 and about 7 or between about 7 and about 8. In some aspects, the pH is about 6, about 6.1, about 6.2, about 6.3, about 6.4, about 6.5, about 6.6, about 6.7, about 6.8, about 6.9, about 7, about 7.1, about 7.2, about 7.3, about 7.4, about 7.5, about 7.6, about 7.7, about 7.8, about 7.9, or about 8. In certain aspects, the pH of the loading buffer is about 7.2. In some aspects, a loading buffer useful for the present disclosure has a pH of about 9.

### II.G. Payload

As will be apparent to those skilled in the arts, the loading methods provided herein can be used to load an EV with any suitable payload known in the art. Non-limiting examples of payloads that can be loaded in an EV using the methods described herein include a peptide, a small molecule, an oligonucleotide, an antisense oligonucleotide (ASO), a phosphorodiamidate morpholino oligomer (PMO), an mRNA, an miRNA, an lcRNA, an antagomir, a tRNA, a siRNA, a peptide nuclei acid, a cell penetrating peptide, an adjuvant, a protein, a carbohydrate, a sugar, an amino acid, or any combination thereof. In some aspects, the payload comprises a nucleic acid. In some aspects, the payload comprises a peptide. In some aspects, the payload comprises a small molecule. In certain aspects, the payload comprises an antisense oligonucleotide (ASO) (also referred to herein as "antisense ASO" and "oligomer"). In some aspects, the payload comprises a mRNA. In some aspects, the payload comprises a miRNA. In some aspects, the payload comprises a lcRNA. In some aspects, the payload comprises an antagomir. In some aspects, the payload comprises a tRNA. In some aspects, the payload comprises a siRNA. In some aspects, the payload comprises a peptide nucleic acid. In some aspects, the payload comprises a cell penetrating peptide. In some aspects, the payload comprises an adjuvant. In some aspects, a payload comprises a protein. In some aspects, a payload comprises a carbohydrate. In some aspects, a payload comprises a sugar. In some aspects, a payload comprises an amino acid. In certain aspects, the payload contains a domain (*e.g.,* charged substituents) designed to increase the solubility of the payload. Non-limiting examples of such domains include cell penetrating peptides, polyanions (*e.g.,* polyglutamic acid), polycations (*e.g.,* polylysine, polyarginine, polyethylenimine), and combinations thereof. Accordingly, the loading methods described herein can be used to increase the number of ASOs that are associated with the exterior surface of an EV.

In some aspects, a payload (*e.g.,* ASO) useful for the present disclosure comprises a contiguous nucleotide sequence of from about 10 to about 30, such as 10-20, 14-20, 16-20, or 15-25, nucleotides in length. In certain aspects, the payload is 20 nucleotides in length. In certain aspects, the payload is 18 nucleotides in length. In certain aspects, the payload is 19 nucleotides in length. In certain aspects, the payload is 17 nucleotides in length. In certain aspects, the payload is 16 nucleotides in length. In certain aspects, the payload is 15 nucleotides in length. In some aspects, the payload is 14 nucleotides in length. In some aspects, the payload is 13 nucleotides in length. In certain aspects, the payload is 12 nucleotides in length. In some aspects, the payload is 11 nucleotides in length. In further aspects, the payload is 10 nucleotides in length.

In some aspects, the payload comprises a contiguous nucleotide sequence of from about 10 to about 50 nucleotides in length, e.g., about 10 to about 45, about 10 to about 40, about 10 or about 35, or about 10 to about 30. In certain aspects, the payload is 21 nucleotides in length. In certain aspects, the payload is 22 nucleotides in length. In certain aspects, the payload is 23 nucleotides in length. In certain aspects, the payload is 24 nucleotides in length. In certain aspects, the payload is 25 nucleotides in length. In certain aspects, the payload is 26 nucleotides in length. In certain aspects, the payload is 27 nucleotides in length. In certain aspects, the payload is 28 nucleotides in length. In certain aspects, the payload is 29 nucleotides in length. In certain aspects, the payload is 30 nucleotides in length. In certain aspects, the payload is 31 nucleotides in length. In certain aspects, the payload is 32 nucleotides in length. In certain aspects, the payload is 33 nucleotides in length. In certain aspects, the payload is 34 nucleotides in length. In certain aspects, the payload is 35 nucleotides in length. In certain aspects, the payload is 36 nucleotides in length. In certain aspects, the payload is 37 nucleotides in length. In certain aspects, the payload is 38 nucleotides in length. In certain aspects, the payload is 39 nucleotides in length. In certain aspects, the payload is 40 nucleotides in length. In certain aspects, the payload is 41 nucleotides in length. In certain aspects, the payload is 42 nucleotides in length. In certain aspects, the payload is 43 nucleotides in length. In certain aspects, the payload is 44 nucleotides in length. In certain aspects, the payload is 45 nucleotides in length. In certain aspects, the payload is 46 nucleotides in length. In certain aspects, the payload is 47 nucleotides in length. In certain aspects, the payload is 48 nucleotides in length. In certain aspects, the payload is 49 nucleotides in length. In certain aspects, the payload is 50 nucleotides in length.

In various aspects, the payload (*e.g.,* ASO) of the disclosure does not comprise RNA (units). In some aspects, the payload comprises one or more DNA units. In certain aspects, the payload useful for the present disclosure is a linear molecule or is synthesized as a linear molecule. In some aspects, the payload is a single stranded molecule, and does not comprise short regions of, for example, at least 3, 4 or 5 contiguous nucleotides, which are complementary to equivalent regions within the same payload (*i.e.* duplexes) - in this regard, the payload is not (essentially) double stranded. In some aspects, the payload is essentially not double stranded. In some aspects, the payload is not a siRNA. In various aspects, the payload that can be used with the loading methods described herein consists entirely of the contiguous nucleotide region. Thus, in some aspects, the payload is not substantially self-complementary.

In some aspects, the present disclosure includes fragments of payloads (*e.g.,* ASOs). For example, the disclosure includes at least one nucleotide, at least two contiguous nucleotides, at least three contiguous nucleotides, at least four contiguous nucleotides, at least five contiguous nucleotides, at least six contiguous nucleotides, at least seven contiguous nucleotides, at least eight contiguous nucleotides, or at least nine contiguous nucleotides of the payload (*e.g.,* ASOs) disclosed herein. Fragments of any of the sequences disclosed herein are contemplated as part of the disclosure.

In some aspects, a payload useful for the present disclosure comprises one or more nucleoside analogs. In certain aspects, one or more of the nucleoside analogs comprise a 2'-O-alkyl-RNA; 2'-O-methyl RNA (2'-OMe); 2'-alkoxy-RNA; 2'-O-methoxyethyl-RNA (2'-MOE); 2'-amino-DNA; 2'-fluro-RNA; 2'-fluoro-DNA; arabino nucleic acid (ANA); 2'-fluoro-ANA; bicyclic nucleoside analog (LNA), or any combination thereof. In certain aspects, one or more of the nucleoside analogs are a sugar modified nucleoside. In further aspects, the sugar modified nucleoside is an affinity enhancing 2' sugar modified nucleoside. In some aspects, one or more of the nucleoside analogs comprises a nucleoside comprising a bicyclic sugar. In certain aspects, one or more of the nucleoside analogs comprises an LNA. In further aspects, one or more of the nucleoside analogs are selected from the group consisting of constrained ethyl nucleoside (cEt), 2',4'-constrained 2'-O-methoxyethyl (cMOE), α-L-LNA, β-D-LNA, 2'-O,4'-C-ethylene-bridged nucleic acids (ENA), amino-LNA, oxy-LNA, thio-LNA, and any combination thereof.

In some aspects, the payload that can be loaded in an EV using the loading methods provided herein comprises a phosphorodiamidate morpholino oligomer (PMO), a peptide-conjugated phosphorodiamidate morpholino oligomer (PPMO), or both.

### II.G.1. The Target

As will be apparent to those skilled in the arts, a payload targeting any specific molecule (*e.g.,* gene) of interest can be used with the present disclosure. For instance, in some aspects, the loading methods provided herein can be used for any molecule (*e.g.,* gene) where a payload (*e.g.,* ASO) that specifically binds to the molecule can be generated. Accordingly, in some aspects, payloads (*e.g.,* ASO) useful for the present disclosure are capable of down-regulating (*e.g.,* reducing or inhibiting) the expression of any gene or protein of interest in a cell. Non-limiting examples of such targets are described further below.

### II.G.1.a. STAT6

In some aspects, the payloads (*e.g.,* ASOs) provided herein are capable of down-regulating (*e.g.,* reducing or inhibiting) the expression of any gene or protein of interest in a cell. In certain aspects, the payloads described herein *(e.g.,* can be loaded in EVs using the loading methods of the present disclosure) target one or more regions of the *STAT6* pre-mRNA *(e.g.,* intron regions, exon regions, and/or exon-intron junction regions). In some aspects, the payload is capable of targeting all isoforms of the STAT6 protein. In certain aspects, the payload targets specific STAT6 isoforms (*e.g.,* Isoform 1 and Isoform 2, Isoform 1 and Isoform 3, or Isoform 2 and Isoform 3). Unless indicated otherwise, the term "STAT6," as used herein, can refer to STAT6 from one or more species (*e.g.,* humans, non-human primates, dogs, cats, guinea pigs, rabbits, rats, mice, horses, cattle, and bears).

STAT6 (*STAT6*) is also known as signal transducer and activator of transcription 6. Synonyms of *STAT6*/*STAT6* are known and include IL-4 STAT; STAT, Interleukin4-Induced; Transcription Factor IL-4 STAT; STAT6B; STAT6C; and D12S1644. The sequence for the human *STAT6* gene can be found under publicly available GenBank Accession Number NC_000012.12:c57111413-57095404. The human *STAT6* gene is found at chromosome location 12q13.3 at 57111413-57095404, complement.

The sequence for the human *STAT6* pre-mRNA transcript (SEQ ID NO: 2) corresponds to the reverse complement of residues 57111413-57095404, complement, of chromosome 12q13.3. The *STAT6* mRNA sequence (GenBank Accession No. NM_001178078.1) is provided in SEQ ID NO: 4, except that the nucleotide "t" in SEQ ID NO: 4 is shown as "u" in the mRNA. The sequence for human STAT6 protein can be found under publicly available Accession Numbers: P42226-1, (canonical sequence, SEQ ID NO: 3), P42226-2 (SEQ ID NO: 5), and P42226-3 (SEQ ID NO: 5).

Natural variants of the human *STAT6* gene product are known. For example, natural variants of human STAT6 protein can contain one or more amino acid substitutions selected from: M118R, D419N, and any combination thereof. Additional variants of human STAT6 protein resulting from alternative splicing are also known in the art. STAT6 Isoform 2 (identifier: P42226-2 at UniProt) differs from the canonical sequence (SEQ ID NO: 4) as follows: deletion of residues 1-174 and substitution of ₁₇₅PSE₁₇₇ with ₁₇₅MEQ₁₇₇ relative to SEQ ID NO: 4. The sequence of STAT6 Isoform 3 (identifier: P42226-3) differs from the canonical sequence (SEQ ID NO: 4) as follows: deletion of residues 1-110 relative to SEQ ID NO: 4. Therefore, the payloads described herein (e.g., ASOs) can be designed to reduce or inhibit expression of the natural variants of the STAT6 protein.

An example of a target nucleic acid sequence of the payloads (*e.g.,* ASOs) is *STAT6* pre-mRNA. SEQ ID NO: 2 represents a human *STAT6* genomic sequence (*i.e.,* reverse complement of nucleotides 57111413-57095404, complement, of chromosome 12q13.3). SEQ ID NO: 2 is identical to a *STAT6* pre-mRNA sequence except that nucleotide "t" in SEQ ID NO: 2 is shown as "u" in pre-mRNA. In certain aspects, the "target nucleic acid" comprises an intron of a STAT6 protein-encoding nucleic acids or naturally occurring variants thereof, and RNA nucleic acids derived therefrom, *e.g*., pre-mRNA. In some aspects, the target nucleic acid comprises an exon region of a STAT6 protein-encoding nucleic acids or naturally occurring variants thereof, and RNA nucleic acids derived therefrom, *e.g*., pre-mRNA. In yet some aspects, the target nucleic acid comprises an exon-intron junction of a STAT6 protein-encoding nucleic acids or naturally occurring variants thereof, and RNA nucleic acids derived therefrom, *e.g*., pre-mRNA. In some aspects, for example when used in research or diagnostics the "target nucleic acid" can be a cDNA or a synthetic oligonucleotide derived from the above DNA or RNA nucleic acid targets. The human STAT6 protein sequence encoded by the *STAT6* pre-mRNA is shown as SEQ ID NO: 3. In some aspects, the target nucleic acid comprises an untranslated region of a STAT6 protein-encoding nucleic acids or naturally occurring variants thereof, *e.g.,* 5' UTR, 3' UTR, or both.

In some aspects, a payload of the disclosure (*e.g.,* ASO) hybridizes to a region within the introns of a *STAT6* transcript, *e.g.,* SEQ ID NO: 11. In certain aspects, the payload hybridizes to a region within the exons of a *STAT6* transcript, *e.g.,* SEQ ID NO: 11. In some aspects, the payload hybridizes to a region within the exon-intron junction of a *STAT6* transcript, *e.g.,* SEQ ID NO: 11. In some aspects, the payload hybridizes to a region within a *STAT6* transcript (*e.g.,* an intron, exon, or exon-intron junction), *e.g.,* SEQ ID NO: 11.

In some aspects, binding of an payload targeting a *STAT6* transcript disclosed herein to a mRNA transcript encoding *STAT6* can reduce expression levels and/or activity levels of *STAT6.*

In some aspects, the payload comprises an ASO that can specifically target a *STAT6* transcript. In some aspects, the nucleotide sequence of an ASO, or the contiguous nucleotide sequence thereof, has at least about 80% sequence identity to a sequence set forth in any one of SEQ ID NOs: 41 to 50 (*i.e.,* the sequences in FIG. 3A), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 41 to 50, or a region of at least 10 contiguous nucleotides thereof, wherein the ASO (or contiguous nucleotide portion thereof) can optionally comprise one, two, three, or four mismatches when compared to the corresponding *STAT6* transcript.

In some aspects, the ASO comprises the sequence set forth in SEQ ID NO: 41. In some aspects, the ASO comprises the sequence set forth in SEQ ID NO: 42. In some aspects, the ASO comprises the sequence set forth in SEQ ID NO: 43. In some aspects, the ASO comprises the sequence set forth in SEQ ID NO: 44. In some aspects, the ASO comprises the sequence set forth in SEQ ID NO: 45. In some aspects, the ASO comprises the sequence set forth in SEQ ID NO: 46. In some aspects, the ASO comprises the sequence set forth in SEQ ID NO: 47. In some aspects, the ASO comprises the sequence set forth in SEQ ID NO: 48. In some aspects, the ASO comprises the sequence set forth in SEQ ID NO: 49. In some aspects, the ASO comprises the sequence set forth in SEQ ID NO: 50.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 41 to 50. In some aspects, the ASO (or contiguous nucleotide portion thereof) is selected from, or comprises, one of the sequences selected from the group consisting of SEQ ID NOs: 41 to 50 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 41 to 50 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *STAT6* transcript. In some aspects, the ASO (or contiguous nucleotide portion thereof) is selected from, or comprises, one of the sequences selected from the group consisting of SEQ ID NOs: 41 to 50 except for 1, 2, 3, 4, 5, 6, 7, 8, 9 or 10 substitutions, wherein the substituted ASO can bind to the *STAT6* transcript. In some aspects, the ASO (or contiguous nucleotide portion thereof) is selected from, or comprises, one of the sequences selected from the group consisting of SEQ ID NOs: 41 to 50 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *STAT6* transcript.

### II.G.1.b KRAS

In some aspects, the payloads (*e.g.,* ASOs) provided herein are capable of down-regulating (*e.g.,* reducing or inhibiting) the expression of a *KRAS* mRNA or KRAS protein, *e.g.,* in a cell. In certain aspects, the payloads described herein (*e.g.,* can be loaded in EVs using the loading methods of the present disclosure) target one or more regions of the *KRAS* pre-mRNA *(e.g.,* intron regions, exon regions, and/or exon-intron junction regions). In some aspects, the payload is capable of targeting all isoforms of the KRAS protein. In certain aspects, the payload targets specific KRAS isoforms. Unless indicated otherwise, the term " KRAS," as used herein, can refer to KRAS from one or more species (*e.g.,* humans, non-human primates, dogs, cats, guinea pigs, rabbits, rats, mice, horses, cattle, and bears).

KRAS is known in the art by various names. Such names include: KRAS Proto-Oncogene, GTPase; V-Ki-Ras2 Kirsten Rat Sarcoma 2 Viral Oncogene Homolog; GTPase KRas; C-Ki-Ras; K-Ras 2; KRAS2; RASK2; V-Ki-Ras2 Kirsten Rat Sarcoma Viral Oncogene Homolog; Kirsten Rat Sarcoma Viral Proto-Oncogene; Cellular Transforming Proto-Oncogene; Cellular C-Ki-Ras2 Proto-Oncogene; Transforming Protein P21; PR310 C-K-Ras Oncogene; C-Kirsten-Ras Protein; K-Ras P21 Protein; and Oncogene KRAS2.

The sequence for the human *KRAS* gene can be found at chromosomal location 12p12.1 and under publicly available GenBank Accession Number NC_000012 (25,204,789 - 25,250,936). The genomic sequence for human wild-type *KRAS* transcript corresponds to the reverse complement of residues 25,204,789 - 25,250,936 of NC_000012 (SEQ ID NO: 11). The *KRAS G12D* genomic sequence provided in SEQ ID NO: 7 differs from SEQ ID NO: 11 in that it has a guanine to adenine substitution at nucleotide position 5,587. An exemplary *KRAS G12D* mRNA sequence is provided in SEQ ID NO: 9, except that the nucleotide "t" in SEQ ID NO: 33 is shown as "u" in the mRNA. The *KRAS G12D* mRNA provided in SEQ ID NO: 9 differs from the wild-type mRNA sequence (*e.g.,* GenBank Accession No. NM_004985.5; SEQ ID NO: 13) in that it has a guanine to adenine substitution at nucleotide position 225. The sequence for human KRAS protein can be found under publicly available Accession Numbers: P01116 (canonical sequence), A8K8Z5, B0LPF9, P01118, and Q96D10.

There are two isoforms of the human KRAS protein (P01116), resulting from alternative splicing. Isoform 2A (Accession Number: P01116-1; SEQ ID NO: 14) is the canonical sequence. It is also known as K-Ras4A. Isoform 2B (Accession Number: P01116-2; also known as K-Ras4B; SEQ ID NO: 12) differs from the canonical sequence as follows: (i) 151-153: RVE → GVD; and (ii) 165-189: QYRLKKISKEEKTPGCVKIKKCIIM (SEQ ID NO: 15) → KHKEKMSKDGKKKKKKSKTKCVIM (SEQ ID NO: 16). In some aspects, payloads disclosed herein can reduce or inhibit expression of KRAS protein Isoform 2A, Isoform 2B, or both.

Natural variants of the human *KRAS* gene product are known. For example, natural variants of human KRAS protein can contain one or more amino acid substitutions selected from: K5E, K5N, G10GG, G10V, G12A, G12C, G12F, G12I, G12L, G12R, G12S, G12V, G13C, G13D, G13E, G13R, G13V, V14I, L19F, T20M, Q22E, Q22H, Q22K, Q22R, Q25H, N26Y, F28L, E31K, D33E, P34L, P34Q, P34R, I36M, R41K, D57N, T58I, A59T, G60D, G60R, G60S, G60V, Q61A, Q61H, Q61K, Q61L, Q61P, Q61R, E63K, S65N, R68S, Y71H, T74A, L79I, R97I, Q99E, M111L, K117N, K117R, D119G, S122F, T144P, A146P, A146T, A146V, K147E, K147T, R149K, L159S, I163S, R164Q, I183N, I84M, or combinations thereof. Natural variants that are specific to KRAS protein Isoform 2B contain one or more amino acid substitutions selected from: V152G, D153V, F156I, F156L, or combinations thereof. The payloads of the present disclosure can be designed to reduce or inhibit expression of one or more of the variants of the KRAS protein (*e.g.,* any variants known in the art). In some aspects, a KRAS mutant has an amino acid substitution of G12D. In some aspects, the payloads of the present disclosure target one or more KRAS mutants. In certain aspects, a KRAS mutant that the payloads target is KRAS G12D (SEQ ID NO: 8). Exemplary sequences for *KRAS G12D* mRNA and KRAS G12D protein are provided in SEQ ID NO: 9 and SEQ ID NO: 8, respectively.

In some aspects, a target nucleic acid sequence of a payload *(e.g.,* ASO) disclosed herein comprises one or more regions of a *KRAS* pre-mRNA. For example, SEQ ID NO: 7 (described above) is identical to a *KRAS* pre-mRNA sequence except that nucleotide "t" in SEQ ID NO: 7 is shown as "u" in the pre-mRNA. As used herein, the term "target nucleic acid sequence" refers to a nucleic acid sequence that is complementary to a payload disclosed herein. In certain aspects, the target nucleic acid sequence comprises an exon region of a KRAS protein-encoding nucleic acids or naturally occurring variants thereof, and RNA nucleic acids derived therefrom, *e.g*., pre-mRNA. In some aspects, the target nucleic acid sequence comprises an intron of a KRAS protein-encoding nucleic acids or naturally occurring variants thereof, and RNA nucleic acids derived therefrom, *e.g*., pre-mRNA. In further aspects, the target nucleic acid sequence comprises an exon-intron junction of a KRAS protein-encoding nucleic acids or naturally occurring variants thereof, and RNA nucleic acids derived therefrom, *e.g*., pre-mRNA. In some aspects, for example, when used in research or diagnostics, the target nucleic acid can be a cDNA or a synthetic oligonucleotide derived from DNA or RNA nucleic acid targets described herein. In some aspects, the target nucleic acid comprises an untranslated region of a KRAS protein-encoding nucleic acids or naturally occurring variants thereof, *e.g.,* 5' UTR, 3' UTR, or both.

Accordingly, in some aspects, a payload (*e.g.,* ASO) disclosed herein hybridizes to an exon region of a *KRAS* transcript, *e.g.,* SEQ ID NO: 7 or SEQ ID NO: 9. In some aspects, a payload of the present disclosure hybridizes to an intron region of a *KRAS* transcript, *e.g.,* SEQ ID NO: 7. In some aspects, a payload hybridizes to an exon-intron junction of a *KRAS* transcript, *e.g.,* SEQ ID NO: 7. In some aspects, a payload of the present disclosure hybridizes to a region within a *KRAS* transcript (*e.g.,* an intron, exon, or exon-intron junction), *e.g.,* SEQ ID NO: 7, wherein the payload has a design described elsewhere herein.

In some aspects, a target nucleic sequence of the payloads disclosed herein is a *KRAS* mRNA, *e.g.,* SEQ ID NO: 9. Accordingly, in certain aspects, a payload disclosed herein can hybridize to one or more regions of a *KRAS* mRNA. In some aspects, payloads of the present disclosure target mRNA encoding a particular isoform of KRAS protein. In certain aspects, payloads disclosed herein can target all isoforms of KRAS protein, including any variants thereof (*e.g.,* those described herein). In some aspects, a KRAS protein that can be targeted by payloads of the present disclosure comprises a G12D amino acid substitution.

In some aspects, the payload comprises an ASO that can specifically target a *KRAS* transcript. In some aspects, the nucleotide sequence of an ASO, or the contiguous nucleotide sequence thereof, has at least about 80% sequence identity to a sequence set forth in any one of SEQ ID NOs: 51 to 61 (*i.e.,* the sequences in FIG. 3B), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 51 to 61, or a region of at least 10 contiguous nucleotides thereof, wherein the ASO (or contiguous nucleotide portion thereof) can optionally comprise one, two, three, or four mismatches when compared to the corresponding *KRAS* transcript.

In some aspects, the ASO comprises the sequence set forth in SEQ ID NO: 51. In some aspects, the ASO comprises the sequence set forth in SEQ ID NO: 52. In some aspects, the ASO comprises the sequence set forth in SEQ ID NO: 53. In some aspects, the ASO comprises the sequence set forth in SEQ ID NO: 54. In some aspects, the ASO comprises the sequence set forth in SEQ ID NO: 55. In some aspects, the ASO comprises the sequence set forth in SEQ ID NO: 56. In some aspects, the ASO comprises the sequence set forth in SEQ ID NO: 57. In some aspects, the ASO comprises the sequence set forth in SEQ ID NO: 58. In some aspects, the ASO comprises the sequence set forth in SEQ ID NO: 59. In some aspects, the ASO comprises the sequence set forth in SEQ ID NO: 60. In some aspects, the ASO comprises the sequence set forth in SEQ ID NO: 610.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 51 to 61. In some aspects, the ASO (or contiguous nucleotide portion thereof) is selected from, or comprises, one of the sequences selected from the group consisting of SEQ ID NOs: 51 to 61 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 51 to 61 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *KRAS* transcript. In some aspects, the ASO (or contiguous nucleotide portion thereof) is selected from, or comprises, one of the sequences selected from the group consisting of SEQ ID NOs: 51 to 61 except for 1, 2, 3, 4, 5, 6, 7, 8, 9 or 10 substitutions, wherein the substituted ASO can bind to the *KRAS* transcript. In some aspects, the ASO (or contiguous nucleotide portion thereof) is selected from, or comprises, one of the sequences selected from the group consisting of SEQ ID NOs: 51 to 61 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *KRAS* transcript.

### II.G.1.c NRAS

In some aspects, the payloads (*e.g*., ASOs) provided herein are capable of down-regulating (*e.g.,* reducing or inhibiting) the expression of a *NRas* mRNA or NRas protein, *e.g.,* in a cell. In certain aspects, the payloads described herein (*e.g.,* can be loaded in EVs using the loading methods of the present disclosure) target one or more regions of the *NRas* pre-mRNA (*e.g.,* intron regions, exon regions, and/or exon-intron junction regions). In some aspects, the payload is capable of targeting all isoforms of the NRas protein. In certain aspects, the payload targets specific NRas isoforms. Unless indicated otherwise, the term " NRas," as used herein, can refer to NRas from one or more species (*e.g.,* humans, non-human primates, dogs, cats, guinea pigs, rabbits, rats, mice, horses, cattle, and bears).

*NRas* is an oncogene encoding a membrane protein that shuttles between the Golgi apparatus and the plasma membrane. *NRa*s-encoding genomic DNA can be found at Chromosomal position 1p13.2 *(i.e.,* nucleotides 5001 to 17438 of GenBank Accession No. NG_007572). Specifically, a combination of time-lapse microscopy and photobleaching techniques have revealed that in the absence of palmitoylation, GFP-tagged N-Ras undergoes rapid exchange between the cytosol and ER/Golgi membranes, and that wild-type GFP-N-Ras is recycled to the Golgi complex by a nonvesicular mechanism. N-ras mutations have been described in melanoma, thyroid carcinoma, teratocarcinoma, fibrosarcoma, neuroblastoma, rhabdomyosarcoma, Burkitt lymphoma, acute promyelocytic leukemia, T cell leukemia, and chronic myelogenous leukemia. Oncogenic N-Ras can induce acute myeloid leukemia (AML)- or chronic myelomonocytic leukemia (CMML)-like disease in mice.

Neuroblastoma RAS viral oncogene (*NRas)* is known in the art by various names. Such names include: GTPase NRas, N-ras protein part 4, neuroblastoma RAS viral (v-ras) oncogene homolog neuroblastoma RAS viral oncogene homolog, transforming protein N-Ras, and v-ras neuroblastoma RAS viral oncogene homolog.

The NRAS gene provides instructions for making a protein called N-Ras that is involved primarily in regulating cell division. The mRNA sequence encoding human NRAS can be found at NCBI Reference sequence NM_002524.5 and is represented by the coding sequence (SEQ ID NO: 19).

Natural variants of the human *NRas* gene product are known. For example, natural variants of human NRas protein can contain one or more amino acid substitutions selected from: G12D, G13D, T50I, G60E, and any combinations thereof. Additional variants of human NRas protein resulting from alternative splicing are also known in the art, such as: G13R, Q61K, Q61R, and P34L. Therefore, the payloads of the present disclosure can be designed to reduce or inhibit expression of the natural variants of the NRas protein.

SEQ ID NO: 17 is identical to a *NRas* pre-mRNA sequence except that nucleotide "t" in SEQ ID NO: 17 is shown as "u" in pre-mRNA. In certain aspects, the "target nucleic acid" comprises an intron of a NRas protein-encoding nucleic acids or naturally occurring variants thereof, and RNA nucleic acids derived therefrom, *e.g*., pre-mRNA. In certain aspects, the target nucleic acid comprises an exon region of a NRas protein-encoding nucleic acids or naturally occurring variants thereof, and RNA nucleic acids derived therefrom, *e.g*., pre-mRNA. In certain aspects, the target nucleic acid comprises an exon-intron junction of a NRas protein-encoding nucleic acids or naturally occurring variants thereof, and RNA nucleic acids derived therefrom, *e.g*., pre-mRNA. In some aspects, for example when used in research or diagnostics the "target nucleic acid" can be a cDNA or a synthetic oligonucleotide derived from the above DNA or RNA nucleic acid targets. The human NRas protein sequence encoded by the *NRas* pre-mRNA is shown as SEQ ID NO: 18. In certain aspects, the target nucleic acid comprises an untranslated region of a NRas protein-encoding nucleic acids or naturally occurring variants thereof, *e.g.,* 5' UTR, 3' UTR, or both.

In certain aspects, the payloads of the disclosure also are capable of down-regulating *(e.g.,* reducing or removing) expression of the *NRas* mRNA or protein. In this regard, the payload of the disclosure can affect indirect inhibition of NRas protein through the reduction in *NRas* mRNA levels, typically in a mammalian cell, such as a human cell, such as a tumor cell. In particular, the present disclosure is directed to payloads that target one or more regions of the *NRas* pre-mRNA (*e.g.,* intron regions, exon regions, and/or exon-intron junction regions). Unless indicated otherwise, the term "NRas," as used herein, can refer to NRas from one or more species (*e.g.,* humans, non-human primates, dogs, cats, guinea pigs, rabbits, rats, mice, horses, cattle, and bears).

In some aspects, a payload of the disclosure hybridizes to a region within the introns of a *NRAS* transcript, *e.g.,* SEQ ID NO: 17 or SEQ ID NO: 19. In certain aspects, a payload of the disclosure hybridizes to a region within the exons of a *NRAS* transcript, *e.g.,* SEQ ID NO: 17 or SEQ ID NO: 19. In certain aspects, a payload of the disclosure hybridizes to a region within the exon-intron junction of a *NRAS* transcript, *e.g.,* SEQ ID NO: 17 or SEQ ID NO: 19. In some aspects, a payload of the disclosure hybridizes to a region within a *NRAS* transcript (*e.g.,* an intron, exon, or exon-intron junction), *e.g.,* SEQ ID NO: 17 or SEQ ID NO: 19.

In some aspects, the payload of the present disclosure hybridizes to multiple target regions within the *NRas* transcript (*e.g.,* genomic sequence, SEQ ID NO: 17). In some aspects, the payload hybridizes to two different target regions within the *NRas* transcript. In some aspects, the payload hybridizes to three different target regions within the *NRas* transcript. In some aspects, the payloads that hybridizes to multiple regions within the *NRas* transcript (*e.g.,* genomic sequence, SEQ ID NO: 17) are more potent (*e.g.,* having lower EC50) at reducing *NRas* expression compared to payloads that hybridizes to a single region within the *NRas* transcript *(e.g.,* genomic sequence, SEQ ID NO: 17).

In some aspects, the payload *(e.g.,* ASO) targets a mRNA encoding a particular isoform of NRAS protein (*e.g.,* Isoform 1, NCBI ID: NP_001229821.1). In some aspects, the payload targets all isoforms of NRas protein. In certain aspects, the payload targets two isoforms (*e.g.,* Isoform 1 and Isoform 2 (NCBI ID:NP_009089.4), Isoform 2 and Isoform 3(NCBI ID: NP_001123995), and Isoform 3 and Isoform 4(NCBI ID: NP_001229820.1)) of NRas protein.

The payloads of the disclosure comprise a contiguous nucleotide sequence which corresponds to the complement of a region of *NRas* transcript, *e.g.,* a nucleotide sequence corresponding to SEQ ID NO: 17.

In some aspects, binding of a payload (*e.g.,* ASO) targeting a *NRas* transcript disclosed herein to a mRNA transcript encoding *NRas* can reduce expression levels and/or activity levels of *NRas.*

In some aspects, the payload comprises an ASO that can specifically target a *NRas* transcript. In some aspects, the nucleotide sequence of an ASO, or the contiguous nucleotide sequence thereof, has at least about 80% sequence identity to a sequence set forth in any one of SEQ ID NOs: 62 to 71 (*i.e.,* the sequences in FIG. 3C), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 62 to 71, or a region of at least 10 contiguous nucleotides thereof, wherein the ASO (or contiguous nucleotide portion thereof) can optionally comprise one, two, three, or four mismatches when compared to the corresponding *NRas* transcript.

In some aspects, the ASO comprises the sequence set forth in SEQ ID NO: 62. In some aspects, the ASO comprises the sequence set forth in SEQ ID NO: 63. In some aspects, the ASO comprises the sequence set forth in SEQ ID NO: 64. In some aspects, the ASO comprises the sequence set forth in SEQ ID NO: 65. In some aspects, the ASO comprises the sequence set forth in SEQ ID NO: 66. In some aspects, the ASO comprises the sequence set forth in SEQ ID NO: 67. In some aspects, the ASO comprises the sequence set forth in SEQ ID NO: 68. In some aspects, the ASO comprises the sequence set forth in SEQ ID NO: 69. In some aspects, the ASO comprises the sequence set forth in SEQ ID NO: 70. n some aspects, the ASO comprises the sequence set forth in SEQ ID NO: 71.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 62 to 71. In some aspects, the ASO (or contiguous nucleotide portion thereof) is selected from, or comprises, one of the sequences selected from the group consisting of SEQ ID NOs: 62 to 71 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 62 to 71 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *NRas* transcript. In some aspects, the ASO (or contiguous nucleotide portion thereof) is selected from, or comprises, one of the sequences selected from the group consisting of SEQ ID NOs: 62 to 71 except for 1, 2, 3, 4, 5, 6, 7, 8, 9 or 10 substitutions, wherein the substituted ASO can bind to the *NRas* transcript. In some aspects, the ASO (or contiguous nucleotide portion thereof) is selected from, or comprises, one of the sequences selected from the group consisting of SEQ ID NOs: 62 to 71 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *NRas* transcript.

### II.G.1.d PMP22

In some aspects, the payloads (*e.g.,* ASOs) provided herein are capable of down-regulating *(e.g.,* reducing or inhibiting) the expression of a *PMP22* mRNA or PMP22 protein, *e.g.,* in a cell. In certain aspects, the payloads described herein *(e.g.,* can be loaded in EVs using the loading methods of the present disclosure) target one or more regions of the *PMP22* pre-mRNA *(e.g.,* intron regions, exon regions, and/or exon-intron junction regions). In some aspects, the payload is capable of targeting all isoforms of the PMP22 protein. In certain aspects, the payload targets specific PMP22 isoforms. Unless indicated otherwise, the term " PMP22," as used herein, can refer to STAT6 from one or more species (*e.g.,* humans, non-human primates, dogs, cats, guinea pigs, rabbits, rats, mice, horses, cattle, and bears).

Peripheral myelin protein 22 (PMP22) is also known as growth arrest-specific protein 3 (GAS-3), is encoded by the PMP22 gene. PMP22 is a 22 kDa transmembrane glycoprotein made up of 160 amino acids, and is mainly expressed in the Schwann cells of the peripheral nervous system. Schwann cells show high expression of PMP22, where it can constitute 2-5% of total protein content in compact myelin. Compact myelin is the bulk of the peripheral neuron's myelin sheath, a protective fatty layer that provides electrical insulation for the neuronal axon. The level of PMP22 expression is relatively low in the central nervous system of adults.

In humans, the PMP22 gene is located on chromosome 17p11.2 and spans approximately 40kb. The gene contains six exons conserved in both humans and rodents, two of which are 5' untranslated exons (1a and 1b) and result in two different RNA transcripts with identical coding sequences. The two transcripts differ in their 5' untranslated regions and have their own promoter regulating expression. The remaining exons (2 to 5) include the coding region of the *PMP22* gene, and are joined together after post-transcriptional modification (i.e. alternative splicing). The PMP22 protein is characterized by four transmembrane domains, two extracellular loops (ECL1 and ECL2), and one intracellular loop. ECL1 has been suggested to mediate a homophilic interaction between two PMP22 proteins, whereas ECL2 has been shown to mediate a heterophilic interaction between PMP22 protein and Myelin protein zero (MPZ or MP0).

PMP22 plays an essential role in the formation and maintenance of compact myelin. When Schwann cells come into contact with a neuronal axon, expression of PMP22 is significantly up-regulated, whereas PMP22 is down-regulated during axonal degeneration or transection. PMP22 has shown association with zonula-occludens 1 and occludin, proteins that are involved in adhesion with other cells and the extracellular matrix, and also support functioning of myelin. Along with cell adhesion function, PMP22 is also up-regulated during Schwann cell proliferation, suggesting a role in cell-cycle regulation. PMP22 is detectable in non-neural tissues, where its expression has been shown to serve as growth-arrest-specific (gas-3) function.

Improper gene dosage of the PMP22 gene can cause aberrant protein synthesis and function of myelin sheath. Since the components of myelin are stoichiometrically set, any irregular expression of a component can cause destabilization of myelin and neuropathic disorders. Alterations of PMP22 gene expression are associated with a variety of neuropathies, such as Charcot-Marie-Tooth type 1A (CMT1A), Dejerine-Sottas disease, and Hereditary Neuropathy with Liability to Pressure Palsy (HNPP). Too much PMP22 (e.g. caused by gene duplication) results in CMT1A. Gene duplication of PMP22 is the most common genetic cause of CMT where the overproduction of PMP22 results in defects in multiple signaling pathways and dysfunction of transcriptional factors like KNOX20, SOX10 and EGR2.

The sequence for the human *PMP22* gene can be found under publicly available as NCBI RefSeq Acc. No. NM_000304. Alternative RefSeq mRNA transcripts have accession numbers NM_001281455, NM-001281456, NM-153321, and NM_153322, respectively. The human *PMP22* gene is found at chromosome location 17p12 at 15,229,777-15,265,326.

The sequence for the human *PMP22* pre-mRNA transcript (SEQ ID NO: 20) corresponds to the reverse complement of residues 15,229,777-15,265,326, of chromosome location 17p12. The *PMP22* mRNA sequence (GenBank Accession No. NM_000304.4) is provided in SEQ ID NO: 21. The sequence for human PMP22 protein can be found under publicly available Uniprot Accession Number Q01453 (canonical sequence, SEQ ID NO: 23). Potential PMP22 isoforms have Uniprot Accession Numbers A8MU75, J3KQW0, A0A2R8Y5L5, J3KT36, and J3QS08, respectively. The publicly available contents of the database entries corresponding to accession numbers disclosed herein.

Natural variants of the human PMP22 gene product are known. For example, natural variants of human PMP22 protein can contain one or more amino acid substitutions selected from L16P, S22F, Δ25-26, D37V, V65F, S72L, S79C, G93R, L105R, G107V, T118N, L147R, H12Q, L16P, L19P, M69K, L71P, S72L, S72P, S72W, S76I, S79P, L80P, L80R, Δ84, G100E, G100R, L105R, C109R, S149R, G150C, G150D, R157W, S22F, V30M, A67T, S23T, W28R, A67P, Δ115-118, and any combination thereof.

The payloads of the present disclosure can be designed to reduce or inhibit expression of the natural variants of the PMP22 protein.

An example of a target nucleic acid sequence of the payloads is *PMP22* pre-mRNA. SEQ ID NO: 20 represents a human *PMP22* genomic sequence *(i.e.,* reverse complement of nucleotides 15,229,777-15,265,326, complement, of chromosome 17p12). SEQ ID NO: 20 is identical to a *PMP22* pre-mRNA sequence except that nucleotide "t" in SEQ ID NO: 20 is shown as "u" in pre-mRNA.

In some aspects, the contiguous nucleotide sequence is at least about 80%, at least about 85%, at least about 90%, at least about 95%, or about 100% complementary to the nucleic acid sequence within the *PMP22* transcript. In some aspects, the payload is capable of reducing PMP22 protein expression in a human cell (e.g., a Schwan cell), wherein the human cell expresses the PMP22 protein.

In some aspects, a payload (*e.g.,* ASO) of the disclosure hybridizes to a region within the introns of a *PMP22* transcript, *e.g.,* SEQ ID NO: 20. In certain aspects, a payload of the disclosure hybridizes to a region within the exons of a *PMP22* transcript, *e.g.,* SEQ ID NO: 20. In certain aspects, a payload of the disclosure hybridizes to a region within the exon-intron junction of a *PMP22* transcript, *e.g.,* SEQ ID NO: 20.

In some aspects, the payload of the present disclosure hybridizes to multiple target regions within the *PMP22* transcript (*e.g.,* genomic sequence, SEQ ID NO: 20). In some aspects, the payload hybridizes to two different target regions within the *PMP22* transcript. In some aspects, the payload hybridizes to three different target regions within the *PMP22* transcript. In some aspects, the payloads that hybridizes to multiple regions within the *PMP22* transcript (*e.g.,* genomic sequence, SEQ ID NO: 20) are more potent (*e.g.,* having lower EC50) at reducing *PMP22* expression compared to payloads that hybridizes to a single region within the *PMP22* transcript (e.g., genomic sequence, SEQ ID NO: 20).

In some aspects, binding of a payload (*e.g.,* ASO) targeting a *PMP22* transcript disclosed herein to a mRNA transcript encoding *PMP22* can reduce expression levels and/or activity levels of *PMP22.*

In some aspects, the payload comprises an ASO that can specifically target a *PMP22* transcript. In some aspects, the nucleotide sequence of an ASO, or the contiguous nucleotide sequence thereof, has at least about 80% sequence identity to a sequence set forth in any one of SEQ ID NOs: 72 to 81 (*i.e.,* the sequences in FIG. 3D), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 72 to 81, or a region of at least 10 contiguous nucleotides thereof, wherein the ASO (or contiguous nucleotide portion thereof) can optionally comprise one, two, three, or four mismatches when compared to the corresponding *PMP22* transcript.

In some aspects, the ASO comprises the sequence set forth in SEQ ID NO: 72. In some aspects, the ASO comprises the sequence set forth in SEQ ID NO: 73. In some aspects, the ASO comprises the sequence set forth in SEQ ID NO: 74. In some aspects, the ASO comprises the sequence set forth in SEQ ID NO: 75. In some aspects, the ASO comprises the sequence set forth in SEQ ID NO: 76. In some aspects, the ASO comprises the sequence set forth in SEQ ID NO: 77. In some aspects, the ASO comprises the sequence set forth in SEQ ID NO: 78. In some aspects, the ASO comprises the sequence set forth in SEQ ID NO: 79. In some aspects, the ASO comprises the sequence set forth in SEQ ID NO: 80. In some aspects, the ASO comprises the sequence set forth in SEQ ID NO: 81.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 72 to 81. In some aspects, the ASO (or contiguous nucleotide portion thereof) is selected from, or comprises, one of the sequences selected from the group consisting of SEQ ID NOs: 72 to 81 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 72 to 81 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *PMP22* transcript. In some aspects, the ASO (or contiguous nucleotide portion thereof) is selected from, or comprises, one of the sequences selected from the group consisting of SEQ ID NOs: 72 to 81 except for 1, 2, 3, 4, 5, 6, 7, 8, 9 or 10 substitutions, wherein the substituted ASO can bind to the *PMP22* transcript. In some aspects, the ASO (or contiguous nucleotide portion thereof) is selected from, or comprises, one of the sequences selected from the group consisting of SEQ ID NOs: 72 to 81 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *PMP22* transcript.

### II.G.1.e C/EBPβ

In some aspects, the payloads (*e.g.,* ASOs) provided herein are capable of down-regulating *(e.g.,* reducing or inhibiting) the expression of a *C*/*EBPβ* mRNA or CEBP/β protein, *e.g.,* in a cell. In certain aspects, the payloads described herein *(e.g.,* can be loaded in EVs using the loading methods of the present disclosure) target one or more regions of the *CEBP*/*β* pre-mRNA (*e.g.,* intron regions, exon regions, and/or exon-intron junction regions). In some aspects, the payload is capable of targeting all isoforms of the CEBP/β protein. In certain aspects, the payload targets specific CEBP/β isoforms. Unless indicated otherwise, the term " CEBP/β," as used herein, can refer to CEBP/β from one or more species (*e.g.,* humans, non-human primates, dogs, cats, guinea pigs, rabbits, rats, mice, horses, cattle, and bears).

CEBP/β (*CEBP*/*β*) is also known as CCAAT/enhancer-binding protein beta. Synonyms of CEBP/β/*CEBP*/*β* are known and include C/EBP beta; Liver activator protein; LAP; Liver-enriched inhibitory protein; LIP; Nuclear factor NF-IL6; transcription factor 5; TCF-5; *CEBPB; CEBPb; CEBPβ*; *CEBP*/*B*; and *TCF5.* The sequence for the human *CEBP*/*β* gene can be found under publicly available GenBank Accession Number NC_000020.11 (50190583..50192690). The human *CEBP*/*β* gene is found at chromosome location 20q13.13 at 50190583-50192690.

The sequence for the human *CEBP*/*β* pre-mRNA transcript (SEQ ID NO: 251) corresponds to the reverse complement of residues 50190583-50192690 of chromosome 20q13.13. The *CEBP*/*β* mRNA sequence (GenBank Accession No. NM_001285878.1) is provided in SEQ ID NO: 27, except that the nucleotide "t" in SEQ ID NO: 27 is shown as "u" in the mRNA. The sequence for human CEBP/β protein can be found under publicly available Accession Numbers: P17676, (canonical sequence, SEQ ID NO: 26), P17676-2 (SEQ ID NO: 28), and P17676-3 (SEQ ID NO: 29).

Natural variants of the human *CEBP*/*β* gene product are known. For example, natural variants of human CEBP/β protein can contain one or more amino acid substitutions selected from: A241P, A253G, G195S, and any combination thereof. Additional variants of human CEBP/β protein resulting from alternative splicing are also known in the art. CEBP/β Isoform 2 (identifier: P17676-2 at UniProt) differs from the canonical sequence (SEQ ID NO: 27) as follows: deletion of residues 1-23 relative to SEQ ID NO: 27. The sequence of CEBP/β Isoform 3 (identifier: P17676-3) differs from the canonical sequence (SEQ ID NO: 23) as follows: deletion of residues 1-198 relative to SEQ ID NO: 27. Therefore, the payload described herein (*e.g.,* ASO) can be designed to reduce or inhibit expression of the natural variants of the protein.

An example of a target nucleic acid sequence of the payloads is *CEBP*/*β* pre-mRNA. SEQ ID NO: 25 represents a human *CEBP*/*β* genomic sequence (*i.e.,* reverse complement of nucleotides 50190583-50192690 of chromosome 20q13.13). SEQ ID NO: 25 is identical to a *CEBP*/*β* pre-mRNA sequence except that nucleotide "t" in SEQ ID NO: 25 is shown as "u" in pre-mRNA. In certain aspects, the "target nucleic acid" comprises an intron of a CEBP/β protein-encoding nucleic acids or naturally occurring variants thereof, and RNA nucleic acids derived therefrom, *e.g*., pre-mRNA. In certain aspects, the target nucleic acid comprises an exon region of a CEBP/β protein-encoding nucleic acids or naturally occurring variants thereof, and RNA nucleic acids derived therefrom, *e.g*., pre-mRNA. In certain aspects, the target nucleic acid comprises an exon-intron junction of a CEBP/β protein-encoding nucleic acids or naturally occurring variants thereof, and RNA nucleic acids derived therefrom, *e.g*., pre-mRNA. In some aspects, for example when used in research or diagnostics the "target nucleic acid" can be a cDNA or a synthetic oligonucleotide derived from the above DNA or RNA nucleic acid targets. The human CEBP/β protein sequence encoded by the *CEBP*/*β* pre-mRNA is shown as SEQ ID NO: 26. In certain aspects, the target nucleic acid comprises an untranslated region of a CEBP/β protein-encoding nucleic acids or naturally occurring variants thereof, *e.g.,* 5' UTR, 3' UTR, or both.

In some aspects, a payload (*e.g.,* ASO) of the disclosure hybridizes to a region within the introns of a *CEBP*/*β* transcript, *e.g.,* SEQ ID NO: 25. In certain aspects, a payload of the disclosure hybridizes to a region within the exons of a *CEBP*/*β* transcript, *e.g.,* SEQ ID NO: 251. In certain aspects, an ASO of the disclosure hybridizes to a region within the exon-intron junction of a *CEBP*/*β* transcript, *e.g.,* SEQ ID NO: 25. In some aspects, a payload of the disclosure hybridizes to a region within a *CEBP*/*β* transcript (*e.g.,* an intron, exon, or exon-intron junction), *e.g.,* SEQ ID NO: 25, wherein the payload has a design according to formula: 5' A-B-C 3' as described elsewhere herein.

In some aspects, the payload targets a mRNA encoding a particular isoform of CEBP/β protein (*e.g.,* Isoform 1). In some aspects, the payload targets all isoforms of CEBP/β protein. In certain aspects, the payload targets two isoforms (*e.g.,* Isoform 1 and Isoform 2, Isoform 1 and Isoform 3, or Isoform 2 and Isoform 3) of CEBP/β protein.

In some aspects, binding of a payload (*e.g.,* ASO) targeting a *CEBPb* transcript disclosed herein to a mRNA transcript encoding *CEBPb* can reduce expression levels and/or activity levels of *CEBPb.*

In some aspects, the payload comprises an ASO that can specifically target a *CEBPb* transcript. In some aspects, the nucleotide sequence of an ASO, or the contiguous nucleotide sequence thereof, has at least about 80% sequence identity to a sequence set forth in any one of SEQ ID NOs: 82 to 91 (*i.e.,* the sequences in FIG. 3E), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 82 to 91, or a region of at least 10 contiguous nucleotides thereof, wherein the ASO (or contiguous nucleotide portion thereof) can optionally comprise one, two, three, or four mismatches when compared to the corresponding *CEBPb* transcript.

In some aspects, the ASO comprises the sequence set forth in SEQ ID NO: 82. In some aspects, the ASO comprises the sequence set forth in SEQ ID NO: 83. In some aspects, the ASO comprises the sequence set forth in SEQ ID NO: 84. In some aspects, the ASO comprises the sequence set forth in SEQ ID NO: 85. In some aspects, the ASO comprises the sequence set forth in SEQ ID NO: 86. In some aspects, the ASO comprises the sequence set forth in SEQ ID NO: 87. In some aspects, the ASO comprises the sequence set forth in SEQ ID NO: 88. In some aspects, the ASO comprises the sequence set forth in SEQ ID NO: 89. In some aspects, the ASO comprises the sequence set forth in SEQ ID NO: 90. In some aspects, the ASO comprises the sequence set forth in SEQ ID NO: 91.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 82 to 91. In some aspects, the ASO (or contiguous nucleotide portion thereof) is selected from, or comprises, one of the sequences selected from the group consisting of SEQ ID NOs: 82 to 91 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 82 to 91 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *CEBPb* transcript. In some aspects, the ASO (or contiguous nucleotide portion thereof) is selected from, or comprises, one of the sequences selected from the group consisting of SEQ ID NOs: 82 to 91 except for 1, 2, 3, 4, 5, 6, 7, 8, 9 or 10 substitutions, wherein the substituted ASO can bind to the *CEBPb* transcript. In some aspects, the ASO (or contiguous nucleotide portion thereof) is selected from, or comprises, one of the sequences selected from the group consisting of SEQ ID NOs: 82 to 91 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *CEBPb* transcript.

### II.G.1.f STAT3

In some aspects, the payloads (*e.g.,* ASOs) provided herein are capable of down-regulating (*e.g.,* reducing or inhibiting) the expression of any gene or protein of interest in a cell. In certain aspects, the payloads described herein (*e.g.,* can be loaded in EVs using the loading methods of the present disclosure) target one or more regions of the *STAT3* pre-mRNA (*e.g.,* intron regions, exon regions, and/or exon-intron junction regions). In some aspects, the payload is capable of targeting all isoforms of the STAT3 protein. In certain aspects, the payload targets specific STAT3 isoforms. Unless indicated otherwise, the term "STAT3," as used herein, can refer to STAT3 from one or more species (*e.g.,* humans, non-human primates, dogs, cats, guinea pigs, rabbits, rats, mice, horses, cattle, and bears).

Signal transducer and activator of transcription 3 (STAT3) is known in the art by various names. Such names include: DNA-binding protein APRF, and acute-phase response factor. The mRNA encoding human STAT3 can be found at Genbank Accession Number NM_003150.3, and is represented by the sequence (SEQ ID NO: 32).

Natural variants of the human *STAT3* gene product are known. For example, natural variants of human STAT3 protein can contain one or more amino acid substitutions selected from: R382L, R382Q, OR R382W, and any combinations thereof. Additional variants of human STAT3 protein resulting from alternative splicing are also known in the art, such as: R382W, F384L, F384S, T389I, N395Y, R423Q, N425Y, H437Y, Del-463, S611N, F621V, T622I, V637L, V637M, Del-644, Y657C, P330S, K392R, N646K, K658N, Del-701, or T716M. Therefore, the payloads of the present disclosure can be designed to reduce or inhibit expression of the natural variants of the STAT3 protein.

SEQ ID NO: 30 is identical to a *STAT3* pre-mRNA sequence except that nucleotide "t" in SEQ ID NO: 30 is shown as "u" in pre-mRNA. In certain aspects, the "target nucleic acid" comprises an intron of a STAT3 protein-encoding nucleic acids or naturally occurring variants thereof, and RNA nucleic acids derived therefrom, *e.g*., pre-mRNA. In certain aspects, the target nucleic acid comprises an exon region of a STAT3 protein-encoding nucleic acids or naturally occurring variants thereof, and RNA nucleic acids derived therefrom, *e.g.*, pre-mRNA. In certain aspects, the target nucleic acid comprises an exon-intron junction of a STAT3 protein-encoding nucleic acids or naturally occurring variants thereof, and RNA nucleic acids derived therefrom, *e.g*., pre-mRNA. In some aspects, for example when used in research or diagnostics the "target nucleic acid" can be a cDNA or a synthetic oligonucleotide derived from the above DNA or RNA nucleic acid targets. The human STAT3 protein sequence encoded by the *STAT3* pre-mRNA is shown as SEQ ID NO: 31. In certain aspects, the target nucleic acid comprises an untranslated region of a STAT3 protein-encoding nucleic acids or naturally occurring variants thereof, *e.g*., 5' UTR, 3' UTR, or both.

In some aspects, the target nucleic acid comprises an exon-intron junction of a STAT3 protein-encoding nucleic acids or naturally occurring variants thereof, and RNA nucleic acids derived therefrom, *e.g*., pre-mRNA. In some aspects, for example when used in research or diagnostics the "target nucleic acid" can be a cDNA or a synthetic oligonucleotide derived from the above DNA or RNA nucleic acid targets. The human STAT3 protein sequence encoded by the *STAT3* pre-mRNA is shown as SEQ ID NO: 32. In certain aspects, the target nucleic acid comprises an untranslated region of a STAT3 protein-encoding nucleic acids or naturally occurring variants thereof, *e.g.,* 5' UTR, 3' UTR, or both.

In some aspects, a payload (*e.g.,* ASO) hybridizes to a region within the introns of *a STAT3* transcript, *e.g.,* SEQ ID NO: 30 or SEQ ID NO: 32. In certain aspects, a payload of the disclosure hybridizes to a region within the exons of a *STAT3* transcript, *e.g.,* SEQ ID NO: 41 or SEQ ID NO: 32. In certain aspects, a payload of the disclosure hybridizes to a region within the exon-intron junction of a *STAT3* transcript, *e.g.,* SEQ ID NO: 30 or SEQ ID NO: 32. In some aspects, a payload of the disclosure hybridizes to a region within a *STAT3* transcript *(e.g.,* an intron, exon, or exon-intron junction), *e.g*., SEQ ID NO: 30 or SEQ ID NO: 32.

In some aspects, the payload targets a mRNA encoding a particular isoform of STAT3 protein (e.g., Isoform 1). In some aspects, the payload targets all isoforms of STAT3 protein. In certain aspects, the payload targets two isoforms (*e.g*., Isoform 1 (UniProt ID: P40763-1) and Isoform 2 (UniProt ID: P40763-2), Isoform 2 and Isoform 3 (UniProt ID: P40763-3) of STAT3 protein.

In some aspects, a payload of the disclosure hybridizes to a region within the introns of a *STAT3* transcript, *e.g.,* SEQ ID NO: 30 or SEQ ID NO: 32. In certain aspects, a payload of the disclosure hybridizes to a region within the exons of a *STAT3* transcript, *e.g.,* SEQ ID NO: 30 or SEQ ID NO: 32. In certain aspects, a payload of the disclosure hybridizes to a region within the exon-intron junction of a *STAT3* transcript, *e.g.,* SEQ ID NO: 30 or SEQ ID NO: 32. In some aspects, a payload of the disclosure hybridizes to a region within a *STAT3* transcript *(e.g.,* an intron, exon, or exon-intron junction), *e.g*., SEQ ID NO: 30 or SEQ ID NO: 32, wherein the payload has a design according to formula: 5' A-B-C 3' as described elsewhere herein.

In some aspects, the payload of the present disclosure hybridizes to multiple target regions within the *STAT3* transcript (*e.g.,* genomic sequence, SEQ ID NO: 30). In some aspects, the payload hybridizes to two different target regions within the *STAT3* transcript. In some aspects, the payload hybridizes to three different target regions within the *STAT3* transcript. In some aspects, the payloads that hybridizes to multiple regions within the *STAT3* transcript (*e.g.,* genomic sequence, SEQ ID NO: 30) are more potent (*e.g.,* having lower EC50) at reducing *STAT3* expression compared to payloads that hybridizes to a single region within the *STAT3* transcript (*e.g.,* genomic sequence, SEQ ID NO: 30).

The payloads of the disclosure comprise a contiguous nucleotide sequence which corresponds to the complement of a region of *STAT3* transcript, *e.g.,* a nucleotide sequence corresponding to SEQ ID NO: 30.

In some aspects, binding of an payload targeting a *STAT3* transcript disclosed herein to a mRNA transcript encoding *STAT3* can reduce expression levels and/or activity levels of *STAT3.*

In some aspects, the payload comprises an ASO that can specifically target *a STAT3* transcript. In some aspects, the nucleotide sequence of an ASO, or the contiguous nucleotide sequence thereof, has at least about 80% sequence identity to a sequence set forth in any one of SEQ ID NOs: 92 to 101 (*i.e.,* the sequences in FIG. 3F), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 92 to 101, or a region of at least 10 contiguous nucleotides thereof, wherein the ASO (or contiguous nucleotide portion thereof) can optionally comprise one, two, three, or four mismatches when compared to the corresponding *STAT3* transcript.

In some aspects, the ASO comprises the sequence set forth in SEQ ID NO: 92. In some aspects, the ASO comprises the sequence set forth in SEQ ID NO: 93. In some aspects, the ASO comprises the sequence set forth in SEQ ID NO: 94. In some aspects, the ASO comprises the sequence set forth in SEQ ID NO: 95. In some aspects, the ASO comprises the sequence set forth in SEQ ID NO: 96. In some aspects, the ASO comprises the sequence set forth in SEQ ID NO: 97. In some aspects, the ASO comprises the sequence set forth in SEQ ID NO: 98. In some aspects, the ASO comprises the sequence set forth in SEQ ID NO: 99. In some aspects, the ASO comprises the sequence set forth in SEQ ID NO: 100. In some aspects, the ASO comprises the sequence set forth in SEQ ID NO: 101.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 92 to 101. In some aspects, the ASO (or contiguous nucleotide portion thereof) is selected from, or comprises, one of the sequences selected from the group consisting of SEQ ID NOs: 92 to 101 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 92 to 101 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *STAT3* transcript. In some aspects, the ASO (or contiguous nucleotide portion thereof) is selected from, or comprises, one of the sequences selected from the group consisting of SEQ ID NOs: 92 to 101 except for 1, 2, 3, 4, 5, 6, 7, 8, 9 or 10 substitutions, wherein the substituted ASO can bind to the *STAT3* transcript. In some aspects, the ASO (or contiguous nucleotide portion thereof) is selected from, or comprises, one of the sequences selected from the group consisting of SEQ ID NOs: 92 to 101 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *STAT3* transcript.

### II.G.1.g NLRP3

In some aspects, the payloads (*e.g.,* ASOs) provided herein are capable of down-regulating (*e.g.,* reducing or inhibiting) the expression of any gene or protein of interest in a cell. In certain aspects, the payloads described herein (*e.g.,* can be loaded in EVs using the loading methods of the present disclosure) target one or more regions of the *NLRP3* pre-mRNA (*e.g.,* intron regions, exon regions, and/or exon-intron junction regions). In some aspects, the payload is capable of targeting all isoforms of the NLRP3 protein. In certain aspects, the payload targets specific NLRP3 isoforms. Unless indicated otherwise, the term "NLRP3," as used herein, can refer to STAT6 from one or more species (*e.g.,* humans, non-human primates, dogs, cats, guinea pigs, rabbits, rats, mice, horses, cattle, and bears).

NLRP3 (*NLRP3*) is also known as NLR family pyrin domain containing 3. Unless indicated otherwise, the term "NLRP3," as used herein, can refer to NLRP3 from one or more species (*e.g.,* humans, non-human primates, dogs, cats, guinea pigs, rabbits, rats, mice, horses, cattle, and bears). Synonyms of NLRP3/NLRP3 are known and include *NLRP3; C1orf7*; *CIAS1*; *NALP3; PYPAF1*; nucleotide-binding oligomerization domain, leucine rich repeat and pyrin domain containing 3; cold-induced autoinflammatory syndrome 1 protein; cryopyin; NACHT, LRR and PYD domains-containing protein 3; angiotensin/vasopressin receptor AII/AVP-like; caterpiller protein 1.1; CLR1.1; cold-induced autoinflammatory syndrome 1 protein; and PYRIN-containing APAF1-like protein 1. The sequence for the human NLRP3 gene can be found under publicly available GenBank Accession Number NC_000001.11:247416156-247449108. The human *NLRP3* gene is found at chromosome location 1q44 at 247,416,156-247,449,108.

The sequence for the human *NLRP3* pre-mRNA transcript (SEQ ID NO: 33) corresponds to the reverse complement of residues 247,416,156-247,449,108 of chromosome 1q44. The *NLRP3* mRNA sequence (GenBank Accession No. NM_001079821.2) is provided in SEQ ID NO: 35, except that the nucleotide "t" in SEQ ID NO: 35 is shown as "u" in the mRNA. The sequence for human NLRP3 protein can be found under publicly available Accession Numbers: Q96P20, (canonical sequence, SEQ ID NO: 34), Q96P20-2 (SEQ ID NO: 36), Q96P20-3 (SEQ ID NO: 37), Q96P20-4 (SEQ ID NO: 38), Q96P20-5 (SEQ ID NO: 39), and Q96P20-6 (SEQ ID NO: 40).

Natural variants of the human *NLRP3* gene product are known. For example, natural variants of human NLRP3 protein can contain one or more amino acid substitutions selected from: D21H, I174T, V200M, R262L, 4262P, R262W, L266H, D305G, D305N, L307P, Q308K, F311S, T350M, A354V, L355P, E356D, H360R, T407P, T438I, T438N, A441T, A441V, R490K, F525C, F525L, G571R, Y572C, F575S, E629G, L634F, M664T, Q705K, Y861C, and R920Q, and any combinations thereof. Additional variants of human NLRP3 protein resulting from alternative splicing are also known in the art. NLRP3 Isoform 1 (identifier: Q96P20-2 at UniProt) differs from the canonical sequence (SEQ ID NO: 35) as follows: deletion of residues 721-777 and 836-892 relative to SEQ ID NO: 35. The sequence of NLRP3 Isoform 3 (identifier: Q96P20-3) differs from the canonical sequence (SEQ ID NO: 35) as follows: deletion of residues 720-1036 relative to SEQ ID NO: 35. The sequence of NLRP3 Isoform 4 (identifier: Q96P20-4) differs from the canonical sequence (SEQ ID NO: 35) as follows: deletion of residues 721-777 relative to SEQ ID NO: 35. The sequence of NLRP3 Isoform 5 (identifier: Q96P20-5) differs from the canonical sequence (SEQ ID NO: 35) as follows: deletion of residues 836-892 relative to SEQ ID NO: 35. The sequence of NLRP3 Isoform 6 (identifier: Q96P20-6) differs from the canonical sequence (SEQ ID NO: 35) as follows: deletion of residues 776-796 relative to SEQ ID NO: 35. Therefore, the payloads of the present disclosure can be designed to reduce or inhibit expression of the natural variants of the NLRP3 protein.

An example of a target nucleic acid sequence of the payloads is *NLRP3* pre-mRNA. SEQ ID NO: 33 represents a human *NLRP3* genomic sequence *(i.e.,* reverse complement of nucleotides 247,416,156-247,449,108 of chromosome 1q44). SEQ ID NO: 33 is identical to a *NLRP3* pre-mRNA sequence except that nucleotide "t" in SEQ ID NO: 33 is shown as "u" in pre-mRNA. In certain aspects, the "target nucleic acid" comprises an intron of a NLRP3 protein-encoding nucleic acids or naturally occurring variants thereof, and RNA nucleic acids derived therefrom, *e.g*., pre-mRNA. In certain aspects, the target nucleic acid comprises an exon region of a NLRP3 protein-encoding nucleic acids or naturally occurring variants thereof, and RNA nucleic acids derived therefrom, *e.g*., pre-mRNA. In certain aspects, the target nucleic acid comprises an exon-intron junction of a NLRP3 protein-encoding nucleic acids or naturally occurring variants thereof, and RNA nucleic acids derived therefrom, *e.g.*, pre-mRNA. In some aspects, for example when used in research or diagnostics the "target nucleic acid" can be a cDNA or a synthetic oligonucleotide derived from the above DNA or RNA nucleic acid targets. The human NLRP3 protein sequence encoded by the *NLRP3* pre-mRNA is shown as SEQ ID NO: 35. In certain aspects, the target nucleic acid comprises an untranslated region of a NLRP3 protein-encoding nucleic acids or naturally occurring variants thereof, *e.g.,* 5' UTR, 3' UTR, or both.

In some aspects, a payload *(e.g.,* ASO) hybridizes to a region within the introns of *a NLRP3* transcript, *e.g.,* SEQ ID NO: 33. In certain aspects, a payload of the disclosure hybridizes to a region within the exons of a *NLRP3* transcript, *e.g.,* SEQ ID NO: 33. In certain aspects, a payload of the disclosure hybridizes to a region within the exon-intron junction of a *NLRP3* transcript, *e.g*., SEQ ID NO: 33. In some aspects, a payload of the disclosure hybridizes to a region within a *NLRP3* transcript (*e.g.,* an intron, exon, or exon-intron junction), *e.g.,* SEQ ID NO: 33.

In some aspects, the payload targets a mRNA encoding a particular isoform of NLRP3 protein (*e.g.,* Isoform 1). In some aspects, the payload targets all isoforms of NLRP3 protein. In certain aspects, the payload targets two isoforms (*e.g.,* Isoform 1 and Isoform 2, Isoform 3 and Isoform 4, and Isoform 5 and Isoform 6) of NLRP3 protein.

In some aspects, binding of an payload targeting a *NLRP3* transcript disclosed herein to a mRNA transcript encoding *NLRP3* can reduce expression levels and/or activity levels of *NLRP3.*

In some aspects, the payload comprises an ASO that can specifically target a*NLRP3* transcript. In some aspects, the nucleotide sequence of an ASO, or the contiguous nucleotide sequence thereof, has at least about 80% sequence identity to a sequence set forth in any one of SEQ ID NOs: 102 to 112 *(i.e.,* the sequences in FIG. 3G), such as at least about 80%, at least about 85%, at least about 90%, at least about 91%, at least about 92%, at least about 93%, at least about 94%, at least about 95%, at least about 96% sequence identity, at least about 97% sequence identity, at least about 98% sequence identity, at least about 99% sequence identity, such as about 100% sequence identity.

In some aspects, the ASO (or contiguous nucleotide portion thereof) is selected from, or comprises, one of the sequences selected from the group consisting of SEQ ID NOs: 102 to 112, or a region of at least 10 contiguous nucleotides thereof, wherein the ASO (or contiguous nucleotide portion thereof) can optionally comprise one, two, three, or four mismatches when compared to the corresponding *NLRP3* transcript.

In some aspects, the ASO comprises the sequence set forth in SEQ ID NO: 102. In some aspects, the ASO comprises the sequence set forth in SEQ ID NO: 103. In some aspects, the ASO comprises the sequence set forth in SEQ ID NO: 104. In some aspects, the ASO comprises the sequence set forth in SEQ ID NO: 105. In some aspects, the ASO comprises the sequence set forth in SEQ ID NO: 106. In some aspects, the ASO comprises the sequence set forth in SEQ ID NO: 107. In some aspects, the ASO comprises the sequence set forth in SEQ ID NO: 108. In some aspects, the ASO comprises the sequence set forth in SEQ ID NO: 109. In some aspects, the ASO comprises the sequence set forth in SEQ ID NO: 110. In some aspects, the ASO comprises the sequence set forth in SEQ ID NO: 111. In some aspects, the ASO comprises the sequence set forth in SEQ ID NO: 112.

In some aspects the ASO comprises or consists of a sequence at least about 60%, at least about 65%, at least about 70%, at least about 75%, at least about 80%, at least about 85%, at least about 90%, at least about 95%, at least about 96%, at least about 97%, at least about 98%, at least about 99%, or about 100% identical to a sequence set forth in SEQ ID NOs: 102 to 112. In some aspects, the ASO (or contiguous nucleotide portion thereof) is selected from, or comprises, one of the sequences selected from the group consisting of SEQ ID NOs: 102 to 112 or a region of at least 8, 9, 10, 11, 12, 13, 14, 15, 16, 17, 18, 19, or 20 contiguous nucleotides thereof. In some aspects, the ASO (or contiguous nucleotide portion thereof) is selected from, or comprises, one of the sequences selected from the group consisting of SEQ ID NOs: 102 to 112 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four mismatches when compared to the corresponding *NLRP3* transcript. In some aspects, the ASO (or contiguous nucleotide portion thereof) is selected from, or comprises, one of the sequences selected from the group consisting of SEQ ID NOs: 102 to 112 except for 1, 2, 3, 4, 5, 6, 7, 8, 9 or 10 substitutions, wherein the substituted ASO can bind to the *NLRP3* transcript. In some aspects, the ASO (or contiguous nucleotide portion thereof) is selected from, or comprises, one of the sequences selected from the group consisting of SEQ ID NOs: 102 to 112 or a region of at least 8, 9, 10, 11, 12, 13, 14, 15, 16, 17, 18, 19, or 20 contiguous nucleotides thereof, wherein the ASO (or contiguous nucleotide portion thereof) can optionally comprise one, two, three, or four additional 5' and/or 3' nucleotides complementary to the corresponding *NLRP3* transcript.

### II.G.2 Anchoring Moiety

One or more anchoring moieties (AMs) can be used to anchor a payload (or any other moieties of interest) to EVs (*e.g.,* exosomes). In some aspects, the payload is linked directly to the anchoring moiety. In some aspects, the payload is linked to the anchoring moiety via a linker, such as those described herein. In some aspects, the payload can be attached to an anchoring moiety and/or linker via reaction between a "reactive group" (RG; e.g., amine, thiol, hydroxy, carboxylic acid, or azide) with a "reactive moiety" (RM; e.g., maleimide, succinate, NHS). Several potential synthetic routes are envisioned, for example:
[AM]-/Reactive moiety/ + /Reactive group/-[payload]
[AM]-[Linker]n-/Reactive moiety/ + /Reactive group/-[payload]
[AM]-/Reactive moiety/ + /Reactive group/-[Linker]n-[payload]
[AM]- [Linker]n-/Reactive moiety/ + /Reactive group/-[Linker]n-[payload]

The anchoring moiety can insert into the lipid bilayer of an EV allowing the loading of the payload. In some aspect, an anchoring moiety can be chemically conjugated to a payload to enhance its hydrophobic character, and thereby, enhance the loading of the EV with the payload. In some aspects, the anchoring moiety comprises a sterol (e.g., cholesterol), GM1, a lipid, a vitamin, a small molecule, a peptide, or a combination thereof.

In some aspects, the anchoring moiety is a lipid. Any suitable lipid known in the art can be used as the anchoring moiety (*e.g.,* palmitic acid or glycosylphosphatidylinositols). In some aspects, the anchoring moiety is a sterol, e.g., cholesterol. Additional hydrophobic moieties include, for example, phospholipids, lysophospholipids, fatty acids, or vitamins (e.g., vitamin D or vitamin E).

In some aspects, anchoring moieties are chemically attached. In certain aspects, an anchoring moiety can be attached to a payload (*e.g.,* ASO) enzymatically. In some aspects, an anchoring moiety can be attached to a payload via modification of cell culture conditions. For example, by using a culture medium where myristic acid is limiting, some other fatty acids including shorter-chain and unsaturated, can be attached to an N-terminal glycine. For example, in BK channels, myristate has been reported to be attached post-translationally to internal serine/threonine or tyrosine residues via a hydroxyester linkage.

The anchoring moiety can be conjugated to a payload directly or indirectly via a linker combination, at any chemically feasible location, e.g., at the 5' and/or 3' end of the payload. In certain aspects, the anchoring moiety is conjugated only to the 3' end of the payload. In certain aspects, the anchoring moiety is conjugated only to the 5' end of the payload. In some aspects, the anchoring moiety is conjugated at a location which is not the 3' end or 5' end of the payload.

Non-limiting examples of anchoring moieties (including their structure) are provided below:

| *Modification* | *Modifying Group* |
|---|---|
| S-Palmitoylation | 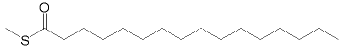 |
| N-Palmitoylation | 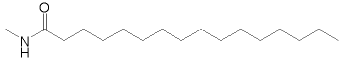 |
| N-Myristoylation | 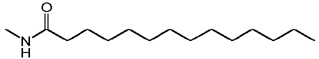 |
| O-Acylation | 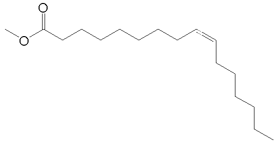 |
| Farnesylation | 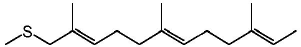 |
| Geranylgeranylation | 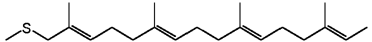 |
| Cholesterol | 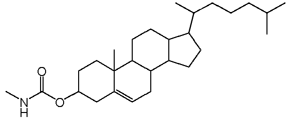 |

In some aspects, an anchoring moiety useful for the present disclosure comprises two or more types of anchoring moieties disclosed herein. For example, in some aspects, an anchoring moiety can comprise two lipids, e.g., a phospholipids and a fatty acid, or two phospholipids, or two fatty acids, or a lipid and a vitamin, or cholesterol and a vitamin, etc. which taken together have 6-80 carbon atoms (i.e., an equivalent carbon number (ECN) of 6-80).

In some aspects, the combination of anchoring moieties, e.g., a combination of the lipids (e.g., fatty acids) has an ECN of 6-80, 8-80, 10-80, 12-80, 14-80, 16-80, 18-80, 20-80, 22-80, 24-80, 26-80, 28-80, 30-80, 4-76, 6-76, 8-76, 10-76, 12-76, 14-76, 16-76, 18-76, 20-76, 22-76, 24-76, 26-76, 28-76, 30-76, 6-72, 8-72, 10-72, 12-72, 14-72, 16-72, 18-72, 20-72, 22-72, 24-72, 26-72, 28-72, 30-72, 6-68, 8-68, 10-68, 12-68, 14-68, 16-68, 18-68, 20-68, 22-68, 24-68, 26-68, 28-68, 30-68, 6-64, 8-64, 10-64, 12-64, 14-64, 16-64, 18-64, 20-64, 22-64, 24-64, 26-64, 28-64, 30-64, 6-60, 8-60, 10-60, 12-56, 14-56, 16-56, 18-56, 20-56, 22-56, 24-56, 26-56, 28-56, 30-56, 6-52, 8-52, 10-52, 12-52, 14-52, 16-52, 18-52, 20-52, 22-52, 24-52, 26-52, 28-52, 30-52, 6-48, 8-48, 10-48, 12-48, 14-48, 16-48, 18-48, 20-48, 22-48, 24-48, 26-48, 28-48, 30-48, 6-44, 8-44, 10-44, 12-44, 14-44, 16-44, 18-44, 20-44, 22-44, 24-44, 26-44, 28-44, 30-44, 6-40, 8-40, 10-40, 12-40, 14-40, 16-40, 18-40, 20-40, 22-40, 24-40, 26-40, 28-40, 30-40, 6-36, 8-36, 10-36, 12-36, 14-36, 16-36, 18-36, 20-36, 22-36, 24-36, 26-36, 28-36, 30-36, 6-32, 8-32, 10-32, 12-32, 14-32, 16-32, 18-32, 20-32, 22-32, 24-32, 26-32, 28-32, or 30-32.

### II.G.1.a. Cholesterol and Other Sterols

In some aspects, the anchoring moiety comprises a sterol, steroid, hopanoid, hydroxysteroid, secosteroid, or analog thereof with lipophilic properties. In some aspects, the anchoring moiety comprises a sterol, such as a phytosterol, mycosterol, or zoosterol. Exemplary zoosterols include cholesterol and 24S-hydroxycholesterol; exemplary phytosterols include ergosterol (mycosterol), campesterol, sitosterol, and stigmasterol. In some aspects, the sterol is selected from ergosterol, 7-dehydrocholesterol, cholesterol, 24S-hydroxycholesterol, lanosterol, cycloartenol, fucosterol, saringosterol, campesterol, β-sitosterol, sitostanol, coprostanol, avenasterol, or stigmasterol. Sterols can be found either as free sterols, acylated (sterol esters), alkylated (steryl alkyl ethers), sulfated (sterol sulfate), or linked to a glycoside moiety (steryl glycosides), which can be itself acylated (acylated sterol glycosides).

In some aspects, the anchoring moiety comprises a steroid. In some aspects, the steroid is selected from dihydrotestosterone, uvaol, hecigenin, diosgenin, progesterone, or cortisol.

For example, sterols can be conjugated to the payload (*e.g.,* ASO) directly or via a linker combination at the available -OH group of the sterol. Exemplary sterols have the general skeleton shown below:

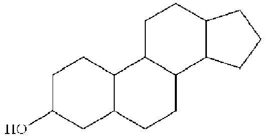

As a further example, ergosterol has the structure below:

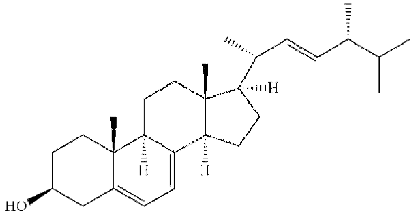

Cholesterol has the structure below:

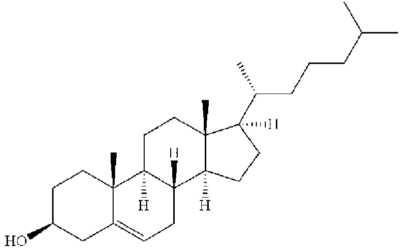

Accordingly, in some aspects, the free -OH group of a sterol or steroid is used to conjugate the payload directly or via a linker combination, to the sterol (e.g., cholesterol) or steroid. In some aspects, the cholesterol that can be used to conjugate a payload described herein *(e.g.,* ASO) has the structure shown in FIG. 2A. In some aspects, the cholesterol that can be used to conjugate a payload described herein *(e.g.,* ASO) has the structure shown in FIG. 2B. As described herein, the cholesterol shown in FIG. 2A can be attached to a linker, *e.g*., triethylene glycol (referred to herein as "chol2"). In some aspects, the cholesterol shown in FIG. 2B can be attached to a linker, *e.g*., tetraethylene glycol (referred to herein as "chol4").

### II.G.2.b. Fatty Acids

In some aspects, the anchoring moiety is a fatty acid. In some aspects, the fatty acid is a short-chain, medium-chain, or long-chain fatty acid. In some aspects, the fatty acid is a saturated fatty acid. In some aspects, the fatty acid is an unsaturated fatty acid. In some aspects, the fatty acid is a monounsaturated fatty acid. In some aspects, the fatty acid is a polyunsaturated fatty acid, such as an omega-3 or omega-6 fatty acid.

In some aspects, the lipid, e.g., fatty acid, has a C₂-C₆₀ chain. In some aspects, the lipid, e.g., fatty acid, has a C₂-C₂₈ chain. In some aspects, the fatty acid, has a C₂-C₄₀ chain. In some aspects, the fatty acid, has a C₂-C₁₂ or C₄-C₁₂ chain. In some aspects, the fatty acid, has a C₄-C₄₀ chain. In some aspects, the fatty acid, has a C₄-C₄₀, C₂-C₃₈, C₂-C₃₆, C₂-C₃₄, C₂-C₃₂, C₂-C₃₀, C₄-C₃₀, C₂-C₂₈, C₄-C₂₈, C₂- C₂₆, C₄-C₂₆, C₂-C₂₄, C₄-C₂₄, C₆-C₂₄, C₈-C₂₄, C₁₀-C₂₄, C₂-C₂₂, C₄-C₂₂, C₆-C₂₂, C₈-C₂₂, C₁₀-C₂₂, C₂-C₂₀, C₄-C₂₀, C₆-C₂₀, C₈-C₂₀, C₁₀-C₂₀, C₂-C₁₈, C₄-C₁₈, C₆-C₁₈, C₈-C₁₈, C₁₀-C₁₈, C₁₂-C₁₈, C₁₄-C₁₈, C₁₆-C₁₈, C₂-C₁₆, C₄-C₁₆, C₆-C₁₆, C₈-C₁₆, C₁₀-C₁₆, C₁₂-C₁₆, C₁₄-C₁₆, C₂-C₁₅, C₄-C₁₅, C₆-C₁₅, C₈-C₁₅, C₉-C₁₅, C₁₀-C₁₅, C₁₁-C₁₅, C₁₂-C₁₅, C₁₃-C₁₅, C₂-C₁₄, C₄-C₁₄, C₆-C₁₄, C₈-C₁₄, C₉-C₁₄, C₁₀-C₁₄, C₁₁-C₁₄, C₁₂-C₁₄, C₂-C₁₃, C₄-C₁₃, C₆-C₁₃, C₇-C₁₃, C₈-C₁₃, C₉-C₁₃, C₁₀-C₁₃, C₁₀-C₁₃, C₁₁-C₁₃, C₂-C₁₂, C₄-C₁₂, C₆-C₁₂, C₇-C₁₂, C₈-C₁₂, C₉-C₁₂, C₁₀-C₁₂, C₂-C₁₁, C₄-C₁₁, C₆-C₁₁, C₇-C₁₁, C₈-C₁₁, C₉-C₁₁, C₂-C₁₀, C₄-C₁₀, C₂-C₉, C₄-C₉, C₂-C₈, C₂-C₇, C₄-C₇, C₂-C₆, or C₄-C₆, chain. In some aspects, the fatty acid has a C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, C₃₁, C₃₂, C₃₃, C₃₄, C₃₅, C₃₆, C₃₇, C₃₈, C₃₉, C₄₀, C₄₁, C₄₂, C₄₃, C₄₄, C₄₅, C₄₆, C₄₇, C₄₈, C₄₉, C₅₀, C₅₁, C₅₂, C₅₃, C₅₄, C₅₅, C₅₆, C₅₇, C₅₈, C₅₉, or C₆₀ chain.

In some aspects, the anchoring moiety comprises two fatty acids, each of which is independently selected from a fatty acid having a chain with any one of the foregoing ranges or numbers of carbon atoms. In some aspects, one of the fatty acids is independently a fatty acid with a C6-C21 chain and one is independently a fatty acid with a C12-C36 chain. In some aspects, each fatty acid independently has a chain of 11, 12, 13, 14, 15, 16, or 17 carbon atoms.

Suitable fatty acids include saturated straight-chain fatty acids, saturated branched fatty acids, unsaturated fatty acids, hydroxy fatty acids, and polycarboxylic acids. In some aspects, such fatty acids have up to 32 carbon atoms.

Examples of useful saturated straight-chain fatty acids include those having an even number of carbon atoms, such as butyric acid, caproic acid, caprylic acid, capric acid, lauric acid, myristic acid, palmitic acid, stearic acid, arachic acid, behenic acid, lignoceric acid, hexacosanoic acid, octacosanoic acid, triacontanoic acid and n-dotriacontanoic acid, and those having an odd number of carbon atoms, such as propionic acid, n-valeric acid, enanthic acid, pelargonic acid, hendecanoic acid, tridecanoic acid, pentadecanoic acid, heptadecanoic acid, nonadecanoic acid, heneicosanoic acid, tricosanoic acid, pentacosanoic acid, and heptacosanoic acid.

Examples of suitable saturated branched fatty acids include isobutyric acid, isocaproic acid, isocaprylic acid, isocapric acid, isolauric acid, 11-methyldodecanoic acid, isomyristic acid, 13-methyl-tetradecanoic acid, isopalmitic acid, 15-methyl-hexadecanoic acid, isostearic acid, 17-methyloctadecanoic acid, isoarachic acid, 19-methyl-eicosanoic acid, α-ethylhexanoic acid, α-hexyldecanoic acid, α-heptylundecanoic acid, 2-decyltetradecanoic acid, 2-undecyltetradecanoic acid, 2-decylpentadecanoic acid, 2-undecylpentadecanoic acid, and Fine oxocol 1800 acid (product of Nissan Chemical Industries, Ltd.). Suitable saturated odd-carbon branched fatty acids include anteiso fatty acids terminating with an isobutyl group, such as 6-methyl-octanoic acid, 8-methyl-decanoic acid, 10-methyl-dodecanoic acid, 12-methyl-tetradecanoic acid, 14-methyl-hexadecanoic acid, 16-methyl-octadecanoic acid, 18-methyl-eicosanoic acid, 20-methyl-docosanoic acid, 22-methyl-tetracosanoic acid, 24-methyl-hexacosanoic acid, and 26-methyloctacosanoic acid.

Examples of suitable unsaturated fatty acids include 4-decenoic acid, caproleic acid, 4-dodecenoic acid, 5-dodecenoic acid, lauroleic acid, 4-tetradecenoic acid, 5-tetradecenoic acid, 9-tetradecenoic acid, palmitoleic acid, 6-octadecenoic acid, oleic acid, 9-octadecenoic acid, 11-octadecenoic acid, 9-eicosenoic acid, cis-11-eicosenoic acid, cetoleic acid, 13-docosenoic acid, 15-tetracosenoic acid, 17-hexacosenoic acid, 6,9,12,15-hexadecatetraenoic acid, linoleic acid, linolenic acid, α-eleostearic acid, β-eleostearic acid, punicic acid, 6,9,12,15-octadecatetraenoic acid, parinaric acid, 5,8,11,14-eicosatetraenoic acid, 5,8,11,14,17-eicosapentaenoic acid, 7,10,13,16,19-docosapentaenoic acid, 4,7,10,13,16,19-docosahexaenoic acid, and the like.

Examples of suitable hydroxy fatty acids include α-hydroxylauric acid, α-hydroxymyristic acid, α-hydroxypalmitic acid, α-hydroxystearic acid, ω-hydroxylauric acid, α-hydroxyarachic acid, 9-hydroxy-12-octadecenoic acid, ricinoleic acid, α-hydroxybehenic acid, 9-hydroxy-trans-10,12-octadecadienic acid, kamolenic acid, ipurolic acid, 9,10-dihydroxystearic acid, 12-hydroxystearic acid and the like.

Examples of suitable polycarboxylic acids include oxalic acid, malonic acid, succinic acid, glutaric acid, adipic acid, pimelic acid, suberic acid, azelaic acid, sebacic acid, D,L-malic acid, and the like.

In some aspects, each fatty acid is independently selected from propionic acid, butyric acid, valeric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, pentacosylic acid, cerotic acid, heptacosylic acid, montanic acid, nonacosylic acid, melissic acid, henatriacontylic acid, lacceroic acid, psyllic acid, geddic acid, ceroplastic acid, hexatriacontylic acid, heptatriacontanoic acid, or octatriacontanoic acid.

In some aspects, each fatty acid is independently selected from α-linolenic acid, stearidonic acid, eicosapentaenoic acid, docosahexaenoic acid, linoleic acid, gamma-linoleic acid, dihomo-gamma-linoleic acid, arachidonic acid, docosatetraenoic acid, palmitoleic acid, vaccenic acid, paullinic acid, oleic acid, elaidic acid, gondoic acid, eurcic acid, nervonic acid, mead acid, adrenic acid, bosseopentaenoic acid, ozubondo acid, sardine acid, herring acid, docosahexaenoic acid, or tetracosanolpentaenoic acid, or another monounsaturated or polyunsaturated fatty acid.

In some aspects, one or both of the fatty acids is an essential fatty acid. In view of the beneficial health effects of certain essential fatty acids, the therapeutic benefits of disclosed therapeutic-loaded exosomes can be increased by including such fatty acids in the therapeutic agent. In some aspects, the essential fatty acid is an n-6 or n-3 essential fatty acid selected from the group consisting of linolenic acid, gamma-linolenic acid, dihomo-gamma-linolenic acid, arachidonic acid, adrenic acid, docosapentaenoic n-6 acid, alpha-linolenic acid, stearidonic acid, the 20:4n-3 acid, eicosapentaenoic acid, docosapentaenoic n-3 acid, or docosahexaenoic acid.

In some aspects, each fatty acid is independently selected from all-cis-7,10,13-hexadecatrienoic acid, α-linolenic acid, stearidonic acid, eicosatrienoic acid, eicosatetraenoic acid, eicosapentaenoic acid (EPA), docosapentaenoic acid, docosahexaenoic acid (DHA), tetracosapentaenoic acid, tetracosahexaenoic acid, or lipoic acid. In some aspects, the fatty acid is selected from eicosapentaenoic acid, docosahexaenoic acid, or lipoic acid. Other examples of fatty acids include all-cis-7,10,13-hexadecatrienoic acid, α-linolenic acid (ALA or all-cis-9,12,15-octadecatrienoic acid), stearidonic acid (STD or all-cis-6,9,12,15-octadecatetraenoic acid), eicosatrienoic acid (ETE or all-cis-11,14,17-eicosatrienoic acid), eicosatetraenoic acid (ETA or all-cis-8,11,14,17-eicosatetraenoic acid), eicosapentaenoic acid (EPA), docosapentaenoic acid (DPA, clupanodonic acid or all-cis-7,10,13,16,19-docosapentaenoic acid), docosahexaenoic acid (DHA or all-cis-4,7,10,13,16,19-docosahexaenoic acid), tetracosapentaenoic acid (all-cis-9,12,15,18,21-docosahexaenoic acid), or tetracosahexaenoic acid (nisinic acid or all-cis-6,9,12,15,18,21-tetracosenoic acid). In some aspects, the fatty acid is a medium-chain fatty acid such as lipoic acid.

Fatty acid chains differ greatly in the length of their chains and can be categorized according to chain length, e.g. as short to very long. Short-chain fatty acids (SCFA) are fatty acids with chains of about five or less carbons (e.g. butyric acid). In some aspects, the fatty acid is a SCFA. Medium-chain fatty acids (MCFA) include fatty acids with chains of about 6-12 carbons, which can form medium-chain triglycerides. In some aspects, the fatty acid is a MCFA. Long-chain fatty acids (LCFA) include fatty acids with chains of 13-21 carbons. In some aspects, the fatty acid is a LCFA. In some aspects, the fatty acid is a LCFA. Very long chain fatty acids (VLCFA) include fatty acids with chains of 22 or more carbons, such as 22-60, 22-50, or 22-40 carbons. In some aspects, the fatty acid is a VLCFA.

### II.G.2.c. Phospholipids

In some aspects, the anchoring moiety comprises a phospholipid. Phospholipids are a class of lipids that are a major component of all cell membranes. They can form lipid bilayers because of their amphiphilic characteristic. The structure of the phospholipid molecule generally consists of two hydrophobic fatty acid "tails" and a hydrophilic "head" consisting of a phosphate group. For example, a phospholipid can be a lipid according to the following formula: 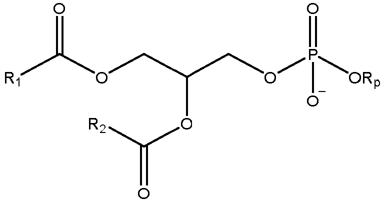 in which Rₚ represents a phospholipid moiety and R₁ and R₂ represent fatty acid moieties with or without unsaturation that can be the same or different.

A phospholipid moiety can be selected, for example, from the non-limiting group consisting of phosphatidyl choline, phosphatidyl ethanolamine, phosphatidyl glycerol, phosphatidyl serine, phosphatidic acid, 2 lysophosphatidyl choline, and a sphingomyelin.

Particular phospholipids can facilitate fusion to a lipid bilayer, e.g., the lipid bilayer of an exosomal membrane. For example, a cationic phospholipid can interact with one or more negatively charged phospholipids of a membrane. Fusion of a phospholipid to a membrane can allow one or more elements of a lipid-containing composition to bind to the membrane or to pass through the membrane.

A fatty acid moiety can be selected, for example, from the non-limiting group consisting of lauric acid, myristic acid, myristoleic acid, palmitic acid, palmitoleic acid, stearic acid, oleic acid, linoleic acid, alpha-linolenic acid, erucic acid, phytanoic acid, arachidic acid, arachidonic acid, eicosapentaenoic acid, behenic acid, docosapentaenoic acid, and docosahexaenoic acid.

The phospholipids using as anchoring moieties in the present disclosure can be natural or non-natural phospholipids. Non-natural phospholipid species including natural species with modifications and substitutions including branching, oxidation, cyclization, and alkynes are also contemplated. For example, a phospholipid can be functionalized with or cross-linked to one or more alkynes (e.g., an alkenyl group in which one or more double bonds is replaced with a triple bond). Under appropriate reaction conditions, an alkyne group can undergo a copper-catalyzed cycloaddition upon exposure to an azide.

Phospholipids include, but are not limited to, glycerophospholipids such as phosphatidylcholines, phosphatidylethanolamines, phosphatidylserines, phosphatidylinositols, phosphatidy glycerols, and phosphatidic acids.

### II.G.2.d. Lysolipids (e.g., lysophospholipids)

In some aspects, the anchoring moiety comprises a lysolipid, e.g., a lysophospholipid. Lysolipids are derivatives of a lipid in which one or both fatty acyl chains have been removed, generally by hydrolysis. Lysophospholipids are derivatives of a phospholipid in which one or both fatty acyl chains have been removed by hydrolysis.

In some aspects, the anchoring moiety comprises any of the phospholipids disclosed above, in which one or both acyl chains have been removed via hydrolysis, and therefore the resulting lysophospholipid comprises one or no fatty acid acyl chain.

In some aspects, the anchoring moiety comprises a lysoglycerophospholipid, a lysoglycosphingoliopid, a lysophosphatidylcholine, a lysophosphatidylethanolamine, a lysophosphatidylinositol, or a lysophosphatidylserine.

### II.G.2.e. Vitamins

In some aspects, the anchoring moiety comprises a lipophilic vitamin, e.g., folic acid, vitamin A, vitamin E, pyridoxone, niacin, or vitamin K.

In some aspects, the anchoring moiety comprises vitamin A. Vitamin A is a group of unsaturated nutritional organic compounds that includes retinol, retinal, retinoic acid, and several provitamin A carotenoids (most notably beta-carotene). In some aspects, the anchoring moiety comprises retinol. In some aspects, the anchoring moiety comprises a retinoid. Retinoids are a class of chemical compounds that are vitamers of vitamin A or are chemically related to it. In some aspects, the anchoring moiety comprises a first generation retinoid (e.g., retinol, tretinoin, isotreatinoin, or alitretinoin), a second-generation retinoid (e.g., etretinate or acitretin), a third-generation retinoid (e.g., adapalene, bexarotene, or tazarotene), or any combination thereof.

### II.G.3 Linker combinations

In some aspects, a payload (*e.g.,* ASO) (or any other moieties of interest described herein) is linked to an anchoring moiety disclosed herein via a linker combination. In certain aspects, the linker combination comprises multiple (*e.g.,* two or more) cleavable linkers. In certain aspects, the linker combination comprises multiple (*e.g.,* two or more) non-cleavable linkers. In some aspects, the linker combination comprises multiple (*e.g.,* two or more) non-cleavable linkers and cleavable linkers.

Linkers can be susceptible to cleavage ("cleavable linker"), and thereby facilitating release of a biologically active molecule (*e.g.,* payload). Thus, in some aspects, a linker combination disclosed herein can comprise a cleavable linker. Such cleavable linkers can be susceptible, for example, to acid-induced cleavage, photo-induced cleavage, peptidase-induced cleavage, esterase-induced cleavage, reactive oxygen species-induced cleavage, diselenide bond cleavage, and disulfide bond cleavage, at conditions under which the biologically active molecule (*e.g.,* payload, *e.g.,* ASO) remains active. Alternatively, linkers can be substantially resistant to cleavage ("non-cleavable linker"). In some aspects, the cleavable linker comprises a spacer. In some aspects the spacer is PEG.

In some aspects, a linker combination comprises at least 2, at least 3, at least 4, at least 5, or at least 6 or more different linkers disclosed herein. In some aspects, linkers in a linker combination can be linked by an ester linkage (e.g., phosphodiester or phosphorothioate ester).

In some aspects, the linker is a direct bond between an anchoring moiety and a payload (*e.g.,* ASO).

### II.G.3.a Non-cleavable linkers

In some aspects, the linker combination comprises a non-cleavable liker. "Non-cleavable linkers" are any chemical moiety capable of linking two or more components of a modified biologically active molecule of the present disclosure (e.g., a biologically active molecule and an anchoring moiety; a biologically active molecule and a cleavable linker; an anchoring moiety and a cleavable linker) in a stable, covalent manner and does not fall off under the categories listed above for cleavable linkers. Thus, non-cleavable linkers are substantially resistant to acid-induced cleavage, photo-induced cleavage, peptidase-induced cleavage, esterase-induced cleavage and disulfide bond cleavage.

Furthermore, non-cleavable refers to the ability of the chemical bond in the linker or adjoining to the linker to withstand cleavage induced by an acid, photolabile-cleaving agent, a peptidase, an esterase, or a chemical or physiological compound that cleaves a disulfide bond, at conditions under which a cyclic dinucleotide and/or the antibody does not lose its activity. In some aspects, the biologically active molecule is attached to the linker via another linker, e.g., a self-immolative linker.

In some aspects, the linker combination comprises a non-cleavable linker comprising, e.g., tetraethylene glycol (TEG), hexaethylene glycol (HEG), polyethylene glycol (PEG), succinimide, or any combination thereof. In some aspects, the non-cleavable linker comprises a spacer unit to link the biologically active molecule to the non-cleavable linker.

In some aspects, one or more non-cleavable linkers comprise smaller units (e.g., HEG, TEG, glycerol, C2 to C12 alkyl, and the like) linked together. In one aspect, the linkage is an ester linkage (e.g., phosphodiester or phosphorothioate ester) or other linkage.

### II.G.3.a.1 Ethylene Glycols (HEG, TEG, PEG)

In some aspects, the linker combination comprises a non-cleavable linker, wherein the non-cleavable linker comprises a polyethylene glycol (PEG) characterized by a formula R³-(O-CH₂-CH₂)ₙ- or R³-(0-CH₂-CH₂)ₙ-O- with R³ being hydrogen, methyl or ethyl and n having a value from 2 to 200. In some aspects, the linker comprises a spacer, wherein the spacer is PEG.

In some aspects, the PEG linker is an oligo-ethylene glycol, e.g., diethylene glycol, triethylene glycol, tetra ethylene glycol (TEG), pentaethylene glycol, or a hexaethylene glycol (HEG) linker.

In some aspects, n has a value of 2, 3, 4, 5, 6, 7, 8, 9, 10, 11, 12, 13, 14, 15, 16, 17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 189, 181, 182, 183, 184, 185, 186, 187, 188, 189, 190, 191, 192, 193, 194, 195, 196, 197, 198, 199, or 200.

In some aspects, n is between 2 and 10, between 10 and 20, between 20 and 30, between 30 and 40, between 40 and 50, between 50 and 60, between 60 and 70, between 70 and 80, between 80 and 90, between 90 and 100, between 100 and 110, between 110 and 120, between 120 and 130, between 130 and 140, between 140 and 150, between 150 and 160, between 160 and 170, between 170 and 180, between 180 and 190, or between 190 and 200.

In some specific aspects, n has a value from 3 to 200, from 3 to 20, from 10 to 30, or from 9 to 45.

In some aspects, the PEG is a branched PEG. Branched PEG has three to ten PEG chains emanating from a central core group.

In certain aspects, the PEG moiety is a monodisperse polyethylene glycol. In the context of the present disclosure, a monodisperse polyethylene glycol (mdPEG) is a PEG that has a single, defined chain length and molecular weight. mdPEG is typically generated by separation from the polymerization mixture by chromatography. In certain formulae, a monodisperse PEG moiety is assigned the abbreviation mdPEG.

In some aspects, the PEG is a Star PEG. Star PEG has 10 to 100 PEG chains emanating from a central core group.

In some aspects, the PEG is a Comb PEG. Comb PEG has multiple PEG chains normally grafted onto a polymer backbone.

In certain aspects, the PEG has a molar mass between 100 g/mol and 3000 g/mol, particularly between 100 g/mol and 2500 g/mol, more particularly of approx. 100 g/mol to 2000 g/mol. In certain aspects, the PEG has a molar mass between 200 g/mol and 3000 g/mol, particularly between 300 g/mol and 2500 g/mol, more particularly of approx. 400 g/mol to 2000 g/mol.

In some aspects, the PEG is PEG₁₀₀, PEG₂₀₀, PEG₃₀₀, PEG₄₀₀, PEG₅₀₀, PEG₆₀₀, PEG₇₀₀, PEG₈₀₀, PEG₉₀₀, PEG₁₀₀₀, PEG₁₁₀₀, PEG₁₂₀₀, PEG₁₃₀₀, PEG₁₄₀₀, PEG₁₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₁₆₀₀, PEG₁₇₀₀, PEG₁₈₀₀, PEG₁₉₀₀, PEG₂₀₀₀, PEG₂₁₀₀, PEG₂₂₀₀, PEG₂₃₀₀, PEG₂₄₀₀, PEG₂₅₀₀, PEG₂₆₀₀, PEG₂₇₀₀, PEG₂₈₀₀, PEG₂₉₀₀, ₒᵣ PEG₃₀₀₀. In certain aspects, the PEG is PEG₄₀₀. In certain aspects, the PEG is PEG₂₀₀₀.

In some aspects, a linker combination of the present disclosure can comprise several PEG linkers, e.g., a cleavable linker flanked by PEG, HEG, or TEG linkers.

In some aspects, the linker combination comprises (HEG)n and/or (TEG)n, wherein n is an integer between 1 and 50, and each unit is connected, e.g., via a phosphate ester linker, a phosphorothioate ester linkage, or a combination thereof.

### II.G.3.a.2. Glycerol and Polyglycerols (PG)

In some aspects, the linker combination comprises a non-cleavable linker comprising a glycerol unit or a polyglycerol (PG) described by the formula ((R₃-O-(CH₂-CHOH-CH₂O)ₙ-) with R3 being hydrogen, methyl or ethyl, and n having a value from 3 to 200. In some aspects, n has a value from 3 to 20. In some aspects, n has a value from 10 to 30.

In some aspects, the PG linker is a diglycerol, triglycerol, tetraglycerol (TG), pentaglycerol, or a hexaglycerol (HG) linker.

In some aspects, n has a value of 2, 3, 4, 5, 6, 7, 8, 9, 10, 11, 12, 13, 14, 15, 16, 17,18,19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 189, 181, 182, 183, 184, 185, 186, 187, 188, 189, 190, 191, 192, 193, 194, 195, 196, 197, 198, 199, or 200.

In some aspects, n is between 2 and 10, between 10 and 20, between 20 and 30, between 30 and 40, between 40 and 50, between 50 and 60, between 60 and 70, between 70 and 80, between 80 and 90, between 90 and 100, between 100 and 110, between 110 and 120, between 120 and 130, between 130 and 140, between 140 and 150, between 150 and 160, between 160 and 170, between 170 and 180, between 180 and 190, or between 190 and 200.

In some alternatives of these aspects, n has a value from 9 to 45. In some aspects, the heterologous moiety is a branched polyglycerol described by the formula (R³-O-(CH₂-CHOR⁵-CH₂-O)ₙ-) with R⁵ being hydrogen or a linear glycerol chain described by the formula (R³-O-(CH₂-CHOH-CH₂-O)ₙ-) and R³ being hydrogen, methyl or ethyl. In some aspects, the heterologous moiety is a hyperbranched polyglycerol described by the formula (R³-O-(CH₂-CHOR⁵-CH₂-O)ₙ-) with R⁵ being hydrogen or a glycerol chain described by the formula (R³-O-(CH₂-CHOR⁶-CH₂-O)ₙ-), with R⁶ being hydrogen or a glycerol chain described by the formula (R³-O-(CH₂-CHOR⁷-CH₂-O)ₙ-), with R⁷ being hydrogen or a linear glycerol chain described by the formula (R³-O-(CH₂-CHOH-CH₂-O)ₙ-) and R³ being hydrogen, methyl or ethyl. Hyperbranched glycerol and methods for its synthesis are described in Oudshorn et al. (2006) Biomaterials 27:5471-5479; Wilms et al. (20100 Acc. Chem. Res. 43, 129-41, and references cited therein.

In certain aspects, the PG has a molar mass between 100 g/mol and 3000 g/mol, particularly between 100 g/mol and 2500 g/mol, more particularly of approx. 100 g/mol to 2000 g/mol. In certain aspects, the PG has a molar mass between 200 g/mol and 3000 g/mol, particularly between 300 g/mol and 2500 g/mol, more particularly of approx. 400 g/mol to 2000 g/mol.

In some aspects, the PG is PG₁₀₀, PG₂₀₀, PG₃₀₀, PG₄₀₀, PG₅₀₀, PG₆₀₀, PG₇₀₀, PG₈₀₀, PG₉₀₀, PG₁₀₀₀, PG₁₁₀₀, PG₁₂₀₀, PG₁₃₀₀, PG₁₄₀₀, PG₁₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₁₆₀₀, PG₁₇₀₀, PG₁₈₀₀, PG₁₉₀₀, PG₂₀₀₀, PG₂₁₀₀, PG₂₂₀₀, PG₂₃₀₀, PG₂₄₀₀, PG₂₅₀₀, PG₂₆₀₀, PG₂₇₀₀, PG₂₈₀₀, PG₂₉₀₀, ₒᵣ PG₃₀₀₀. In one particular aspect, the PG is PG₄₀₀. In another particular aspect, the PG is PG₂₀₀₀.

In some aspects, the linker combination comprises (glycerol)n, and/or (HG)n and/or (TG)n, wherein n is an integer between 1 and 50, and each unit is connected, e.g., via a phosphate ester linker, a phosphorothioate ester linkage, or a combination thereof.

### II.G.3.a.3. Aliphatic (Alkyl) linkers

In some aspects, the linker combination comprises at least one aliphatic (alkyl) linker, e.g., propyl, butyl, hexyl, or C2-C12 alkyl, such as C2-C10 alkyl or C2-C6 alkyl.

In some aspects, the linker combination comprises an alkyl chain, e.g., an unsubstituted alkyl. In some aspects, the linker combination comprises an substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, Aryl, heteroaryl, heterocyclyl, cycloalkyl, cycloalkenyl, alkylarylalkyl, alkylarylalkenyl, alkylarylalkynyl, alkenylarylalkyl, alkenyl Reyl alkenyl, alkenyl aryl alkynyl, alkynyl aryl alkyl, alkynyl aryl alkenyl, alkynyl aryl alkynyl, alkyl heteroaryl alkyl, alkyl heteroaryl alkyl, alkyl heteroaryl alkenyl, alkyl heteroaryl alkynyl, alkenyl heteroaryl alkyl, alkenyl heteroaryl alkenyl, alkenyl heteroaryl alkynyl, alkynyl Heteroarylalkyl, alkynylheteroarylalkenyl, alkynylheteroarylalkynyl, alkylheterocyclylalkyl, alkylheterocyclylalkenyl, alkylheterocyclylalkynyl, alkenylheterocyclylalkyl, alkenylheterocyclylalkenyl, or alkenylheterocyclylalkynyl.

Optionally these components are substituted. Substituents include alcohol, alkoxy (such as methoxy, ethoxy, and propoxy), straight or branched chain alkyl (such as C1-C12 alkyl), amine, aminoalkyl (such as amino C1-C12 alkyl), phosphoramidite, phosphate, phosphoramidate, phosphorodithioate, thiophosphate, hydrazide, hydrazine, halogen, (such as F, Cl, Br, or I), amide, alkylamide (such as amide C1-C12 alkyl), carboxylic acid, carboxylic ester, carboxylic anhydride, carboxylic acid halide, ether, sulfonyl halide, imidate ester, isocyanate, isothiocyanate, haloformate, carboduimide adduct, aldehydes, ketone, sulfhydryl, haloacetyl, alkyl halide, alkyl sulfonate, C(=O)CH=CHC(=O) (maleimide), thioether, cyano, sugar (such as mannose, galactose, and glucose), α,β-unsaturated carbonyl, alkyl mercurial, or α,β-unsaturated sulfone.

Examples of aliphatic linkers include the following structures:

-O-CO-O-

-NH-CO-O-

-NH-CO-NH-

-NH-(CH₂)ₙ₁-

-S-(CH₂)ₙ₁-

-CO-(CH₂)ₙ₁-CO-

-CO-(CH₂)ₙ₁-NH-

-NH-(CH₂)ₙ₁-NH-

-CO-NH-(CH₂)ₙ₁-NH-CO-

-C(=S)-NH-(CH₂)ₙ₁-NH-CO-

-C(=S)-NH-(CH₂)ₙ₁-NH-C-(=S)-

-CO-O-(CH₂)ₙ₁-O-CO-

-C(=S)-O-(CH₂)ₙ₁-O-CO-

-C(=S)-O-(CH₂)ₙ₁-O-C-(=S)-

-CO-NH-(CH₂)ₙ₁-O-CO-

-C(=S)-NH-(CH₂)ₙ₁-O-CO-

-C(=S)-NH-(CH₂)ₙ₁-O-C-(=S)-

-CO-NH-(CH₂)ₙ₁-O-CO-

-C(=S)-NH-(CH₂)ₙ₁-CO-

-C(=S)-O-(CH₂)ₙ₁-NH-CO-

-C(=S)-NH-(CH₂)ₙ₁-O-C-(=S)-

-NH-(CH₂CH₂O)ₙ₂-CH(CH₂OH)-

-NH-(CH₂CH₂O)ₙ₂-CH₂-

-NH-(CH₂CH₂O)ₙ₂-CH₂-CO-

-O-(CH₂)ₙ₃-S-S-(CH₂)ₙ₄-O-P(=O)₂-

-CO-(CH₂)ₙ₃-O-CO-NH-(CH₂)ₙ₄-

-CO-(CH₂)ₙ₃-CO-NH-(CH₂)ₙ₄-

- (CH2)ₙ₁NH-

-C(O)(CH2)ₙ₁NH-

-C(O)-(CH2)ₙ₁-C(O)-

-C(O)-(CH2)ₙ₁-C(O)O-

-C(O)-O-

-C(O)-(CH2)ₙ₁-NH-C(O)-

-C(O)-(CH2)ₙ₁-

-C(O)-NH-

-C(O)-

- (CH2)ₙ₁-C(O)-

- (CH2)ₙ₁-C(O)O-

- (CH2)ₙ₁-

- (CH2)ₙ₁-NH-C(O)-

n1 is an integer between 1 and 40 (e.g., 2 to 20, or 2 to 12); n2 is an integer between 1 and 20 (e.g., 1 to 10, or 1 to 6); n3 and n4 can be the same or different, and are an integer between 1 and 20 (e.g., 1 to 10, or 1 to 6).

In some aspects, the linker combination comprises (C3)n, (C4)n, (C5)n, (C6)n, (C7)n, or (C8)n, or a combination thereof, wherein n is an integer between 1 and 50, and each unit is connected, e.g., via a phosphate ester linker, a phosphorothioate ester linkage, or a combination thereof.

### II.G.3.b. Cleavable linkers

In some aspects, the linker combination comprises a cleavable linker. The term "cleavable linker" refers to a linker comprising at least one linkage or chemical bond that can be broken or cleaved. Cleavage can be mediated, e.g., by a nuclease, peptidase, protease, phosphatase, oxidase, esterase, or reductase, for example, or by specific physicochemical conditions, e.g., redox environment, pH, presence of reactive oxygen species, or specific wavelengths of light.

In some aspects, the cleavable linker comprises a dinucleotide or trinucleotide or tetrapeptide linker, a disulfide, a diselenide, an imine, a beta-carboxylic amide, a thioketal, a val-cit dipeptide, a glutamic acid-valine-citrulline tripeptide, a glycine-histidine-leucine-glycine tetrapeptide, or any combination thereof. In some aspects, the cleavable linker comprises valine-alanine-p-aminobenzylcarbamate or valine-citrulline-p-aminobenzylcarbamate.

### II.B.4. Specific examples and topologies

In specific aspects of the present disclosure, the linker combination consists of a linker of formula

[Alkyl linker]m-[PEG1]n-[PEG2]o

wherein m, n, and o are 0 or 1, and at least one of m, n, or o is not zero. Exemplary linker combinations according to such formula are C6-TEG-HEG, C6-HEG, C6-TEG, C6, TEG-HEG, TEG, C8-TEG-HEG, C8-HEG, C8-TEG, and C8.

In some aspects, the linker combination comprises a non-cleavable linker (e.g., TEG or HEG) in combination with one or more cleavable linkers, e.g., an enzymatic cleavable linker and a self immolative linker.

In a specific aspect, the linker combination the linker combination comprises the linker combination TEG (non-cleavable linker)-Val-Cit(cleavable linker)-pAB(self-immolative linker), as shown below

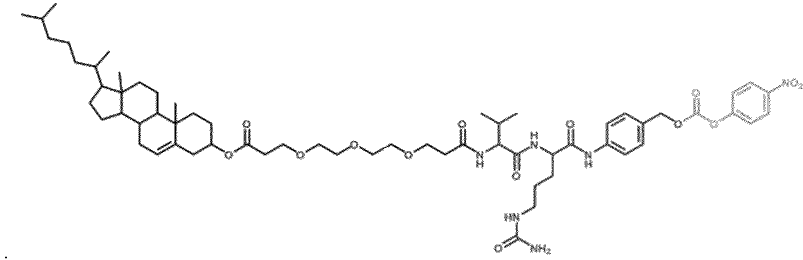

Specific combinations of anchoring moieties and linker combinations are illustrated in the tables below.

**Table 1. Anchoring Moieties and Linker Combinations**

| | **Linker combination** | | |
|---|---|---|---|
| **Anchoring moiety** | **1^{st} Linker** | **2^{nd} Linker** | **3^{rd} Linker** |
| Cholesterol | C6 | TEG | HEG |
| Cholesterol | C6 | HEG | No |
| Cholesterol | C6 | TEG | No |
| Cholesterol | C6 | No | No |
| Cholesterol | TEG | HEG | No |
| Cholesterol | TEG | No | No |
| Tocopherol | C8 | TEG | HEG |
| Tocopherol | C8 | HEG | No |
| Tocopherol | C8 | TEG | No |
| Tocopherol | C8 | No | No |
| Tocopherol | TEG | HEG | No |
| Tocopherol | HEG | No | No |
| Tocopherol | TEG | No | No |
| Tocopherol | No | No | No |
| Palmitate | C6 | TEG | HEG |
| Palmitate | C6 | HEG | No |
| Palmitate | C6 | TEG | No |
| Palmitate | C6 | No | No |
| Cholesterol | TEG | Glycerol | HEG |

**Table 2. Exemplary Linker Combinations**

| **Linker Combination** | | |
|---|---|---|
| **Linker 1** | **Cleavable Linker 2** | **Linker 3** |
| C6 | Disulfide Imine Thioketal Tri/Dinucleotide Val-Cit | C6 |
| None | | None |
| TEG | | TEG |
| HEG | | HEG |
| TEG-HEG | | TEG-HEG |

Specific payloads (*e.g*., ASOs) useful for the present disclosure are exemplified below:
[Cholesterol]-[TEG]-[HEG]-[ASO] 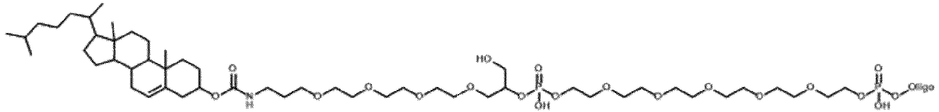
[Cholesterol]-[SMal]-[Val-Cit]-[pAB]-[ASO] 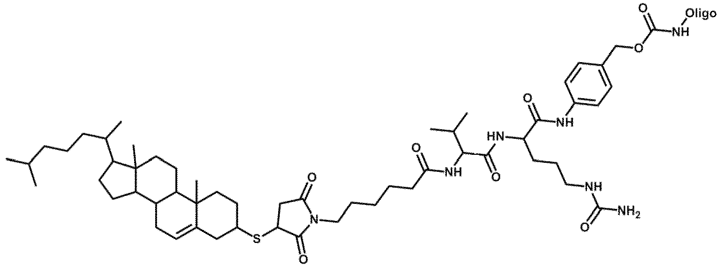
[Cholesterol]-[TEG]-[Val-Cit]-[C6]-[ASO] 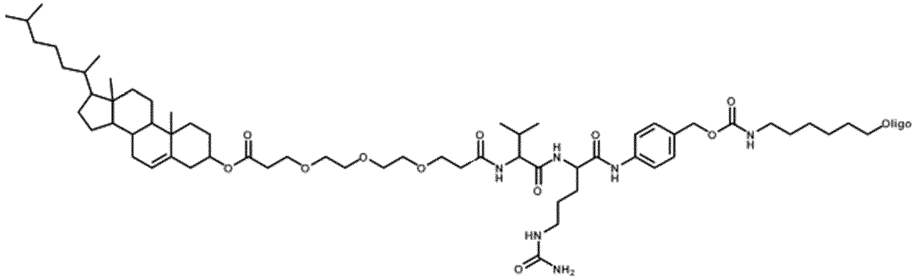
[Cholesterol]-[TEG]-[SS]-[C6]-[ASO] 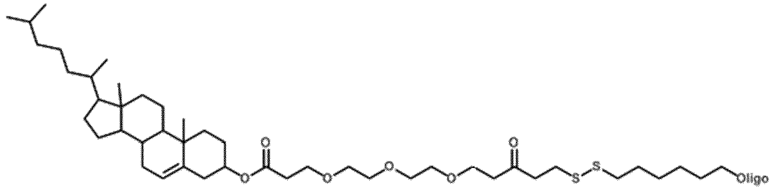 wherein [Cholesterol] is a cholesterol anchoring moiety, [TEG] is a TEG non-cleavable linker, [HEG] is a HEG non-cleavable linker, [SS] is a disulfide redox cleavable linker, [C6] is an alkyl non-cleavable linker, [SMal] is S-maleimide, [Val-Cit] is a valine-citrulline cleavable linker, [pAB] is a pAB self-immolative linker. In some aspects, a payload *(e.g.,* ASO) has a structure according to the exemplary structures provided above, in which one or more components has been replaced by a component in the same class as those depicted in the example. For example, the [cholesterol] anchoring moiety can be substituted by another anchoring moiety disclosed herein, a [TEG] can be substituted by another polymeric non-cleavable linker disclosed herein (e.g., HEG, PEG, PG), [Val-Cit] can be replaced by another peptidase cleavable linker, or [pAB] can be substituted by another self-immolative linker.

### III. EVs

In some aspects, the present disclosure provides an EV which has been modified to comprise an increased amount of a moiety of interest (*e.g.,* payload, *e.g.,* antisense oligonucleotide). For instance, as further described elsewhere in the present disclosure, loading an EV with a payload (*e.g.,* antisense oligonucleotide) at the loading conditions described herein (*e.g.,* at the recited salt concentrations, loading temperature, loading duration, payload feed concentration, and/or EV feed concentration) can increase the loading of the payload in the EV. Accordingly, in some aspects, an EV described herein comprises a payload, wherein the amount of the payload that is associated with the exterior surface of the EV (*e.g.,* payload concentration) is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 15-fold, at least about 20-fold, at least about 25-fold, at least about 30-fold, at least about 35-fold, at least about 40-fold, at least about 45-fold, or at least about 50-fold or more, compared to a reference EV (*e.g.,* an EV that has not been modified using the loading methods described herein).

In some aspects, the EVs of the present disclosure comprises a payload on the exterior surface of the EV, wherein the number of payloads on the exterior surface of the EV is at least about 1,000, at least about 2,000, at least about 3,000, at least about 4,000, at least about 5,000, at least about 6,000, at least about 7,000, at least about 8,000, at least about 9,000, at least about 10,000, at least about 11,000, at least about 12,000, at least about 13,000, at least about 14,000, at least about 15,000, at least about 16,000, at least about 17,000, at least about 18,000, at least about 19,000, at least about 20,000, at least about 21,000, at least about 22,000, at least about 23,000, at least about 24,000, at least about 25,000, at least about 30,000, at least about 35,000, at least about 40,000, at least about 45,000, or at least about 50,000. In some aspects, the number of payloads on the exterior surface of the EV is between about 5,000 and about 20,000, between about 6,000 and about 20,000, between about 7,000 and about 20,000, between about 8,000 and about 20,000, between about 9,000 and about 20,000, between about 10,000 and about 20,000, between about 5,000 and about 18,000, between about 6,000 and about 18,000, between about 7,000 and about 18,000, between about 8,000 and about 18,000, between about 9,000 and about 18,000, between about 10,000 and about 18,000, between about 5,000 and about 15,000, between about 6,000 and about 15,000, between about 7,000 and about 15,000, between about 8,000 and about 15,000, between about 9,000 and about 15,000, or between about 10,000 and about 15,000. In some aspects, the number of payloads on the exterior surface of the EV is between about 10,000 and about 15,000.

In some aspects, each of the payloads on the exterior surface of the EV are the same. In some aspects, one or more of the payloads on the exterior surface of the EV are different. As described herein, in some aspects, an EV of the present disclosure can comprise one or more additional payloads. In certain aspects, the one or more additional payloads can be in the lumen of the EVs (*i.e.,* encapsulated), associated with the exterior surface of the EVs, associated with the luminal surface of the EVs, or combinations thereof.

As described herein, EVs that are useful for the present disclosure can have a diameter between about 20-300 nm. In certain aspects, an EV of the present disclosure has a diameter between about 20-290 nm, 20-280 nm, 20-270 nm, 20-260 nm, 20-250 nm, 20-240 nm, 20-230 nm, 20-220 nm, 20-210 nm, 20-200 nm, 20-190 nm, 20-180 nm, 20-170 nm, 20-160 nm, 20-150 nm, 20-140 nm, 20-130 nm, 20-120 nm, 20-110 nm, 20-100 nm, 20-90 nm, 20-80 nm, 20-70 nm, 20-60 nm, 20-50 nm, 20-40 nm, 20-30 nm, 30-300 nm, 30-290 nm, 30-280 nm, 30-270 nm, 30-260 nm, 30-250 nm, 30-240 nm, 30-230 nm, 30-220 nm, 30-210 nm, 30-200 nm, 30-190 nm, 30-180 nm, 30-170 nm, 30-160 nm, 30-150 nm, 30-140 nm, 30-130 nm, 30-120 nm, 30-110 nm, 30-100 nm, 30-90 nm, 30-80 nm, 30-70 nm, 30-60 nm, 30-50 nm, 30-40 nm, 40-300 nm, 40-290 nm, 40-280 nm, 40-270 nm, 40-260 nm, 40-250 nm, 40-240 nm, 40-230 nm, 40-220 nm, 40-210 nm, 40-200 nm, 40-190 nm, 40-180 nm, 40-170 nm, 40-160 nm, 40-150 nm, 40-140 nm, 40-130 nm, 40-120 nm, 40-110 nm, 40-100 nm, 40-90 nm, 40-80 nm, 40-70 nm, 40-60 nm, 40-50 nm, 50-300 nm, 50-290 nm, 50-280 nm, 50-270 nm, 50-260 nm, 50-250 nm, 50-240 nm, 50-230 nm, 50-220 nm, 50-210 nm, 50-200 nm, 50-190 nm, 50-180 nm, 50-170 nm, 50-160 nm, 50-150 nm, 50-140 nm, 50-130 nm, 50-120 nm, 50-110 nm, 50-100 nm, 50-90 nm, 50-80 nm, 50-70 nm, 50-60 nm, 60-300 nm, 60-290 nm, 60-280 nm, 60-270 nm, 60-260 nm, 60-250 nm, 60-240 nm, 60-230 nm, 60-220 nm, 60-210 nm, 60-200 nm, 60-190 nm, 60-180 nm, 60-170 nm, 60-160 nm, 60-150 nm, 60-140 nm, 60-130 nm, 60-120 nm, 60-110 nm, 60-100 nm, 60-90 nm, 60-80 nm, 60-70 nm, 70-300 nm, 70-290 nm, 70-280 nm, 70-270 nm, 70-260 nm, 70-250 nm, 70-240 nm, 70-230 nm, 70-220 nm, 70-210 nm, 70-200 nm, 70-190 nm, 70-180 nm, 70-170 nm, 70-160 nm, 70-150 nm, 70-140 nm, 70-130 nm, 70-120 nm, 70-110 nm, 70-100 nm, 70-90 nm, 70-80 nm, 80-300 nm, 80-290 nm, 80-280 nm, 80-270 nm, 80-260 nm, 80-250 nm, 80-240 nm, 80-230 nm, 80-220 nm, 80-210 nm, 80-200 nm, 80-190 nm, 80-180 nm, 80-170 nm, 80-160 nm, 80-150 nm, 80-140 nm, 80-130 nm, 80-120 nm, 80-110 nm, 80-100 nm, 80-90 nm, 90-300 nm, 90-290 nm, 90-280 nm, 90-270 nm, 90-260 nm, 90-250 nm, 90-240 nm, 90-230 nm, 90-220 nm, 90-210 nm, 90-200 nm, 90-190 nm, 90-180 nm, 90-170 nm, 90-160 nm, 90-150 nm, 90-140 nm, 90-130 nm, 90-120 nm, 90-110 nm, 90-100 nm, 100-300 nm, 110-290 nm, 120-280 nm, 130-270 nm, 140-260 nm, 150-250 nm, 160-240 nm, 170-230 nm, 180-220 nm, or 190-210 nm. The size of the EV described herein can be measured according to methods known in the art.

In some aspects, an EV described herein comprises a bi-lipid membrane, comprising an interior (luminal) surface and an exterior surface. In certain aspects, the interior (luminal) surface faces the inner core (*i.e.,* lumen) of the EV. In certain aspects, the exterior surface (also referred to herein as the "external surface") can be in contact with the endosome, the multivesicular bodies, or the membrane/cytoplasm of a producer cell or a target cell.

In some aspects, an EV membrane comprises lipids and fatty acids. In some aspects, the EV membrane comprises phospholipids, glycolipids, fatty acids, sphingolipids, phosphoglycerides, sterols, cholesterols, and phosphatidylserines.

In some aspects, the EV membrane comprises an inner leaflet and an outer leaflet. The composition of the inner and outer leaflet can be determined by transbilayer distribution assays known in the art, *see, e.g.,* Kuypers et al., Biohim Biophys Acta 819:170 (1985). In some aspects, the composition of the outer leaflet is between approximately 70-90% choline phospholipids, between approximately 0-15% acidic phospholipids, and between approximately 5-30% phosphatidylethanolamine. In some aspects, the composition of the inner leaflet is between approximately 15-40% choline phospholipids, between approximately 10-50% acidic phospholipids, and between approximately 30-60% phosphatidylethanolamine.

In some aspects, the EV membrane comprises one or more polysaccharides, such as a glycan. Glycans on the surface of the EV can serve as an attachment to a maleimide moiety or a linker that connect the glycan and a maleimide moiety. The glycan can be present on one or more proteins on the surface of an EV, for example, a PTGFRN polypeptide, or on the lipid membrane of the EV.

### III.A. EV Transmembrane Protein

In some aspects, the EVs useful for the present disclosure comprises one or more EV transmembrane proteins (also referred to herein as "exosomal protein" or "EV protein").

In certain aspects, the one or more transmembrane proteins are introduced into the EV (by transfection. In some aspects, the one or more transmembrane proteins can be introduced into the EV using synthetic macromolecules such as cationic lipids and polymers (Papapetrou et al., Gene Therapy 12: S118-S130 (2005)). In certain aspects, chemicals such as calcium phosphate, cyclodextrin, or polybrene, can be used to introduce the one or more transmembrane proteins to the EV.

In some aspects, one or more transmembrane proteins can be CD47, CD55, CD49, CD40, CD133, CD59, glypican-1, CD9, CD63, CD81, integrins, selectins, lectins, cadherins, other similar polypeptides known to those of skill in the art, or any combination thereof.

In some aspects, one or more transmembrane proteins are expressed in the membrane of the EVs by recombinantly expressing the transmembrane proteins in the producer cells. The EVs obtained from the producer cells can be further modified to be conjugated to a maleimide moiety or to a linker.

Examples of transmembrane proteins include, but are not limited to, PTGFRN, BSG, IGSF2, IGSF3, IGSF8, ITGB1, ITGA4, SLC3A2, ATP transporter, or a fragment or a variant thereof). In some aspects, the transmembrane proteins can be CD9, CD63, CD81, PDGFR, GPI anchor proteins, lactadherin, LAMP2, or LAMP2B, or any combination thereof. Non-limiting examples of other transmembrane proteins that can be used with the present disclosure include: aminopeptidase N (CD13); Neprilysin, AKA membrane metalloendopeptidase (MME); ectonucleotide pyrophosphatase/phosphodiesterase family member 1 (ENPP1); Neuropilin-1 (NRP1); or any combination thereof.

In some aspects, further examples of the transmembrane proteins can be found in U.S. Patent Nos. 10,195,290, issued Feb. 5, 2019 and 10,561,740, issued Feb. 18, 2020, and International Application No. PCT/US2018/048026, filed August 24, 2018 (published as WO 2019/040920A1).

In some aspects, EVs of the present disclosure comprise an internal space (*i.e.,* lumen) that is different from that of the naturally occurring EVs. For example, the EV can be changed such that the composition on the luminal surface of the EV has the protein, lipid, or glycan content different from that of the naturally-occurring EVs.

In some aspects, engineered EVs can be produced from a cell transformed with an exogenous sequence encoding one or more exosomal proteins or a modification or a fragment of the exosomal proteins that changes the composition or content of the luminal surface of the exosome. Various modifications or fragments of the EV protein that can be expressed on the luminal surface of the EV can be used for the aspects of the present disclosure.

In some aspects, the EV proteins that can change the luminal surface of the EV include, but are not limited to the MARCKS protein, MARCKSL1 protein, BASP1 protein, or any combination thereof. In some aspects, the exosomal protein comprises Brain Acid Soluble Protein 1 (the BASP1 protein). The BASP1 protein is also known as 22 kDa neuronal tissue-enriched acidic protein or neuronal axonal membrane protein NAP-22. The full-length human BASP1 protein sequence (isomer 1) is shown below. Non limiting examples of exosome proteins include, but are not limited to, International Application No. PCT/US2018/061679, filed November 16, 2018 (published as WO 2019/099942A1), and PCT/US2019/033629, filed May 22, 2019 (published as WO 2020/101740A1).

### IV. Methods of Producing EVs

Generally, a method of producing an EV useful for the present disclosure comprises modifying a producer cell with one or more moieties of interest (*e.g.,* EV transmembrane protein).

The producer cell can be a mammalian cell line, a plant cell line, an insect cell line, a fungi cell line, or a prokaryotic cell line. In certain aspects, the producer cell is a mammalian cell line. Non-limiting examples of mammalian cell lines include: a human embryonic kidney (HEK) cell line, a Chinese hamster ovary (CHO) cell line, an HT-1080 cell line, a HeLa cell line, a PERC-6 cell line, a CEVEC cell line, a fibroblast cell line, an amniocyte cell line, an epithelial cell line, a mesenchymal stem cell (MSC) cell line, and combinations thereof. In certain aspects, the mammalian cell line comprises HEK-293 cells, BJ human foreskin fibroblast cells, fHDF fibroblast cells, AGE.HN^{®} neuronal precursor cells, CAP^{®} amniocyte cells, adipose mesenchymal stem cells, RPTEC/TERT1 cells, or combinations thereof. The producer cell can be a primary cell. The primary cell can be a primary mammalian cell, a primary plant cell, a primary insect cell, a primary fungi cell, or a primary prokaryotic cell.

The producer cell may not be an immune cell, such as an antigen presenting cell, a T cell, a B cell, a natural killer cell (NK cell), a macrophage, a T helper cell, or a regulatory T cell (Treg cell). The producer cell may not be an antigen presenting cell (*e.g.,* dendritic cells, macrophages, B cells, mast cells, neutrophils, Kupffer-Browicz cell, or a cell derived from any such cells). A producer cell may not be a naturally-existing antigen-presenting cell (*i.e.,* has been modified). A producer cell may not be naturally-existing dendritic cell, a B cell, a mast cell, a macrophage, a neutrophil, Kupffer-Browicz cell, cell derived from any of these cells, or any combination thereof.

The one or more moieties of interest (*e.g.,* EV transmembrane protein) can be introduced into the producer cell methods that are known to the skilled in the art. *See, e.g.,* WO 2020/191369 A1.

### IV.A. Isolating an EV

Generally, methods of producing EVs disclosed herein comprises isolating the EV from the producer cells. In certain aspects, the EVs are released by the producer cell into the cell culture medium. It is contemplated that all known manners of isolation of EVs are deemed suitable for use herein. For example, physical properties of EVs can be employed to separate them from a medium or other source material, including separation on the basis of electrical charge (*e.g.,* electrophoretic separation), size (*e.g.,* filtration, molecular sieving, *etc*.)*,* density (*e.g.,* regular or gradient centrifugation), Svedberg constant (*e.g.,* sedimentation with or without external force, *etc*.). Alternatively, or additionally, isolation can be based on one or more biological properties, and include methods that can employ surface markers *(*e.g., for precipitation, reversible binding to solid phase, FACS separation, specific ligand binding, non-specific ligand binding, affinity purification *etc*.).

Isolation and enrichment can be done in a general and non-selective manner, typically including serial centrifugation. Alternatively, isolation and enrichment can be done in a more specific and selective manner, such as using EV or producer cell-specific surface markers. For example, specific surface markers can be used in immunoprecipitation, FACS sorting, affinity purification, and magnetic separation with bead-bound ligands.

Size exclusion chromatography can be utilized to isolate the EVs. Size exclusion chromatography techniques are known in the art. Exemplary, non-limiting techniques are provided herein. A void volume fraction can be isolated and comprises the EVs of interest. Further, the EVs can be further isolated after chromatographic separation by centrifugation techniques (of one or more chromatography fractions), as is generally known in the art. For example, density gradient centrifugation can be utilized to further isolate the extracellular vesicles. It can be desirable to further separate the producer cell-derived EVs from EVs of other origin. For example, the producer cell-derived EVs can be separated from non-producer cell-derived EVs by immunosorbent capture using an antigen antibody specific for the producer cell.

The isolation of EVs can involve combinations of methods that include, but are not limited to, differential centrifugation, size-based membrane filtration, immunoprecipitation, FACS sorting, and magnetic separation.

In addition to the increased loading efficiency described herein, adjusting one or more of the different loading parameters (*e.g.,* salt concentration) can also greatly reduce dissociation of the moiety of interest (*e.g.,* payload, *e.g.,* ASO) from the EVs after loading and purification (*e.g.,* chromatography purification). As demonstrated herein (*see, e.g.,* Example 4), maintaining a high salt concentration (*e.g.,* greater than about 100 mM) during the purification step can reduce dissociation of the moiety of interest from the EVs by at least about 5%, at least about 10%, at least about 20%, at least about 30%, at least about 40%, at least about 50%, at least about 60%, at least about 70%, at least about 80%, at least about 90%, or about 100%, compared to the dissociation observed when purifying the loaded EVs under low salt concentration (*e.g.*, less than about 100 mM).

### V. Therapeutic Uses

As described herein, the EVs described herein can be rapidly engineered to comprise various moieties of interest (*e.g.,* payload, *e.g.,* antisense oligonucleotide), which can be useful in treating a wide range of diseases and disorders. Accordingly, in some aspects, the present disclosure provides methods of treating a disease or condition in a subject in need thereof comprising administering a composition comprising EVs of the present disclosure to the subject.

In some aspects, a disease or disorder that can be treated with the present EVs comprises a cancer. Non-limiting examples of cancers that can be treated with the present disclosure include a colorectal cancer, lung cancer (e.g., non-small cell lung cancer (NSCLC)), pancreatic cancer (*e.g.,* pancreatic ductal adenocarcinoma (PDAC)), leukemia, uterine cancer, ovarian cancer, bladder cancer, bile duct cancer, gastric cancer, or any combination thereof. In some aspects, the cancer is selected from colon adenocarcinoma, rectum adenocarcinoma, pancreatic adenocarcinoma, pancreatic ductal adenocarcinoma (PDAC), ovarian serous cystadenocarcinoma, acute myeloid leukemia, testicular germ cell tumors, lung adenocarcinoma, brain lower grade glioma, glioblastoma multiforme, uveal melanoma, thyroid carcinoma, uterine corpus endometrial carcinoma, uterine carcinosarcoma, pheochromocytoma, paraganglioma, and any combination thereof. In certain aspects, the cancer is a myeloid-rich cancer. In some aspects, the cancer comprises a liver cancer. In some aspects, the cancer comprises hepatocellular cancer (HCC). In some aspects, the cancer comprises pancreatic ductal adenocarcinoma (PDAC), in some aspects, the cancer comprises colorectal carcinoma (CRC). In some aspects, the cancer comprises ovarian cancer. In some aspects, the cancer comprises leptomeningeal cancer.

When administered to a subject with a cancer, in certain aspects, EVs of the present disclosure can up-regulate an immune response and enhance the tumor targeting of the subject's immune system. In some aspects, the cancer being treated is characterized by infiltration of leukocytes (T-cells, B-cells, macrophages, dendritic cells, monocytes) into the tumor microenvironment, or so-called "hot tumors" or "inflammatory tumors". In some aspects, the cancer being treated is characterized by low levels or undetectable levels of leukocyte infiltration into the tumor microenvironment, or so-called "cold tumors" or "non-inflammatory tumors". In some aspects, an EV is administered in an amount and for a time sufficient to convert a "cold tumor" into a "hot tumor", *i.e.,* said administering results in the infiltration of leukocytes (such as T-cells) into the tumor microenvironment. In certain aspects, cancer comprises bladder cancer, cervical cancer, renal cell cancer, testicular cancer, colorectal cancer, lung cancer, head and neck cancer, and ovarian, lymphoma, liver cancer, glioblastoma, melanoma, myeloma, leukemia, pancreatic cancers, or combinations thereof. In other term, "distal tumor" or "distant tumor" refers to a tumor that has spread from the original (or primary) tumor to distant organs or distant tissues, *e.g.,* lymph nodes. In some aspects, the EVs of the disclosure treats a tumor after the metastatic spread.

In some aspects, the EVs are administered intravenously to the circulatory system of the subject. In some aspects, the EVs are infused in suitable liquid and administered into a vein of the subject.

In some aspects, the EVs are administered intra-arterially to the circulatory system of the subject. In some aspects, the EVs are infused in suitable liquid and administered into an artery of the subject.

In some aspects, the EVs are administered to the subject by intrathecal administration

In some aspects, the EVs are administered *via* an injection into the spinal canal, or into the subarachnoid space so that it reaches the cerebrospinal fluid (CSF).

In some aspects, the EVs are administered intratumorally into one or more tumors of the subject.

In some aspects, the EVs are administered to the subject by intranasal administration. In some aspects, the EVs can be insufflated through the nose in a form of either topical administration or systemic administration. In certain aspects, the EVs are administered as nasal spray.

In some aspects, the EVs are administered to the subject by intraperitoneal administration. In some aspects, the EVs are infused in suitable liquid and injected into the peritoneum of the subject. In some aspects, the intraperitoneal administration results in distribution of the EVs to the lymphatics. In some aspects, the intraperitoneal administration results in distribution of the EVs to the thymus, spleen, and/or bone marrow. In some aspects, the intraperitoneal administration results in distribution of the EVs to one or more lymph nodes. In some aspects, the intraperitoneal administration results in distribution of the EVs to one or more of the cervical lymph node, the inguinal lymph node, the mediastinal lymph node, or the sternal lymph node. In some aspects, the intraperitoneal administration results in distribution of the EVs to the pancreas.

In some aspects, the EVs are administered to the subject by periocular administration. In some aspects, the s are injected into the periocular tissues. Periocular drug administration includes the routes of subconjunctival, anterior sub-Tenon's, posterior sub-Tenon's, and retrobulbar administration.

Certain aspects of the present disclosure are directed to methods of treating a brain tumor in a subject in need thereof. In some aspects, the method comprises administering to the subject a therapeutically effective amount of an EV described herein. In some aspects, the EV is capable of targeted delivery of a payload (*e.g.,* ASO) to the CNS to treat the brain tumor. In some aspects, the EV is capable of up-regulating an immune response in the subject, thereby enhancing the subject's immune response against the neuroimmunological disorder. In some aspects, the composition is administered intratumorally or intrathecally to the subject.

Also provided herein are methods of preventing metastasis of a brain tumor in a subject. The method comprises administering to the subject a therapeutically effective amount of the compositions disclosed herein, wherein the composition is capable of preventing a brain tumor at one location in the subject from promoting the growth of one or more tumors at another location in the subject. In some aspects, the composition is administered intratumorally or intrathecally in a first tumor in one location, and the composition administered in a first tumor prevents metastasis of one or more tumors at a second location.

### VI. Pharmaceutical Compositions and Methods of Administration

The present disclosure also provides pharmaceutical compositions comprising EVs described herein that are suitable for administration to a subject. The pharmaceutical compositions generally comprise a plurality of EVs comprising a biologically active molecule covalently linked to the plurality of EVs via a maleimide moiety and a pharmaceutically-acceptable excipient or carrier in a form suitable for administration to a subject. Pharmaceutically acceptable excipients or carriers are determined in part by the particular composition being administered, as well as by the particular method used to administer the composition. Accordingly, there is a wide variety of suitable formulations of pharmaceutical compositions comprising a plurality of EVs. (See, *e.g.,* Remington's Pharmaceutical Sciences, Mack Publishing Co., Easton, Pa. 18th ed. (1990)). The pharmaceutical compositions are generally formulated sterile and in full compliance with all Good Manufacturing Practice (GMP) regulations of the U.S. Food and Drug Administration. In some aspects, the pharmaceutical composition comprises one or more chemical compounds, such as for example, small molecules covalently linked to an EV described herein.

In some aspects, a pharmaceutical composition comprises one or more therapeutic agents and an EV described herein. In certain aspects, the EVs are co-administered with of one or more additional therapeutic agents, in a pharmaceutically acceptable carrier. In some aspects, the pharmaceutical composition comprising the EV is administered prior to administration of the additional therapeutic agents. In some aspects, the pharmaceutical composition comprising the EV is administered after the administration of the additional therapeutic agents. In further aspects, the pharmaceutical composition comprising the EV is administered concurrently with the additional therapeutic agents.

Provided herein are pharmaceutical compositions comprising an EV of the present disclosure having the desired degree of purity, and a pharmaceutically acceptable carrier or excipient, in a form suitable for administration to a subject. Pharmaceutically acceptable excipients or carriers can be determined in part by the particular composition being administered, as well as by the particular method used to administer the composition. Accordingly, there is a wide variety of suitable formulations of pharmaceutical compositions comprising a plurality of extracellular vesicles. (*See, e.g.,* Remington's Pharmaceutical Sciences, Mack Publishing Co., Easton, Pa. 21st ed. (2005)). The pharmaceutical compositions are generally formulated sterile and in full compliance with all Good Manufacturing Practice (GMP) regulations of the U.S. Food and Drug Administration.

In some aspects, a pharmaceutical composition comprises one or more therapeutic agents and an EV described herein. In certain aspects, the EVs are co-administered with of one or more additional therapeutic agents, in a pharmaceutically acceptable carrier. In some aspects, the pharmaceutical composition comprising the EV is administered prior to administration of the additional therapeutic agents. In some aspects, the pharmaceutical composition comprising the EV is administered after the administration of the additional therapeutic agents. In further aspects, the pharmaceutical composition comprising the EV is administered concurrently with the additional therapeutic agents.

Acceptable carriers, excipients, or stabilizers are nontoxic to recipients (*e.g.,* animals or humans) at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes); and/or non-ionic surfactants such as TWEEN^{™}, PLURONICS^{™} or polyethylene glycol (PEG).

Examples of carriers or diluents include, but are not limited to, water, saline, Ringer's solutions, dextrose solution, and 5% human serum albumin. The use of such media and compounds for pharmaceutically active substances is well known in the art. Except insofar as any conventional media or compound is incompatible with the extracellular vesicles described herein, use thereof in the compositions is contemplated. Supplementary therapeutic agents can also be incorporated into the compositions. Typically, a pharmaceutical composition is formulated to be compatible with its intended route of administration. The EVs of the present disclosure can be administered by parenteral, topical, intravenous, oral, subcutaneous, intra-arterial, intradermal, transdermal, rectal, intracranial, intraperitoneal, intranasal, intratumoral, intramuscular route or as inhalants. In certain aspects, the pharmaceutical composition comprising EVs is administered intravenously, *e.g.* by injection. The EVs can optionally be administered in combination with other therapeutic agents that are at least partly effective in treating the disease, disorder or condition for which the EVs are intended.

Solutions or suspensions can include the following components: a sterile diluent such as water, saline solution, fixed oils, polyethylene glycols, glycerin, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates, and compounds for the adjustment of tonicity such as sodium chloride or dextrose. The pH can be adjusted with acids or bases, such as hydrochloric acid or sodium hydroxide. The preparation can be enclosed in ampoules, disposable syringes or multiple dose vials made of glass or plastic.

Pharmaceutical compositions suitable for injectable use include sterile aqueous solutions (if water soluble) or dispersions and sterile powders. For intravenous administration, suitable carriers include physiological saline, bacteriostatic water, Cremophor EL^{™} (BASF, Parsippany, N.J.) or phosphate buffered saline (PBS). The composition is generally sterile and fluid to the extent that easy syringeability exists. The carrier can be a solvent or dispersion medium containing, *e.g.,* water, ethanol, polyol (*e.g.,* glycerol, propylene glycol, and liquid polyethylene glycol, and the like), and suitable mixtures thereof. The proper fluidity can be maintained, *e.g.,* by the use of a coating such as lecithin, by the maintenance of the required particle size in the case of dispersion and by the use of surfactants. Prevention of the action of microorganisms can be achieved by various antibacterial and antifungal compounds, *e.g.,* parabens, chlorobutanol, phenol, ascorbic acid, thimerosal, and the like. If desired, isotonic compounds, *e.g.,* sugars, polyalcohols such as mannitol, sorbitol, and sodium chloride can be added to the composition. Prolonged absorption of the injectable compositions can be brought about by including in the composition a compound which delays absorption, *e.g.,* aluminum monostearate and gelatin.

Sterile injectable solutions can be prepared by incorporating the EVs of the present disclosure in an effective amount and in an appropriate solvent with one or a combination of ingredients enumerated herein, as desired. Generally, dispersions are prepared by incorporating the EVs into a sterile vehicle that contains a basic dispersion medium and any desired other ingredients. In the case of sterile powders for the preparation of sterile injectable solutions, methods of preparation are vacuum drying and freeze-drying that yields a powder of the active ingredient plus any additional desired ingredient from a previously sterile-filtered solution thereof. The EVs can be administered in the form of a depot injection or implant preparation which can be formulated in such a manner to permit a sustained or pulsatile release of the EVs.

Systemic administration of compositions comprising EVs of the present disclosure can also be by transmucosal means. For transmucosal administration, penetrants appropriate to the barrier to be permeated are used in the formulation. Such penetrants are generally known in the art, and include, *e.g.,* for transmucosal administration, detergents, bile salts, and fusidic acid derivatives. Transmucosal administration can be accomplished through the use of, *e.g.,* nasal sprays.

In certain aspects the pharmaceutical composition comprising EVs of the present disclosure is administered intravenously into a subject that would benefit from the pharmaceutical composition. In certain aspects, the composition is administered to the lymphatic system, *e.g.,* by intralymphatic injection or by intranodal injection (*see e.g.,* Senti et al., PNAS 105( 46): 17908 (2008)), or by intramuscular injection, by subcutaneous administration, by intratumoral injection, by direct injection into the thymus, or into the liver.

In certain aspects, the pharmaceutical composition comprising EVs of the present disclosure is administered as a liquid suspension. In certain aspects, the pharmaceutical composition is administered as a formulation that is capable of forming a depot following administration. In certain preferred aspects, the depot slowly releases the EVs into circulation, or remains in depot form.

Typically, pharmaceutically-acceptable compositions are highly purified to be free of contaminants, are biocompatible and not toxic, and are suited to administration to a subject. If water is a constituent of the carrier, the water is highly purified and processed to be free of contaminants, e.g., endotoxins.

The pharmaceutically-acceptable carrier can be lactose, dextrose, sucrose, sorbitol, mannitol, starch, gum acacia, calcium phosphate, alginates, gelatin, calcium silicate, microcrystalline cellulose, polyvinylpyrrolidone, cellulose, water, syrup, methyl cellulose, methylhydroxy benzoate, propylhydroxy benzoate, talc, magnesium stearate, and/or mineral oil, but is not limited thereto. The pharmaceutical composition can further include a lubricant, a wetting agent, a sweetener, a flavor enhancer, an emulsifying agent, a suspension agent, and/or a preservative.

The pharmaceutical compositions described herein comprise the EVs described herein and optionally a pharmaceutically active or therapeutic agent. The therapeutic agent can be a biological agent, a small molecule agent, or a nucleic acid agent.

Dosage forms are provided that comprise a pharmaceutical composition comprising the EVs described herein. In some aspects, the dosage form is formulated as a liquid suspension for intravenous injection. In some aspects, the dosage form is formulated as a liquid suspension for intratumoral injection.

The EVs of the present disclosure can be used concurrently with other drugs. To be specific, the EVs of the present disclosure can be used together with medicaments such as hormonal therapeutic agents, chemotherapeutic agents, immunotherapeutic agents, medicaments inhibiting the action of cell growth factors or cell growth factor receptors and the like.

### VII. Kits

Also disclosed are kits, or products of manufacture comprising one or more EVs of the present disclosure and optionally instructions for use. Generally, the kit, or product of manufacture contains a pharmaceutical composition described herein which comprises at least one EV of the present disclosure, and instructions for use. Generally, the kit, or product of manufacture comprises at least one EV of the present disclosure or a pharmaceutical composition comprising the EVs in one or more containers. One skilled in the art will readily recognize that the EVs of the present disclosure, pharmaceutical composition comprising the EVs of the present disclosure, or combinations thereof can be readily incorporated into one of the established kit formats which are well known in the art.

Generally, the kit, or product of manufacture comprises EVs one or more biologically active molecules, reagents to covalently attach the one or more biologically active molecules to the EVs, or any combination thereof, and instructions to conduct the reaction to covalently attach the one or more biologically active molecules to the EVs .

Generally, the kit comprises reagents to conjugate a biologically active molecule to an EV, and instructions to conduct the conjugation.

The practice of the present disclosure will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Sambrook et al., ed. (1989) Molecular Cloning A Laboratory Manual (2nd ed.; Cold Spring Harbor Laboratory Press); Sambrook et al., ed. (1992) Molecular Cloning: A Laboratory Manual, (Cold Springs Harbor Laboratory, NY); D. N. Glover ed., (1985) DNA Cloning, Volumes I and II; Gait, ed. (1984) Oligonucleotide Synthesis; Mullis et al. U.S. Pat. No. 4,683,195; Hames and Higgins, eds. (1984) Nucleic Acid Hybridization; Hames and Higgins, eds. (1984) Transcription And Translation; Freshney (1987) Culture Of Animal Cells (Alan R. Liss, Inc.); Immobilized Cells And Enzymes (IRL Press) (1986); Perbal (1984) A Practical Guide To Molecular Cloning; the treatise, Methods In Enzymology (Academic Press, Inc., N.Y.); Miller and Calos eds. (1987) Gene Transfer Vectors For Mammalian Cells, (Cold Spring Harbor Laboratory); Wu et al., eds., Methods In Enzymology, Vols. 154 and 155; Mayer and Walker, eds. (1987) Immunochemical Methods In Cell And Molecular Biology (Academic Press, London); Weir and Blackwell, eds., (1986) Handbook Of Experimental Immunology, Volumes I-IV; Manipulating the Mouse Embryo, Cold Spring Harbor Laboratory Press, Cold Spring Harbor, N.Y., (1986); ); Crooke, Antisense drug Technology: Principles, Strategies and Applications, 2nd Ed. CRC Press (2007) and in Ausubel et al. (1989) Current Protocols in Molecular Biology (John Wiley and Sons, Baltimore, Md.).

The following examples are offered by way of illustration and not by way of limitation.

### EXAMPLES

### EXAMPLE 1 -EFFECT OF SALT CONCENTRATION ON EV LOADING

To begin identifying the various loading parameters that are important in improving the payload loading efficiency, ASOs (*e.g.,* targeting STAT6) were mixed with EVs in loading buffers comprising different NaCl (25 mM, 50 mM, 100 mM, or 150 mM) and sucrose concentrations (2.5%, 3.75%, or 5%). The ASO and the EVs were mixed at 37 °C for 24 hours. Then, the mixture was cooled to room temperature, and subsequently, filtered using 0.45 µm (PVDF) filtration. The filtrate was sampled and tested for AEX-UHPLC (ASO content) and nanoparticle tracking analysis (NTA) (EV content) to determine the ASO loading efficiency prior to chromatography cleanup. Non-limiting examples of alternative methods for determining ASO content include (1) Quant-iT^{™} RiboGreen^{™} RNA Reagent (e.g., fluorescent dye for the quantitation of RNA in solution), (2) UV absorbance at 260 nm. Non-limiting examples of alternative methods for determining EV content include (1) intensity of dynamic light scattering and (2) UV absorbance at 405 nm. *See, e.g.,* WO 2019/246591 A1.

As shown in Table 3 below, increasing NaCl concentration resulted in greater loading of the ASOs onto the exterior surface of the exosomes. Among the different NaCl concentrations tested, the greatest ASO loading efficiency was observed with 150 mM, regardless of the sucrose concentration. These results suggest that modulating the salt concentration of the loading buffer can be useful in improving the loading efficiency of EVs.

**Table 3.**

| **Feed (250 µM ASO, 1x10¹³ EV particles)** | | **Sucrose (%)** | | |
|---|---|---|---|---|
| | | **5%** | **3.75%** | **2.5%** |
| **mM NaCl** | **25 mM** | 2030 | 1878 | 1949 |
| | **50 mM** | 2360 | 2200 | 2299 |
| | **100 mM** | 3080 | 3019 | 3136 |
| | **150 mM** | 3924 | 4058 | 4079 |

### EXAMPLE 2 -EFFECT OF TIME AND TEMPERATURE ON EV LOADING

Next, to characterize the effects of loading time and loading temperature on ASO loading of EVs, EVs were mixed with ASOs at 5 °C, 22 °C, and 37 °C for up to 48 hours. Then, the mixture was cooled, filtered, and the filtrate analyzed for ASO and EV contents as described in Example 1.

As shown in FIG. 1A, loading time had no impact when the ASOs and EVs were mixed at 5 °C (diamond). In contrast, when the ASOs and EVs were mixed at either 22 °C or 37 °C, increasing the loading time did have a positive effect on ASO loading efficiency (*see* square and triangle symbols, respectively, in FIG. 1A). At 22 °C, a loading plateau *(i.e.,* no further increase in ASO concentration of the EVs) was observed starting at around 24 hours. At 37 °C, there was a continual increase in ASO concentration in the EVs with increased loading time.

The above results demonstrate that both loading time and temperature can have an effect on the loading efficiency of the EVs. By increasing both the loading time and temperature, the results suggest that it can be possible to produce more potent EVs (*i.e.,* comprising greater amount of the loaded payload).

### EXAMPLE 3 - EFFECT OF MODULATING SALT CONCENTRATION, LOADING TIME, EV FEED CONCENTRATION, AND PAYLOAD FEED CONCENTRATION ON EV LOADING

To further assess the effect that different loading parameters (*e.g.,* those described herein) have on loading efficiency of EVs, the ASOs and EVs were mixed under one of the following two loading conditions: (i) Loading Buffer: 20 mM PO₄, 50 mM NaCl, 5.0% sucrose, pH 7.2, mixing temperature was room temperature, for 1 hour ("loading condition #1"); and (ii) Loading Buffer: 20 mM PO₄, 100 mM NaCl, 2.5% sucrose, pH 7.2, mixing temperature was room temperature, for 24 hours ("loading condition #2"). As a further variable, the EVs and ASOs were added to the mixture at various concentrations. In particular, the EVs were added at the following feed concentration: 1.4x10¹³, 1.0x10¹³, or 6.0x10¹². The ASOs were added at the following feed concentration: 800 µM, 600 µM, 400 µM, 200 µM, or 100 µM. Then, the ASO and EV contents were measured as described in the earlier Examples.

As shown in Tables 4 and 5 (below), the ASO loading efficiency was positively correlated with both increase in the EV feed concentration and increase in the ASO concentration. For instance, the highest loading efficiency (*i.e.,* highest ASO concentration of the EVs) was observed when both the EV feed concentration and the ASO concentration were the greatest (*i.e.,* 1.4x10¹³ and 800 µM, respectively). As between the two loading conditions, there was approximately a four-fold increase in the ASO loading efficiency when the ASOs and EVs were mixed under loading condition #2.

The above results confirm the positive effect that increased salt concentration and loading time have on the loading efficiency of EVs. The results further demonstrate that both the EV feed concentration and ASO feed concentration can affect loading efficiency.

**Table 4. ASO Concentration Per EV under Loading Condition #1**

| | **Feed [EV] particles** | | | |
|---|---|---|---|---|
| | | **1.4x10¹³** | **1.0x10¹³** | **6.0x10¹²** |
| **Feed [ASO] (µM)** | **800** | 6297 | 4731 | 3149 |
| | **600** | 4893 | 3885 | 2713 |
| | **400** | 5944 | 3117 | 1740 |
| | **200** | 3970 | 2587 | 1338 |
| | **100** | 5039 | 2209 | 1158 |

**Table 5. ASO Concentration Per EV under Loading Condition #2**

| | **Feed [EV] particles** | | | |
|---|---|---|---|---|
| | | **1.4x10¹³** | **1.0x10¹³** | **6.0x10¹²** |
| **Feed [ASO] (µM)** | **800** | 18280 | 16521 | 10439 |
| | **600** | 16848 | 9012 | 5107 |
| | **400** | 17775 | 13913 | 6121 |
| | **200** | 16263 | 6263 | 3903 |
| | **100** | 8083 | 6555 | 2207 |

### EXAMPLE 4 - ANALYSIS OF LOADING PARAMETERS ON EV POTENCY

To further understand the effect that different loading parameters on EV loading efficiency, two different anti-STAT6 ASOs were used as payloads: one targeting human STAT6 ("human ASO") and the other targeting mouse STAT6 ("mouse ASO"). The mouse ASO contains a sequence complementary with the mouse STAT6 transcript, and the human ASO is complementary to the human STAT6 transcript. It will be apparent to those skilled in the arts that the mouse and human STAT6 transcripts differ in overall length. Similar to the methods provided in the earlier Examples, the mouse and human ASOs were independently mixed with EVs in a loading buffer comprising NaCl at a concentration of 150 mM. The mouse ASO was added at one of the following feed concentrations: 100 mM, 800 mM, or 950 mM. The human ASO was added at one of the following feed concentrations: 100 mM, 800 mM, or 1000 mM. The additional loading conditions were as follows: 37 °C for 24 hours at EV feed concentration of 1x10¹³ p/mL. Then, the ASO and EV contents were measured as described in the earlier Examples.

As shown in Table 6 and in agreement with the earlier data (*see, e.g.,* Example 3), increase in ASO feed concentration resulted in greater loading of the ASOs on a per EV basis. This was observed with both the mouse and human ASOs, indicating that loading methods provided herein can be used to increase loading efficiency for ASOs varying in sequence lengths. Similar results (at least as to the loading density) were observed with ASOs targeting other targets and conjugated to different lipid-linkers. For instance, as shown in FIG. 5, using the loading methods provided herein, on average, 10-14-fold increase in ASO loading was observed compared to the control loading condition.

**Table 6. Human and Mouse ASO Concentration Per EV**

| **ASO** | **ASO Feed** | **Loading Buffer NaCl** | **ASO/EV** |
|---|---|---|---|
| Mouse | 100 mM | 150 mM | 4279 |
| Mouse | 800 mM | 150 mM | 12855 |
| Mouse | 950 mM | 150 mM | 14912 |
| Human | 100 mM | 150 mM | 4851 |
| Human | 800 mM | 150 mM | 14089 |
| Human | 1000 mM | 150 mM | 14811 |

Next, to confirm the potency of the resulting EVs, the EVs loaded with the mouse or human ASOs were used to knockdown STAT6 expression in liver cells. As shown in FIG. 4, when the total mass of ASO was dosed, there was no significant difference in the knockdown of STAT6 expression, demonstrating that the increased loading density of the ASOs allows the use of fewer EVs to achieve the same therapeutic effect.

The above results suggest that the loading methods provided herein allow for the generation of more potent EVs, which would allow a given batch of EVs produced to treat many more patients.

### EXAMPLE 5 -EFFECT OF LOADING TEMPERATURE, SODIUM CONCENTRATION, AND SUCROSE CONCENTRATION ON ASO CONCENTRATION OF EVS AFTER CHROMATOGRAPHY PURIFICATION

To understand whether the different loading parameters described herein have an effect on ASO concentration of EVs after purification, the EVs and ASOs were mixed using loading buffers comprising either high salt and low sucrose (150 mM NaCl and 2.5% sucrose) or low salt and high sucrose (50 mM NaCl and 5% sucrose) concentrations. The loading temperature was either 24 °C (*i.e.,* room temperature) or 37 °C. The EVs were added to the mixture at a feed concentration of **1x10¹³** p/mL. The ASOs were added at a feed concentration of 650 µM. Afterwards, the mixture of the EVs and the ASOs were filtered, and the loaded EVs cleaned up by chromatography with Capto Core 700 resin. The flow through were measured for EV loaded ASOs and free ASO by RiboGreen fluorescence.

As shown in Table 7 (below), the EVs had the highest ASO concentration when the EVs and ASOs were mixed using loading buffer with high salt concentration and at 37 °C loading temperature. The least amount of free ASOs were also observed under the same loading condition. With the low salt concentration loading buffer, there were less EVs loaded with the ASOs and more free ASO observed in the flow through at both of the loading temperatures tested. While the increase in loading temperature (37 °C v. 24 °C) did improve the loading efficiency observed with the low salt concentration loading buffer, it was still less than that observed with the high salt concentration at the 37 °C loading temperature.

**Table 7.**

| | **24 °C** | **37 °C** | |
|---|---|---|---|
| **Loading Buffer** | 50 mM NaCl | 50 mM NaCl | 150 mM NaCl |
| | 5% Sucrose | 5% Sucrose | 2.5% Sucrose |
| **CC700 Flow Throughs - ASO:EV** | 13,200 | 17,500 | 23,200 |
| **CC700 Flow Throughs - % Free ASO** | 4% | 2% | 1% |

The above results further confirm the positive effects that increased salt concentration and loading temperature have on the loading efficiency of EVs. The results also demonstrate that Capto Core can clean up the free ASO independent of the loading temperature and salt concentration, indicating its robust cleanup capabilities.

### EXAMPLE 6 - EFFECT OF DIFFERENT LOADING PARAMETERS ON ASO DISSOCIATION FROM EVS AFTER CHROMATOGRAPHY PURIFICATION

Next, ASO dissociation after chromatography purification in EVs loaded with ASOs under different loading conditions was assessed. Briefly, the EVs and ASOs were mixed using one of the following two loading buffers: (i) CEF (20 mM PO₄, 50 mM NaCl, 5% sucrose); and (ii) Hi (20 mM PO₄, 150 mM NaCl, 2.5% sucrose). The EVs and ASOs were mixed at either room temperature (~24 °C) or 37 °C. The ASO loaded EVs were then cleaned up by chromatography with Capto Core 700 resin. The effect of dilution factor, diluent, and hold time of diluted material on ASOs that dissociate from EVs after cleaning with Capto Core 700 was evaluated, and results are shown in Table 8.

**Table 8.**

| | **% Free in Sup at Time 0 Hour** | | | | | | | | |
|---|---|---|---|---|---|---|---|---|---|
| **Loading Buffer** | CEF | | | Hi | | | | | |
| **Loading Temperature** | 22 °C | | | Room Temperature | | | 37 °C | | |
| **Diluent** | CEF | Hi | PB S | CEF | Hi | PBS | CEF | Hi | PBS |
| **1x** | 4% | | | 2% | | | 1% | | |
| **5x** | 14% | 2% | 5% | 11% | 3% | 2% | 6% | 1% | 1% |
| **10x** | 14% | 2% | 2% | 15% | 2% | 3% | 10% | 1% | 1% |

| | **% Free in Sup at Time 20 Hours** | | | | | | | | |
|---|---|---|---|---|---|---|---|---|---|
| **Loading Buffer** | CEF | | | Hi | | | | | |
| **Loading Temperature** | 22 °C | | | Room Temperature | | | 37 °C | | |
| **Diluent** | CEF | Hi | PB S | CEF | Hi | PBS | CEF | Hi | PBS |
| **1x** | 7% | | | 2% | | | 1% | | |
| **5x** | 17% | 2% | 2% | 14% | 2% | 2% | 7% | 1% | 1% |
| **10x** | 29% | 1% | 1% | 23% | 2% | 3% | 15% | 1% | 1% |

The percent free indicated that when diluting loaded and cleaned up material in a loading buffer containing a lower salt concentration, more ASOs dissociate from the EVs. The dissociation became more drastic as the dilution factor increases. However, when diluting material in the higher salt containing loading buffer (150 mM NaCl), there was no dissociation of ASOs observed. When the diluted cleaned up material was held for 20 hours at room temperature, additional dissociation was observed only when the diluent contains lower salt concentrations (50 mM). For the higher salt containing diluents, no dissociation was observed when the diluted material was held for 20 hours at room temperature. This data indicates that the stability of the loaded exosomes can be optimized by maintaining a salt concentration.

### EXAMPLE 7 - SCALING OF ASO EV LOADING PROCESS

As is apparent from the above disclosures, the loading methods provided herein can be useful in producing EVs loaded with payloads on various scales. Table 9 (below) provides exemplary loading parameters for lab-scale, non-GMP, and GMP production.

**Table 9. Exemplary Loading Parameters**

| | **Lab-Scale Development Lot** | **Non-GMP Manufacturing Lot** | **GMP Manufacturing Lot** |
|---|---|---|---|
| Loading Buffer | 146 mM Sucrose, 150 mM Sodium Chloride, 15 mM Sodium Phosphate Dibasic, mm Potassium Phosphate Monobasic, pH 7.2 | | |
| ASO Feed Concentration (µM) | 636 | 601 | 638 |
| Exosome Feed Concentration (p/mL) | 7.0x10¹² | 7.0x10¹² | 6.5x10¹² |
| Loading Volume (mL) | 21 | 2488 | 1445 |
| Loading Temperature (°C) | 37 | 37 | 37 |
| Loading Time (hr) | 20 | 20 | 20 |
| Final Loading Density (ASO:EV) | 18,176 | 20,608 | 18,484 |
| Free ASO (%) | 2.4 | 2.5 | 1.8 |

## Claims

1. A method of loading a payload to an extracellular vesicle (EV) comprising mixing the payload with the EV
(i) in a loading buffer comprising a salt at a concentration between about 110 mM to lower than about 200 mM;
(ii) at a loading temperature higher than 4 °C; and
(iii) for a loading duration longer than about one hour,
wherein the loading buffer comprising the salt increases the loading of the payload to the EV by at least about 2 fold compared to a corresponding loading buffer without the salt or with a lower concentration of the salt.

2. The method of claim 1, wherein the salt comprises NaCl (sodium chloride), KCl (potassium chloride), PO₄ (phosphate salt), CaCl₂ (calcium chloride), MgCl₂ (magnesium chloride), Mg₂SO₄ (magnesium sulfate), ZnCl₂ (zinc chloride), MnCl₂ (manganese chloride), MnSO₄ (manganese sulfate), NaSCN (sodium thiocyanate), KSCN (potassium thiocyanate), LiCl (lithium chloride), K₂HPO₄ (dipotassium phosphate), Na₂SO₄ (sodium sulfate), NaPO₄ (sodium phosphate), K₂SO₄ (potassium phosphate), sodium acetate, sodium bromide, sodium iodide, potassium bromide, lithium bromide, sodium fluoride, potassium fluoride, lithium fluoride, lithium iodide, potassium acetate, lithium acetate, potassium iodide, calcium sulfate, sodium sulfate, chromium trichloride, chromium sulfate, sodium citrate, iron (III) chloride, yttrium (III) chloride, potassium sulfate, ferrous chloride, calcium citrate, magnesium phosphate, ferric chloride, arginine-HCl, or any combination thereof.

3. A method of improving one or more properties of an extracellular vesicle after loading with a payload comprising loading the payload to the EV according to the method of claim 1 or 2, wherein the one or more properties comprise: decreasing the dissociation of the payload from the EV, increasing the stability of the EV loaded with the payload, inter-particle interaction, particle rigidity, particle size, or a combination thereof, preferably wherein the improvement in the one or more properties allow for increased filterability of the EV.

4. The method of any one of claims 1 to 3, wherein the loading buffer further comprises one or more of the following components: tris, sucrose, glucose, trehalose, mannose, sorbitol, mannitol, glycerol, histidine, arginine, methionine, tryptophan, tyrosine, sodium phosphate, potassium phosphate, polymers, surfactants (e.g., polysorbates 80 and 20), chelating agents (e.g., EDTA, citrate), inorganic acids, or any combination thereof.

5. The method of claim 4, wherein the one or more components is sucrose, preferably wherein the sucrose is at a concentration lower than about 10 % w/v, lower than about 9 % w/v, lower than about 8 % w/v, lower than about 7 % w/v, lower than about 6% w/v, lower than about 5 % w/v, lower than about 4 % w/v, lower than about 3% w/v, or lower than about 2.5 % w/v.

6. The method of any one of claims 1 to 5, wherein the payload comprises a peptide, a small molecule, an oligonucleotide, an antisense oligonucleotide (ASO), a PMO, an mRNA, an miRNA, an lcRNA, an antagomir, a tRNA, a siRNA, peptide nucleic acid, a cell penetrating peptide, an adjuvant, a protein, a carbohydrate, a sugar, an amino acid, or any combination thereof, preferably wherein the payload in the loading buffer is at a concentration between about 100 µM and about 2500 mM.

7. The method of claim 1, wherein the payload is conjugated to an anchoring moiety, preferably wherein the anchoring moiety comprises a sterol, GM1, a lipid, a phospholipid, a vitamin, a small molecule, a peptide, or a combination thereof.

8. The method of claim 7, wherein the anchoring moiety further comprises a linker between the payload and the anchoring moiety.

9. The method of claim 7, which comprises (i) a payload comprising the nucleotide sequence set forth in any one of SEQ ID NOs: 41 to 112, and (ii) a linker selected from the linker combinations of TABLE 1 or TABLE 2.

10. The method of claim 9, wherein the payload is conjugated to a cholesterol.

11. The method of any one of claims 1 to 10, wherein the extracellular vesicle is at a concentration of at least about 1x10¹¹ to about 5x10¹³ particles/mL.

12. The method of any one of claims 1 to 11, wherein the EV further comprises a protein in the membrane of the EV, preferably wherein the protein comprises prostaglandin F2 receptor negative regulator (the PTGFRN protein); basigin (the BSG protein); immunoglobulin superfamily member 2 (the IGSF2 protein); immunoglobulin superfamily member 3 (the IGSF3 protein); immunoglobulin superfamily member 8 (the IGSF8 protein); integrin beta-1 (the ITGB1 protein); integrin alpha-4 (the ITGA4 protein); 4F2 cell-surface antigen heavy chain (the SLC3A2 protein); a class of ATP transporter proteins (the ATP1A1, ATP1A2, ATP1A3, ATP1A4, ATP1B3, ATP2B1, ATP2B2, ATP2B3, ATP2B4 proteins); a functional fragment thereof; and any combination thereof.

13. An extracellular vesicle (EV) prepared by the method of any one of claims 1 to 12.

14. A pharmaceutical composition comprising the EV of claim 13.

15. The extracellular vesicle of claim 13, or the pharmaceutical composition of claim 14, for use in treating a disease or condition in a subject in need thereof.

## Patentansprüche

1. Ein Verfahren zum Laden einer Nutzlast in ein extrazelluläres Vesikel (EV), umfassend das Mischen der Nutzlast mit dem EV
(i) in einem Ladepuffer, der ein Salz in einer Konzentration zwischen etwa 110 mM bis weniger als etwa 200 mM umfasst;
(ii) bei einer Ladetemperatur von mehr als 4 °C; und
(iii) für eine Ladedauer von mehr als etwa einer Stunde,
wobei der Ladepuffer, der das Salz umfasst, das Laden der Nutzlast in das EV um mindestens das Zweifache im Vergleich zu einem entsprechenden Ladepuffer ohne das Salz oder mit einer geringeren Salzkonzentration erhöht.

2. Das Verfahren nach Anspruch 1, wobei das Salz NaCl (Natriumchlorid), KCI (Kaliumchlorid), PO₄ (Phosphatsalz), CaCl₂ (Calciumchlorid), MgCl₂ (Magnesiumchlorid), Mg₂SO₄ (Magnesiumsulfat), ZnCl₂ (Zinkchlorid), MnCl₂ (Manganchlorid), MnSO₄ (Mangansulfat), NaSCN (Natriumthiocyanat), KSCN (Kaliumthiocyanat), LiCI (Lithiumchlorid), K₂HPO₄ (Dikaliumphosphat), Na₂SO₄ (Natriumsulfat), NaPO₄ (Natriumphosphat), K₂SO₄ (Kaliumphosphat), Natriumacetat, Natriumbromid, Natriumiodid, Kaliumbromid, Lithiumbromid, Natriumfluorid, Kaliumfluorid, Lithiumfluorid, Lithiumiodid, Kaliumacetat, Lithiumacetat, Kaliumiodid, Calciumsulfat, Natriumsulfat, Chromtrichlorid, Chromsulfat, Natriumcitrat, Eisen(III)-chlorid, Yttrium(III)-chlorid, Kaliumsulfat, Eisen(II)-chlorid, Calciumcitrat, Magnesiumphosphat, Eisen(III)-chlorid, Arginin-HCl oder eine beliebige Kombination davon umfasst.

3. Ein Verfahren zur Verbesserung einer oder mehrerer Eigenschaften eines extrazellulären Vesikels nach Laden mit einer Nutzlast, umfassend das Laden der Nutzlast in das EV gemäß dem Verfahren nach Anspruch 1 oder 2, wobei die eine oder mehrere Eigenschaften umfassen: Verringerung der Dissoziation der Nutzlast vom EV, Erhöhung der Stabilität des mit der Nutzlast beladenen EV, Wechselwirkung zwischen Partikeln, Partikelsteifigkeit, Partikelgröße oder eine Kombination davon, wobei die Verbesserung der einen oder mehreren Eigenschaften bevorzugt eine erhöhte Filtrierbarkeit des EV ermöglicht.

4. Das Verfahren nach einem der Ansprüche 1 bis 3, wobei der Ladepuffer weiter eine oder mehrere der folgenden Komponenten umfasst: Tris, Saccharose, Glucose, Trehalose, Mannose, Sorbit, Mannitol, Glycerin, Histidin, Arginin, Methionin, Tryptophan, Tyrosin, Natriumphosphat, Kaliumphosphat, Polymere, Tenside (*z. B.* Polysorbate 80 und 20), Chelatbildner (z. B.EDTA, Citrat), anorganische Säuren oder eine beliebige Kombination davon.

5. Das Verfahren nach Anspruch 4, wobei die eine oder mehrere Komponenten Saccharose ist, wobei die Saccharosekonzentration bevorzugt unter etwa 10 Gew./Vol.-%, unter etwa 9 Gew./Vol.-%, unter etwa 8 Gew./Vol.-%, unter etwa 7 Gew./Vol.-%, unter etwa 6 Gew./Vol.-%, unter etwa 5 Gew./Vol.-%, unter etwa 4 Gew./Vol.-%, unter etwa 3 Gew./Vol.-%, oder unter etwa 2,5 Gew./Vol.-% liegt.

6. Das Verfahren nach einem der Ansprüche 1 bis 5, wobei die Nutzlast ein Peptid, ein kleines Molekül, ein Oligonukleotid, ein Antisense-Oligonukleotid (ASO), ein PMO, eine mRNA, eine miRNA, eine IcRNA, ein Antagomir, eine tRNA, eine siRNA, Peptidnukleinsäure, ein zellpenetrierendes Peptid, ein Adjuvans, ein Protein, ein Kohlenhydrat, einen Zucker, eine Aminosäure oder eine beliebige Kombination davon umfasst, wobei die Nutzlast im Ladepuffer bevorzugt in einer Konzentration zwischen etwa 100 µM und etwa 2500 mM vorliegt.

7. Das Verfahren nach Anspruch 1, wobei die Nutzlast an eine Verankerungseinheit konjugiert ist, wobei die Verankerungseinheit bevorzugt ein Sterol, GM1, ein Lipid, ein Phospholipid, ein Vitamin, ein kleines Molekül, ein Peptid oder eine Kombination davon umfasst.

8. Das Verfahren nach Anspruch 7, wobei die Verankerungseinheit weiter ein Verbindungsglied zwischen der Nutzlast und der Verankerungseinheit umfasst.

9. Das Verfahren nach Anspruch 7, umfassend (i) eine Nutzlast, welche die in einer der SEQ ID NOs: 41 bis 112 festgelegte Nukleotidsequenz enthält, und (ii) ein Verbindungsglied ausgewählt aus den Verbindungsgliedkombinationen aus TABELLE 1 oder TABELLE 2.

10. Das Verfahren nach Anspruch 9, wobei die Nutzlast an Cholesterin gebunden ist.

11. Das Verfahren nach einem der Ansprüche 1 bis 10, wobei das extrazelluläre Vesikel in einer Konzentration von mindestens etwa 1x10¹¹ bis etwa 5x10¹³ Partikeln/mL vorliegt.

12. Das Verfahren nach einem der Ansprüche 1 bis 11, wobei das EV weiter ein Protein in der Membran des EV umfasst, wobei das Protein bevorzugt den Prostaglandin-F2-Rezeptor-Negativregulator (das PTGFRN-Protein); Basigin (das BSG-Protein); Mitglied der Immunglobulin-Superfamilie 2 (das IGSF2-Protein); Mitglied der Immunglobulin-Superfamilie 3 (das IGSF3-Protein); Mitglied der Immunglobulin- Superfamilie 8 (das IGSF8-Protein); Integrin beta-1 (das ITGB1-Protein); Integrin alpha-4 (das ITGA4-Protein); schwere Kette des 4F2-Zelloberflächenantigens (das SLC3-A2-Protein); eine Klasse von ATP-Transporterproteinen (die ATP1A1-, ATP1A2-, ATP1A3-, ATP1A4-, ATP1B3-, ATP2B1-, ATP2B2-, ATP2B3- und ATP2B4-Proteine); ein funktionelles Fragment davon; und eine beliebige Kombination davon umfasst.

13. Ein Extrazelluläres Vesikel (EV), das gemäß dem Verfahren nach einem der Ansprüche 1 bis 12 hergestellt ist.

14. Eine Pharmazeutische Zusammensetzung, die das EV nach Anspruch 13 umfasst.

15. Das extrazelluläres Vesikel nach Anspruch 13 oder die pharmazeutische Zusammensetzung nach Anspruch 14 zur Verwendung bei der Behandlung einer Erkrankung oder eines Zustands bei einem Patienten, der dies benötigt.

## Revendications

1. Procédé de chargement d'une charge utile dans une vésicule extracellulaire (EV) comprenant le mélange de la charge utile avec l'EV
(i) dans un tampon de chargement comprenant un sel à une concentration comprise entre environ 110 mM et moins d'environ 200 mM ;
(ii) à une température de chargement supérieure à 4 °C ; et
(iii) pendant une durée de chargement supérieure à environ une heure,
dans lequel le tampon de chargement comprenant le sel augmente le chargement de la charge utile dans l'EV d'au moins environ 2 fois par rapport à un tampon de chargement correspondant sans sel ou avec une concentration de sel plus faible.

2. Procédé selon la revendication 1, dans lequel le sel comprend NaCl (chlorure de sodium), KCI (chlorure de potassium), PO₄ (sel de phosphate), CaCl₂ (chlorure de calcium), MgCl₂ (chlorure de magnésium), Mg₂SO₄ (sulfate de magnésium), ZnCl₂ (chlorure de zinc), MnCl₂ (chlorure de manganèse), MnSO₄ (sulfate de manganèse), NaSCN (thiocyanate de sodium), KSCN (thiocyanate de potassium), LiCl (chlorure de lithium), K₂HPO₄ (phosphate dipotassique), Na₂SO₄ (sulfate de sodium), NaPO₄ (phosphate de sodium), K₂SO₄ (phosphate de potassium), acétate de sodium, bromure de sodium, iodure de sodium, bromure de potassium, bromure de lithium, fluorure de sodium, fluorure de potassium, fluorure de lithium, iodure de lithium, acétate de potassium, acétate de lithium, iodure de potassium, sulfate de calcium, sulfate de sodium, trichlorure de chrome, sulfate de chrome, citrate de sodium, chlorure de fer (III), chlorure d'yttrium (III), sulfate de potassium, chlorure ferreux, citrate de calcium, phosphate de magnésium, chlorure ferrique, arginine-HCl, ou toute combinaison de ceux-ci.

3. Procédé d'amélioration d'une ou plusieurs propriétés d'une vésicule extracellulaire après chargement d'une charge utile, comprenant le chargement de la charge utile dans l'EV selon le procédé de la revendication 1 ou 2, dans lequel les une ou plusieurs propriétés comprennent : la diminution de la dissociation de la charge utile de l'EV, l'augmentation de la stabilité de l'EV chargée de la charge utile, l'interaction interparticulaire, la rigidité des particules, la taille des particules, ou une combinaison de celles-ci, de préférence dans lequel l'amélioration des une ou plusieurs propriétés permet une filtrabilité accrue de l'EV.

4. Procédé selon l'une quelconque des revendications 1 à 3, dans lequel le tampon de chargement comprend en outre un ou plusieurs des composants suivants : tris, saccharose, glucose, tréhalose, mannose, sorbitol, mannitol, glycérol, histidine, arginine, méthionine, tryptophane, tyrosine, phosphate de sodium, phosphate de potassium, polymères, tensioactifs (*par exemple,* polysorbates 80 et 20), agents chélateurs (*par exemple,* EDTA, citrate), acides inorganiques ou toute combinaison de ceux-ci.

5. Procédé selon la revendication 4, dans lequel les un ou plusieurs composants sont du saccharose, de préférence dans lequel le saccharose présente une concentration inférieure à environ 10 % m/v, inférieure à environ 9 % m/v, inférieure à environ 8 % m/v, inférieure à environ 7 % m/v, inférieure à environ 6 % m/v, inférieure à environ 5 % m/v, inférieure à environ 4 % m/v, inférieure à environ 3 % m/v, ou inférieure à environ 2,5 % m/v.

6. Procédé selon l'une quelconque des revendications 1 à 5, dans lequel la charge utile comprend un peptide, une petite molécule, un oligonucléotide, un oligonucléotide antisens (ASO), un PMO, un ARNm, un miARN, un IcARN, un antagomir, un ARNt, un siARN, un acide nucléique peptidique, un peptide pénétrant dans les cellules, un adjuvant, une protéine, un glucide, un sucre, un acide aminé ou toute combinaison de ceux-ci, de préférence dans lequel la charge utile dans le tampon de chargement est à une concentration comprise entre environ 100 µM et environ 2 500 mM.

7. Procédé selon la revendication 1, dans lequel la charge utile est conjuguée à une fraction d'ancrage, de préférence dans lequel la fraction d'ancrage comprend un stérol, du GM1, un lipide, un phospholipide, une vitamine, une petite molécule, un peptide ou une combinaison de ceux-ci.

8. Procédé selon la revendication 7, dans lequel la fraction d'ancrage comprend en outre un coupleur entre la charge utile et la fraction d'ancrage.

9. Procédé de la revendication 7, qui comprend (i) une charge utile comprenant la séquence nucléotidique définie dans l'une quelconque parmi SEQ ID NO : 41 à 112, et (ii) un coupleur sélectionné parmi les combinaisons de coupleurs du TABLEAU 1 ou du TABLEAU 2.

10. Procédé selon la revendication 9, dans lequel la charge utile est conjuguée à un cholestérol.

11. Procédé selon l'une quelconque des revendications 1 à 10, dans lequel la vésicule extracellulaire est à une concentration d'au moins environ 1x10¹¹ à environ 5x10¹³ particules/ml.

12. Procédé selon l'une quelconque des revendications 1 à 11, dans lequel l'EV comprend en outre une protéine dans la membrane de l'EV, de préférence dans lequel la protéine comprend le régulateur négatif du récepteur de la prostaglandine F2 (protéine PTGFRN) ; la basigine (protéine BSG) ; le membre 2 de la superfamille des immunoglobulines (protéine IGSF2) ; le membre 3 de la superfamille des immunoglobulines (protéine IGSF3) ; le membre 8 de la superfamille des immunoglobulines (protéine IGSF8) ; l'intégrine bêta-1 (protéine ITGB1) ; l'intégrine alpha-4 (protéine ITGA4) ; la chaîne lourde de l'antigène de surface cellulaire 4F2 (protéine SLC3 A2) ; une classe de protéines de transport d'ATP (protéines ATP1A1, ATP1A2, ATP1A3, ATP1A4, ATP1B3, ATP2B1, ATP2B2, ATP2B3, ATP2B4) ; un fragment fonctionnel de celles-ci ; ou toute combinaison de celles-ci.

13. Vésicule extracellulaire (EV) préparée suivant le procédé selon l'une quelconque des revendications 1 à 12.

14. Composition pharmaceutique comprenant l'EV selon la revendication 13.

15. Vésicule extracellulaire selon la revendication 13, ou composition pharmaceutique de la revendication 14, destinée à être utilisée dans le traitement d'une maladie ou d'un état pathologique chez un sujet en ayant besoin.
